# EUROPEAN PATENT SPECIFICATION

(11) **EP 3 863 400 B1**
(45) Date of publication and mention of the grant of the patent: **02.12.2026**
(21) Application number: 19870724.2
(22) Date of filing: 10.10.2019
(51) Int. Cl.: A01K 67/0275, A61F 2/28, A61K 35/33, A61L 26/00, A61L 27/22, A61L 27/24, A61L 27/36, A61L 27/50, A61P 37/08, C12N 9/10

(54) **COMPOSITIONS AND METHODS FOR PREVENTING ALLERGIES**
ZUSAMMENSETZUNGEN UND VERFAHREN ZUR PRÄVENTION VON ALLERGIEN
COMPOSITIONS ET MÉTHODES DE PRÉVENTION DES ALLERGIES

(30) Priority: 10.10.2018 US 201862744061 P
(43) Date of publication of application: 18.08.2021
(73) Proprietor: Revivicor Inc., Blacksburg, VA 24060 (US)
(72) Inventor: BIANCHI, John, Blacksburg, VA 24060 (US); AYARES, David, Blacksburg, VA 24060 (US); WALTERS, Anneke, Christiansburg, VA 24073 (US); DANDRO, Amy, Blacksburg, VA 24060 (US)
(74) Representative: Carpmaels & Ransford LLP
(86) International application number: PCT/US2019/055713
(87) International publication number: WO 2020/077144

(56) References cited:
- EP-A2- 2 726 604
- WO-A1-2016/065046
- WO-A2-2005/089411
- US-A1- 2012 282 226
- US-A1- 2014 115 728
- US-A1- 2018 249 688
- FILIPPO NASO ET AL: "Alpha-Gal Inactivated Heart Valve Bioprostheses Exhibit an Anti-Calcification Propensity Similar to Knockout Tissues", TISSUE ENGINEERING PART A, vol. 23, no. 19-20, October 2017 (2017-10-01), US, pages 1181 - 1195, XP055716328, ISSN: 1937-3341, DOI: 10.1089/ten.tea.2016.0474
- JOSE L. ESTRADA ET AL: "Evaluation of human and non-human primate antibody binding to pig cells lacking GGTA1/CMAH/beta4GalNT2 genes", XENOTRANSPLANTATION, vol. 22, no. 3, March 2015 (2015-03-01), US, pages 194 - 202, XP055501188, ISSN: 0908-665X, DOI: 10.1111/xen.12161
- KLAUDIA TALAFOVÁ ET AL: ""Lost sugars" - reality of their biological and medical applications", CENTRAL EUROPEAN JOURNAL OF BIOLOGY, CENTRAL EUROPEAN SCIENCE JOURNALS, WA, vol. 7, no. 5, 3 August 2012 (2012-08-03), pages 777 - 793, XP035094054, ISSN: 1644-3632, DOI: 10.2478/S11535-012-0079-3
- RAMM ROBERT ET AL: "Decellularized GGTA1-KO pig heart valves do not bind preformed human xenoantibodies", BASIC RESEARCH IN CARDIOLOGY, SPRINGER BERLIN HEIDELBERG, BERLIN/HEIDELBERG, vol. 111, no. 4, 6 May 2016 (2016-05-06), pages 1 - 13, XP035996764, ISSN: 0300-8428, [retrieved on 20160506], DOI: 10.1007/S00395-016-0560-7
- HAWKINS ROBERT B. ET AL: "Premature Bioprosthetic Aortic Valve Degeneration Associated with Allergy to Galactose-Alpha-1,3-Galactose", JOURNAL OF CARDIAC SURGERY., vol. 31, no. 7, 30 May 2016 (2016-05-30), US, pages 446 - 448, XP055925119, ISSN: 0886-0440, Retrieved from the Internet <URL:https://onlinelibrary.wiley.com/doi/full-xml/10.1111/jocs.12764> DOI: 10.1111/jocs.12764
- PLATTS-MILLS THOMAS A ET AL: "Delayed Anaphylaxis Involving IgE to Galactose-alpha-1,3-galactose", CURRENT ALLERGY AND ASTHMA REPORTS, SPRINGER US, NEW YORK, vol. 15, no. 4, 1 July 2015 (2015-07-01), pages 1 - 6, XP035510405, ISSN: 1529-7322, [retrieved on 20150701], DOI: 10.1007/S11882-015-0512-6
- COMMINS ET AL.: "Delayed Anaphylaxis, Angioedema, or Urticaria After Consumption of Red Meat in Patients with IgE Antibodies Specific for galactose-alpha-1,3-galactose", THE JOURNAL OF ALLERGY AND CLINICAL IMMUNOLOGY, vol. 123, no. 2, 1 February 2009 (2009-02-01), pages 426 - 433, XP025907162, DOI: 10.1016/j.jaci.2008.10.052
- CABEZAS-CRUZ ET AL.: "Tick galactosyltransferases are Involved in alpha-Gal synthesis and play a role During Anaplasma phagocytophilum infection and Ixodes scapularis tick vector development", SCIENTIFIC REPORTS, vol. 8, no. 14224, 21 September 2018 (2018-09-21), pages 1 - 18, XP055703743
- HILGER ET AL.: "Role and Mechanism of Galactose-Alpha-1,3-Galactose in the Elicitation of Delayed Anaphylactic Reactions to Red Meat", CURRENT ALLERGY AND ASTHMA REPORTS, vol. 19, no. 3, 23 January 2019 (2019-01-23), pages 1 - 11, XP036714110, DOI: 10.1007/s11882-019-0835-9

## Description

### FIELD

Disclosed herein are compositions and methods for use in preventing or reducing the risk or severity of an allergic reaction to a carbohydrate epitope in a subject in need thereof, such as a subject with α-Gal Syndrome (AGS). Also disclosed are method of making such compositions.

### BACKGROUND

In the United States alone, more than 50 million people suffer at least one allergy. Food allergies, in particular, are on the rise. (Low, W. Et al. Into J Environ Res Public Health. 2018 Sep 18). IgE-mediated reactions are responsible for the majority of food hypersensitivity disorders and produce allergic symptoms. Allergies can result in considerable morbidity, impact negatively on quality of life and prove costly in terms of medical care.

α-Gal Syndrome (AGS) refers to a disorder associated with allergy or anaphylaxis (a severe allergic reaction) upon exposure to galactose-alpha 1,3-galactose (alpha-gal), for example, by eating beef or pork. In the United States, sensitization to α-Gal is recognized as a consequence of bites from the tick *Amblyomma americanum.* (Commins, SP, et al. J Allergy Clin Immunol. 2011;127:1286-1293). AGS is increasingly prevalent in tick-endemic areas of Europe, Australia and the United States, occurring worldwide where ticks are endemic.

Despite the risk of severe allergic reactions and even death, the current approach to management of AGS substantially relies on allergen avoidance and preparation to promptly treat allergic reactions. Yet, allergen avoidance is difficult, and accidental exposure to causal allergens may occur.

There remains a need for novel approaches to the prevention of allergies to carbohydrate epitopes, including, but not limited to, alpha-gal.

US 2012/0282226 A1 describes tissue products derived from animals lacking any expression of functional alpha 1,3 galactosyltransferase. US 2014/0115728 A1 describes double knockout (GT/CMAH-KO) pigs, organs and tissues. US 2018/0249688 A1 describes multi-transgenic pig for xenotransplantation. WO 2016/065046 A1 describes triple transgenic pigs suitable for xenograft. EP 2726604 describes cells deficient in CMP-n-acetylneuraminic acid hydroxylase and/or glycoprotein alpha-1,3-galactosyltransferase. WO2005/89411 describes tissue products derived from animals lacking any expression of functional alpha 1,3 galactosyltransferase. Commins et al (2009) describe delayed anaphylaxis, angioedema, or urticaria after consumption of red meat in patients with IgE antibodies specific for galactose-α-1,3-galactose. Cabezas-Cruz et al (2018) describe tick galactosyltransferases' involvement in α-Gal synthesis and role played during Anaplasma phagocytophilum infection and Ixodes scapularis tick vector development. Hilger et al (2019) describe the role and mechanism of galactose-alpha-1,3-galactose in the elicitation of delayed anaphylactic reactions to red meat. Naso et al (2017) describe alpha-gal inactivated heart valve bioprostheses exhibiting an anticalcification propensity similar to knockout tissues. Estrada et al (2015) describe evaluation of human and nonhuman primate antibody binding to pig cells lacking GGTA1/CMAH/β4GaINT2 genes. Talafová et al (2012) describe biological and medical applications of sialic acids and α-1,3-galactose. Ramm et al (2016) describe how decellularized GGTA1-KO pig heart valves do not bind to preformed human xenoantibodies. Platts-Mills (2015) discuss delayed anaphylaxis involving IgE to galactose-alpha-1,3-galactose. Hawkins (2016) discuss premature bioprosthetic aortic valve degeneration associated with allergy to galactose-alpha-1,3-galactose.

### SUMMARY

The invention is defined in the appending claims. The present invention provides a medical product comprising a biologic that is derived from a transgenic ungulate lacking an alpha 1,3 galactosyltransferase gene for use in the treatment of a cardiovascular disease in a human that has been diagnosed with α-Gal syndrome (AGS), wherein the medical product does not comprise galactose-alpha-1,3-galactose (alpha-gal), wherein the medical product prevents or reduces the risk or severity of an allergic reaction to the alpha-gal in the human, wherein the biologic is a cardiovascular implant comprising a heart valve, optionally wherein the heart valve is a bioprosthetic heart valve, and wherein the medical product does not exhibit premature degradation.

Disclosed are compositions and methods for use in preventing or reducing the risk or severity of an allergic reaction to alpha-gal. Also disclosed are methods of making such compositions.

Disclosed herein are compositions comprising at least one component derived from a non-primate mammal lacking expression of alpha-gal, i.e., a GalSafe^{®} mammal.

In one instance, the compositions are provided in the form of products for human use. In a particular instance, compositions can be selected from the group consisting of consumer products, medical products, medical devices, products used in laboratory research or products used in manufacture of medical products.

In one instance, the GalSafe^{®} mammal is an ungulate. In a particular instance, the ungulate is a cow, pig, goat, camel, or sheep.

In one instance, the GalSafe^{®} mammal further comprises one or more additional genetic modifications selected from the group consisting of inactivation or reduction of expression of Neu5Gc (CMAH knockout) and/or Beta4Gal (knockout of Beta4GalNT2).

In one instance, the GalSafe^{®} mammal does not exhibit any health or phenotypic differences compared with standard domestic, nonengineered mammals (wild-type).

In a particular instance, the tissue of the GalSafe^{®} mammal has similar or the same morphology, composition, mechanics, bioactive molecules, hematologic, biochemical, and/ or coagulation parameters as the wild-type mammal.

Consumer products are disclosed comprising at least one component derived from a GalSafe^{®} mammal. In a particular instance of the disclosure, the consumer produced is a food product, food additive, cosmetic product, cosmetic additive or medical product for consumer use.

In a particular instance of the disclosure, the food product is meat or meat by-product.

In a particular instance of the disclosure, the food product is a dairy product or dairy by-product (e.g., milk protein).

In a particular instance of the disclosure, the food product is consumed as derived from the GalSafe^{®} mammal, e.g., as a cut of meat. In other instances of the disclosure, the food product is further processed prior to consumption, e.g., a sausage patty, cured ham, cold cuts, smoke beef; salami; bacon, emulsion products (viennas, polonies, bratwurst)

In another particular instance of the disclosure, the food additive is selected from the group consisting of gelatin, rennet, edible tallows, lactose, whey and combinations thereof.

In a particular instance of the disclosure, the cosmetic product or cosmetic additive comprises at least one component selected from gelatin, keratin, collagen, elastin, lanolin, estrogen, hyalouronic acid or a combination thereof.

The medical product of the invention comprises at least one component derived from a GalSafe^{®} ungulate.

The medical product comprises a biologic.

A medical device is disclosed having at least one component derived from a GalSafe^{®} mammal.

In one instance of the disclosure, the medical device is a selected from the group consisting of bone fillers, dental implants or collagen fillers.

In one instance of the disclosure, the medical device is an injectable material comprising collagen for use in soft tissue augmentation.

The biologic is a cardiovascular implant and more particularly, a heart valve wherein the heart valve is not characterized by premature degradation. In a particular embodiment, the heart valve is suitable for clinical use about 10, about 11, about 12, about 13, about 14 or about 15 years or more after implantation.

Collagen is disclosed derived from a GalSafe^{®} mammal. The collagen may be, for example, type I collagen.

Gelatin is disclosed derived from a GalSafe^{®} mammal. The gelatin may be used as an ingredient, for example, in a food product, cosmetic product or medical product.

Reagents or proteins derived from a GalSafe^{®} mammal are disclosed for use in cell culture.

Reagents or proteins derived from a GalSafe^{®} mammal are disclosed for use in producing antibodies for human therapeutics.

Growth factors, serum, or serum proteins derived from a GalSafe^{®} mammal are disclosed, are such as albumin, for use in cell culture are provided.

Textile products are disclosed that contain at least one component derived from a GalSafe^{®} mammal.

Disclosed is a method of preventing or reducing the risk or severity of an allergic reaction in a subject in need thereof, comprising providing a composition disclosed herein to the subject in need thereof, thereby preventing or reducing the risk of severity of an allergic reaction in a subject in need thereof.

In a particular instance of the disclosure, the composition is provided in the form of a food product, food additive, cosmetic product, cosmetic additive, medical product, medical device or textile product.

The subject in need thereof has α-Gal Syndrome (AGS). In one embodiment, the subject has previously been diagnosed with AGS by serum testing, patient history or a combination thereof.

In certain embodiments, the subjects has IgE antibodies directed to alpha 1, 3 galactosyltransferase.

In certain embodiments, the subject has IgG4 antibodies to alpha 1, 3 galactosyltransferase.

In a particular embodiment, the allergic reaction is a type I hypersensitivity selected from the group consisting of cutaneous, gastrointestinal, respiratory, general hypersensitivity or a combination thereof.

In certain embodiments, the allergic reaction is gastrointestinal hypersensitivity and the method disclosed herein prevents or reduces the severity of one or more symptoms selected from the group consisting of nausea, vomiting, abdominal pain or a combination thereof.

In certain embodiments, the allergic reaction is cutaneous hypersensitivity and the method disclosed herein prevents or reduces the severity of one of more symptoms selected from the group consisting of itching, redness, rash or the like.

In certain embodiments, the allergic reaction is respiratory hypersensitivity and the method disclosed herein prevents or reduces the severity of one of more symptoms selected from the wheezing, nasal congestion or the like.

In certain embodiments, the allergic reaction is general hypersensitivity and the method disclosed herein prevents or reduces one or more symptoms of anaphylaxis.

Disclosed is a method of manufacturing the composition disclosed herein, comprising (i) providing a non-primate mammal having reduced (e.g., lack of) expression of alpha 1,3 galactosyltransferase; (ii) deriving at least one component from the non-primate mammal; and (iii) optionally adding the at least one component to a matrix, thereby providing the composition disclosed herein. In additional instance the method of manufacturing is conducted in a facility that does not process animals or animal components that express alpha 1, 3 galactosyltransferase.

In a particular instance, the composition is provided in the form of a food product, food additive, cosmetic product, cosmetic additive, medical product, medical device or textile product.

In certain instances, the composition and/or the product are manufactured in a facility that does not process animals or animal components that express alpha 1, 3 galactosyltransferase.

Herein, the terminologies "embodiment" and "embodiments" are to be construed as embodiment(s) of the invention only in as far as they fall within the scope of the appended claims. The references to methods of treatment of the human or animal body by surgery or therapy herein are to be interpreted as references to at least a medical product comprising a biologic that is derived from a transgenic ungulate, wherein the biologic is a cardiovascular implant comprising a heart valve, as described herein for use in those methods.

### BRIEF DESCRIPTION OF THE FIGURES

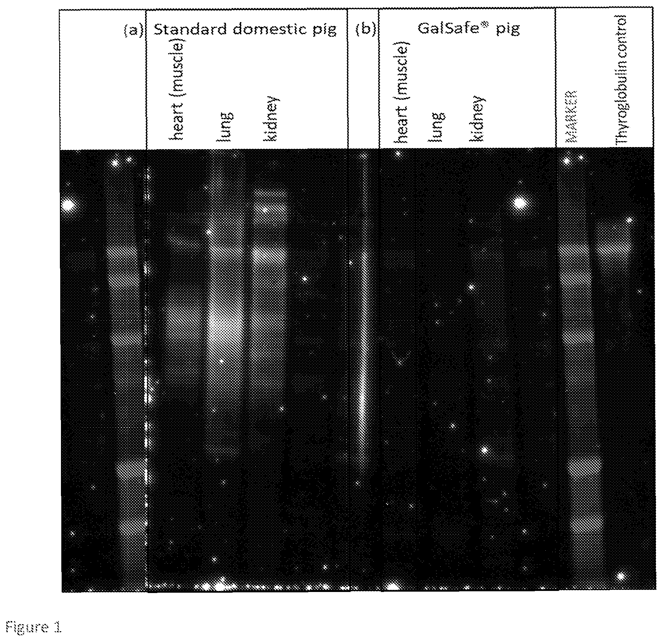

**FIG. 1:** Figure 1 depicts Western blot analysis of heart, lung, and kidney samples collected from a standard domestic breed "farm" pig and GalSafe^{®} pig. Proteins carrying the alpha-gal epitope were detected by commercially available mouse monoclonal anti-alpha Gal antibody(M86). The positive M86 signal specifies the alpha gal glycosylated proteins present on the domestic pig tissue samples. All the tissue types tested were positive (heart, lung kidney). In contrast, no alpha-gal signal was detected for the equivalent tissue samples collected from the GalSafe^{®} pig. Thus, demonstrating the absence of alpha-gal on GalSafe^{®} tissues. Commercially available porcine thyroglobulin serves as positive control.

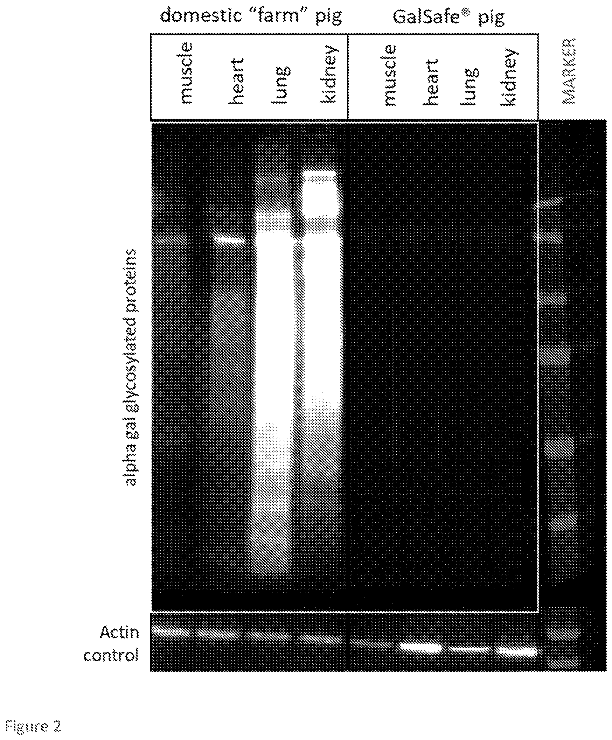

**FIG. 2.:** Figure 2 depicts Western blot analysis of Serum IgE-reactive proteins in porcine muscle, heart, lung and kidney tissue extracts. AGS patient plasma and healthy human control sera with specific IgE to alpha-gal glycosylated proteins in tissue lysates was detected using mouse anti-Human IgE antibodies. AGS patient plasma showed strong reactivity to standard domestic pig tissue and no reactivity towards GalSafe^{®} pig tissue lysates. The control sera did not show any reactivity towards any of the test samples. This data suggests porcine Gal-safe products may not trigger anaphylactic reaction in AGS patients. Actin serves as loading control indicating equal amount of total protein has been loaded in all lanes.

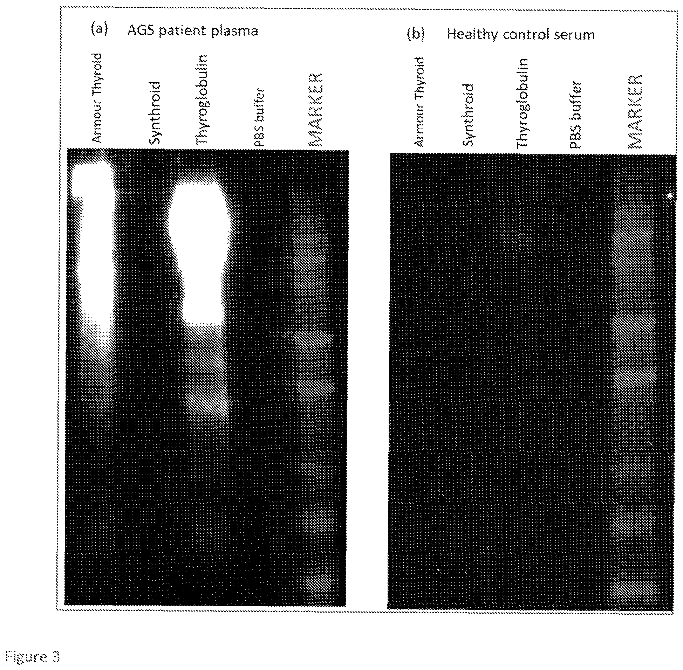

**FIG. 3:** Figure 3 depicts Western blot analysis of serum IgE reactive proteins in porcine derived or synthetic drugs. AGS patient plasma and healthy control serum with specific IgE to alpha- gal glycosylated proteins in porcine derived drug was detected using mouse anti-human IgE (horseradish peroxidase) HRP antibodies. AGS patient plasma shows strong reactivity to protein composition of the Armour^{®} Thyroid drug derived from standard domestic "farm" pig thyroid and shows no reaction to non-mammalian Synthroid^{®}.

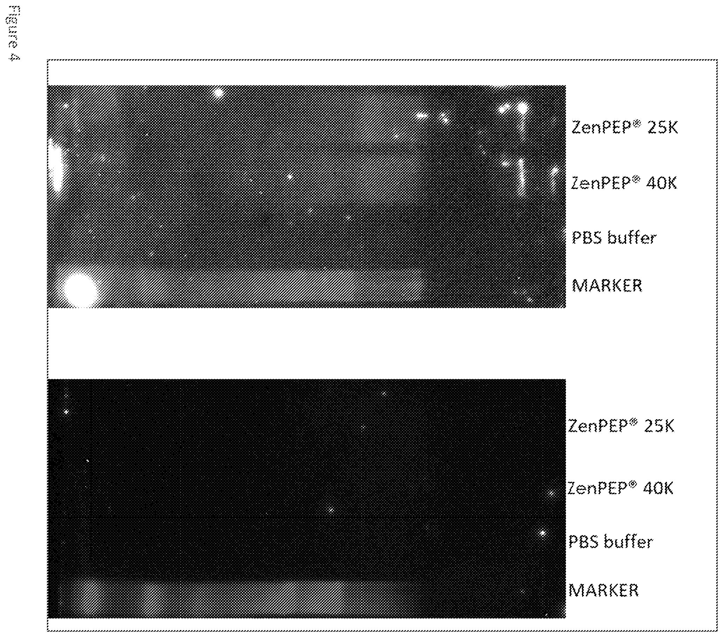

**FIG. 4:** Figure 4 depicts Western blot analysis of IgE-reactive proteins in ZENPEP^{®} 25K (Lipase 25,000, Protease 79,000, Amylase105,000 USP units) and ZENPEP^{®} 40K (Lipase 40,000, Protease 136,000, Amylase 218,000). AGS patient sera and healthy human control sera with specific IgE to alpha-Gal were analyzed for human IgE reactivity to alpha-gal glycosylated proteins and/or enzymes in ZENPEP^{®} 25K and 40K.

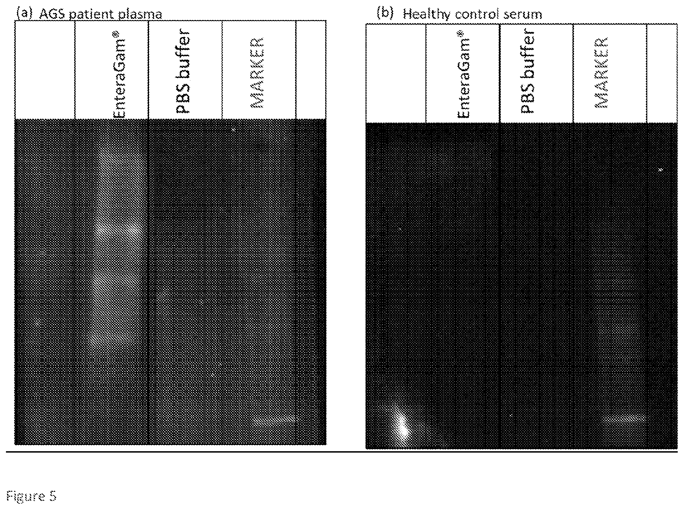

**FIG. 5:** Figure 5 depicts Western blot analysis of anti-gal serum IgE reactive proteins in the bovine derived medical product, EnteraGam^{®}. The reactivity of sera from health human controls (normal anti-gal IgE levels) and AGS patient plasma (high levels of anti-gal IgE antibodies) to alpha-gal glycosylated proteins in EnteraGam^{®} were tested using mouse anti-Human IgE HRP (horseradish peroxidase) secondary antibodies. AGS patient plasma showed strong reactivity to alpha-gal glycosylated proteins present in EnteraGam^{®}, whereas healthy human control serum did not show any reactivity to EnteraGam^{®}.

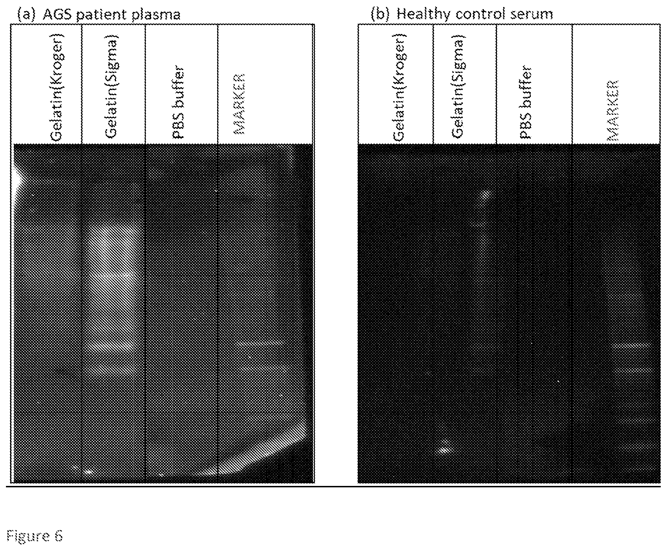

**FIG. 6:** Figure 6 depicts Western blot analysis of anti-gal IgE-reactive proteins in food grade gelatin (from grocery store) and gelatin derived from porcine skin (Sigma). AGS patient sera and healthy human control sera with specific IgE to alpha-Gal were analyzed for their reactivity to alpha-gal glycosylated proteins in gelatin products. AGS patient plasma showed strong reactivity towards gelatin from both sources. While, healthy human control sera did not show any reactivity towards gelatin products from either source.

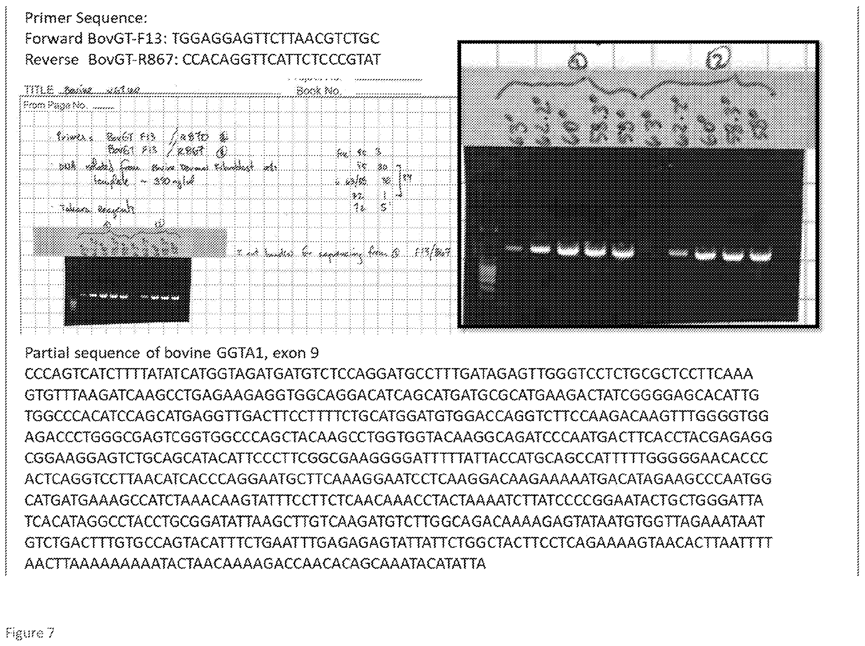

**FIG. 7:** Figure 7 depicts a partial sequence of exon 9 of Bovine GTTA1 gene and primers designed to amplified across the ~90 bp deletion site after CRISPR single guide RNA (sgRNA) were used.

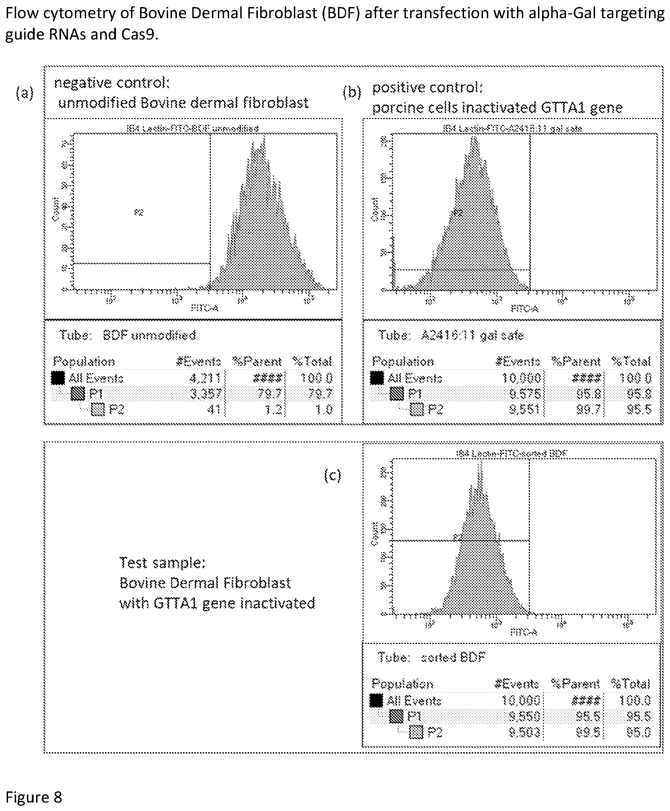

**FIG. 8:** Figure 8 depicts bovine dermal fibroblast cells transfected with a mixture of two guide RNAs and Cas-9 protein and subjected to flow cytometry. a) Unmodified bovine cells (GTTA1 gene active) stained with FITC IB4 lectin and sorted via flow cytometry served as a positive control and b) porcine cells (GTTA1 gene inactivated) and stained with FITC-IB4 lectin served as a negative control. c) the modified bovine dermal fibroblast stained with FITC-IB4 lectin were confirmed negative as an indicator that the bovine GTTA1 gene was inactivated in these cells.

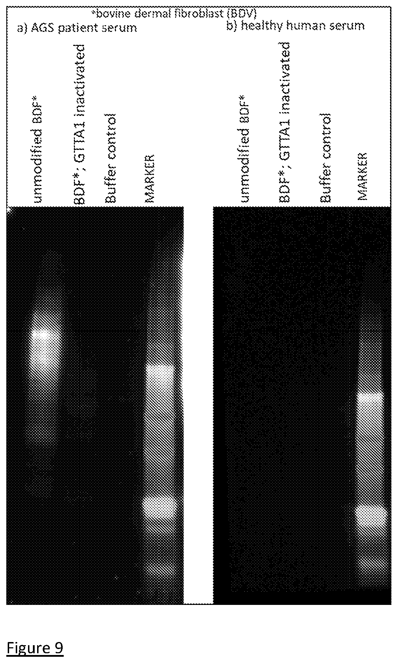

**FIG. 9:** Figure 9 depicts human IgE Immunoblot Western blot analysis of serum IgE-reactive proteins in bovine fibroblasts. AGS patient plasma and healthy human control sera with specific IgE to alpha-gal glycosylated proteins in cell lysates was detected using mouse anti-human IgE antibodies. (a) AGS patient serum showed strong reactivity to unmodified bovine dermal fibroblast cell lysate and no reactivity towards alpha-gal knockout bovine dermal fibroblast cell lysate. (b)The healthy human control sera did not show any reactivity towards unmodified and alpha-gal knockout bovine dermal fibroblast cell lysate.

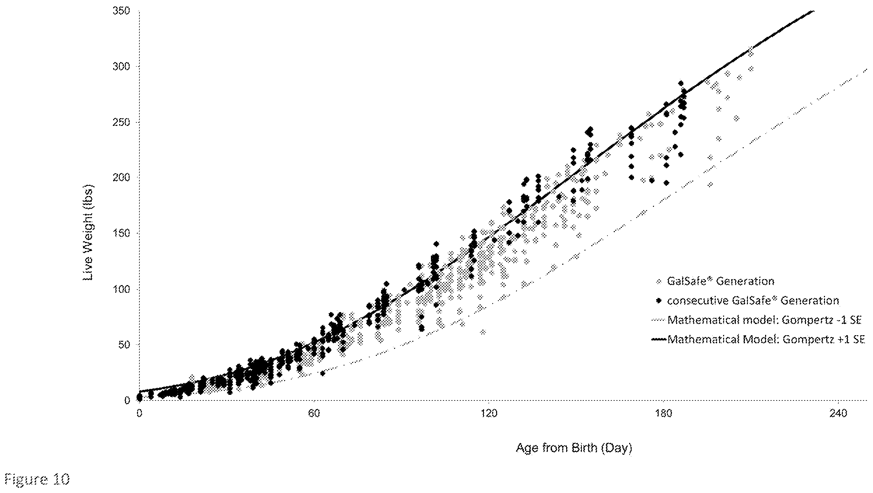

**FIG. 10:** Figure 10 depicts GalSafe^{®} live growth for consecutive generations compared to the live growth of standard domestic breed pigs predicted by the Compertz mathematical model. The live growth of GalSafe^{®} pigs fall within the normal range as predicted by the growth model.

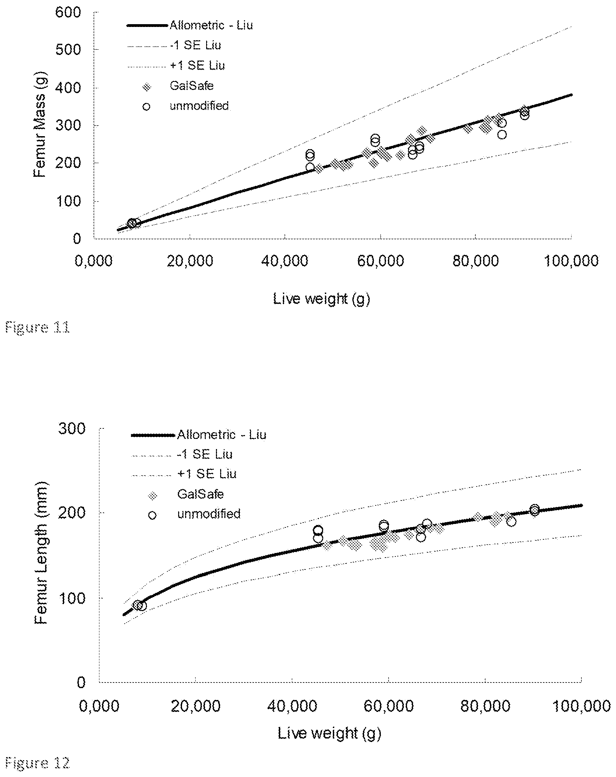

**FIG. 11:** Figure 11 depicts the mass of GalSafe^{®}(n=36) and standard domestic breed (n=17)pig femurs as a function of live body weight and compared to Liu's allometric predictions for pig femurs.
**FIG. 12:** Figure 12 depicts the Length of GalSafe^{®}(n=36) and standard domestic breed pig femurs (n=17) as a function of live body weight and compared to Liu's allometric predictions for pig femurs.

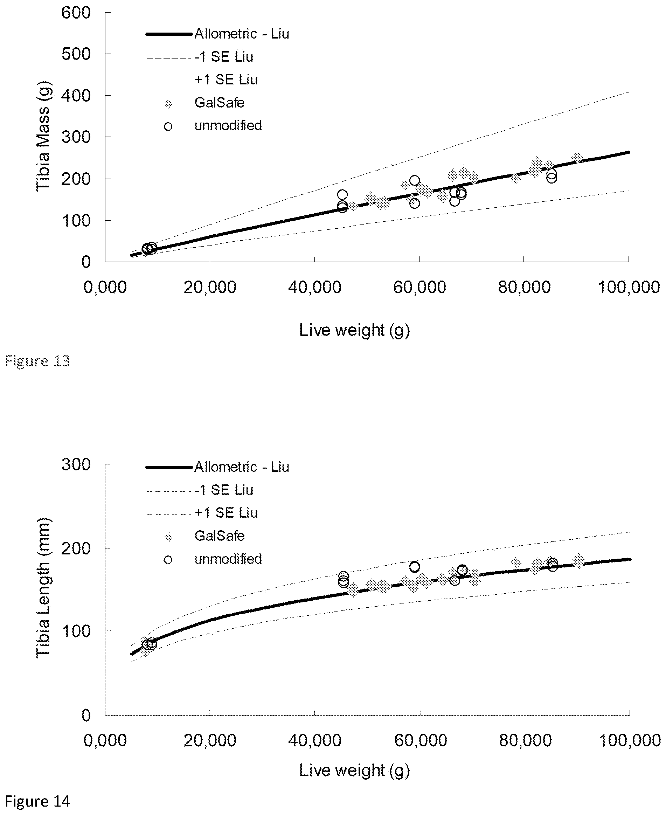

**FIG. 13:** Figure 13 depicts the mass of GalSafe^{®}(n=37) and standard domestic breed pig tibias (n=15) as a function of live body weight and compared to Liu's allometric predictions for pig tibias.
**FIG. 14:** Figure 14 depicts the length of GalSafe^{®}(n=37) and standard domestic breed pig tibias (n=15) as a function of live body weight and compared to Liu's allometric predictions for pig tibias.

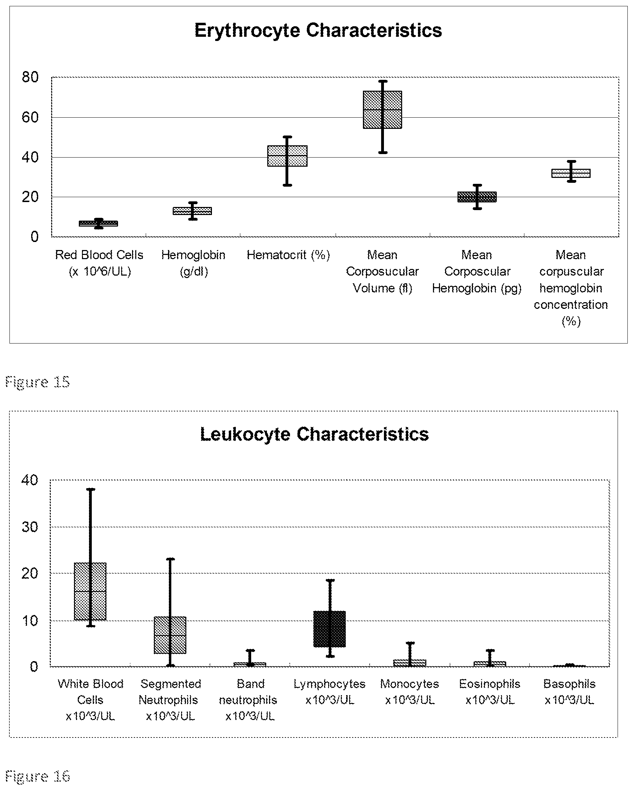

**FIG. 15:** Figure 15 depicts erythrocyte characteristics of GalSafe^{®} pigs (± 1 standard deviation; boxes) compared to literature reference values for standard domestic breed pigs (maximum/minimum; whiskers). Erythrocyte characteristics of blood derived from the GalSafe^{®} pigs fell within the normal range for pig.
**FIG. 16:** Figure 16 depicts leukocyte characteristics of GalSafe^{®} pigs (± 1 standard deviation; boxes) compared to literature reference values for standard domestic breed pigs (maximum/minimum; whiskers). The leukocyte characteristics of blood from GalSafe^{®} pigs fell within the normal range for pig. Of note, GalSafe^{®} leukocytes values fell toward the lower bound of the reference range. This is indicative of healthy animals that have low exposure to pathogens and may be due to the environmental containment practices that are in place for the GalSafe^{®} animals.

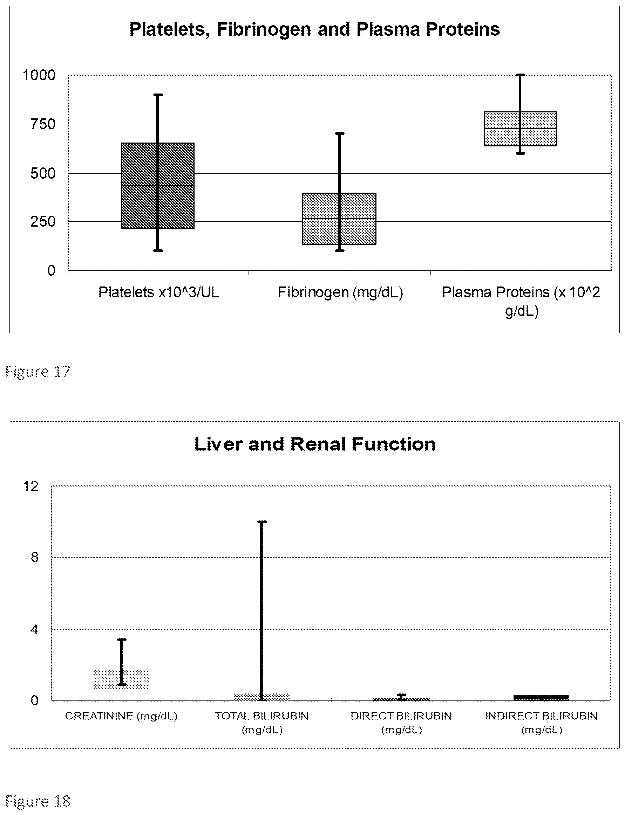

**FIG. 17:** Figure 17. Depicts platelets, fibrinogen and plasma proteins of GalSafe^{®} pigs (± 1 standard deviation; boxes) compared to literature reference values for standard domestic breed pigs (maximum/minimum; whiskers). These characteristics in blood fell within the normal range for commercial (standard domestic breed) pigs with details of age provided in appendix.
**FIG. 18:** Figure 18 depicts renal function and glucose via blood serum from GalSafe^{®} pigs (± 1 standard deviation; boxes) compared to reference values for standard domestic breed pigs (maximum/minimum; whiskers). Liver/Renal Function and Glucose of blood derived from the GalSafe^{®} pigs fell within the normal range for pig.

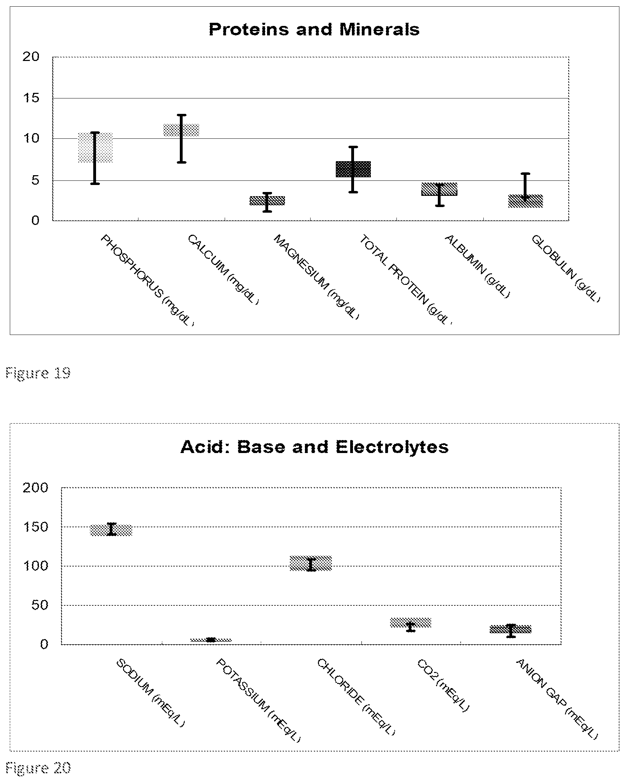

**FIG. 19:** Figure 19 depicts proteins and minerals via blood serum from GalSafe^{®} pigs (± 1 standard deviation; boxes) compared to reference values for standard domestic breed pigs (maximum/minimum; whiskers). The majority of proteins and minerals derived from the GalSafe^{®} animals fell within the normal range for pig.
**FIG. 20:** Figure 20 depicts acid: base and electrolytes via blood serum from GalSafe^{®} pigs (± 1 standard deviation; boxes) compared to reference values for standard domestic breed pigs (maximum/minimum; whiskers). The majority of acid: base and electrolytes characteristics derived from the GalSafe^{®} animals fell within the normal range for pig.

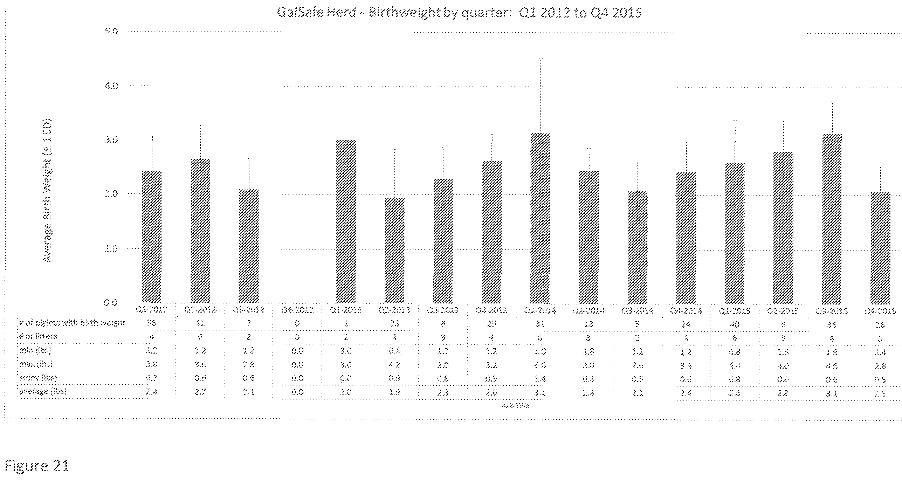

**FIG. 21:** Figure 21 depicts birthweights were collected from 321 piglets representing 58 litters. Birthweights have varied over each quarterly periods with individual pigs ranging from a low of 0.4lbs to a high of 6.6lbs.

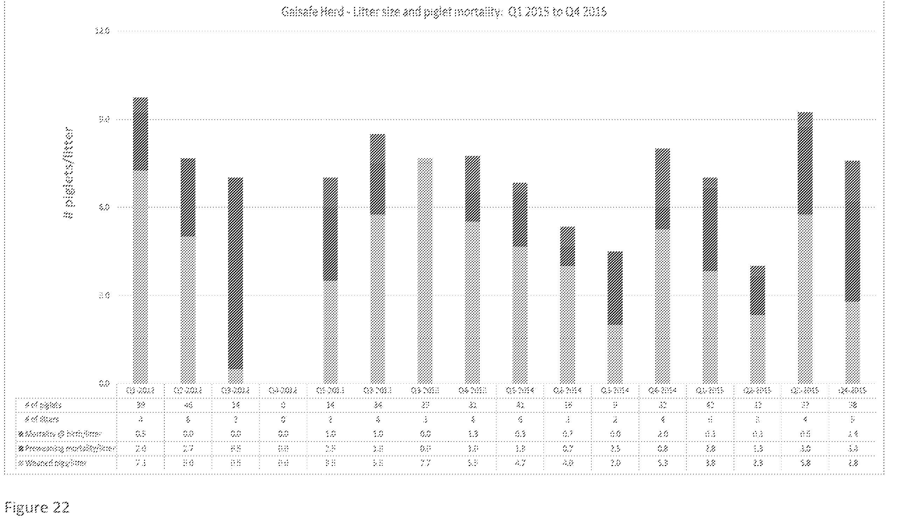

**FIG. 22:** Figure 22 depicts Farrowing statistics have varied over the 16 quarterly periods and the average quarterly litter size ranged from 4.0 to 9.8 pigs/litter. During these intervals mortality at birth (stillborn or mummies) ranged from 0 to 2 pigs/litter while mortality before weaning (death by mother, low viability, etc.) ranged from 0 to 3.4 piglets/litter.

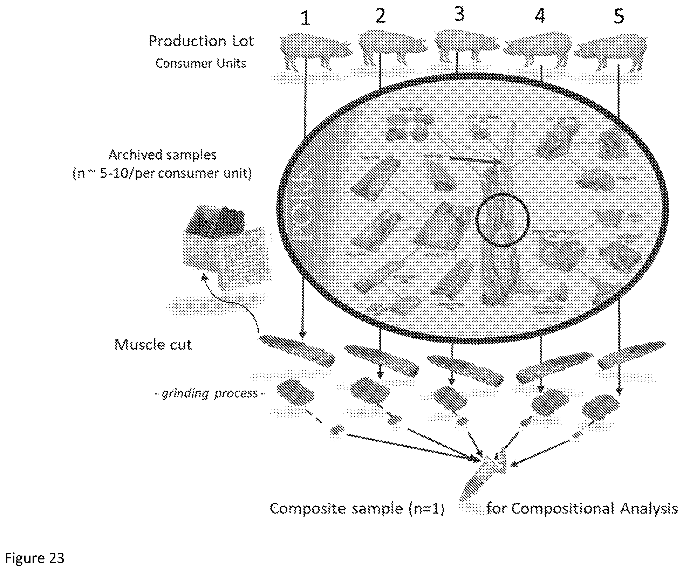

**FIG. 23:** Figure 23 depicts selection of primal cut for compositional analysis.

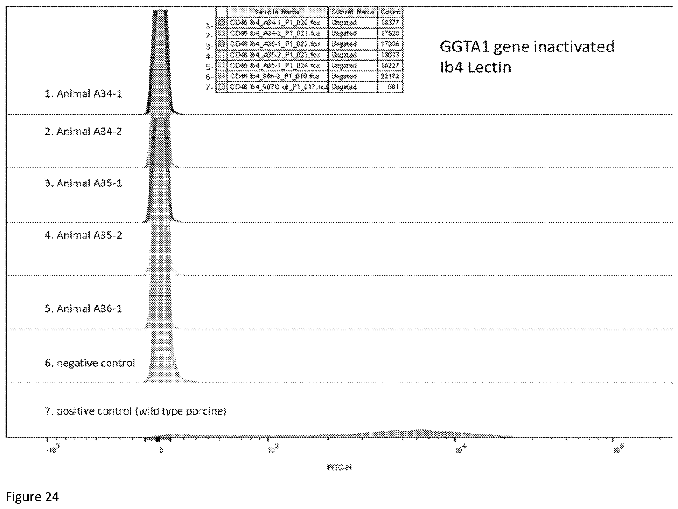

**FIG. 24:** Figure 24 depicts flow cytometry results: Porcine GTTA1 gene is inactivated. Alpha-1,3 galactosyltransferase (GGTA1) was knocked out by targeting exon 9 via homologous recombination with a gene-trapped neomycin resistant selectable marker (nptII, neoR). NeoR-expressing cells were selected by neomycin resistance and negative staining with IB4 lectin by fluorescence-activated cell sorting (FACS; flowcytometry). The results confirmed that animals A34-1; A34-2; A35-1; A35-2; A36-1 are GTTA1 "GalSafe^{®}" knockouts.

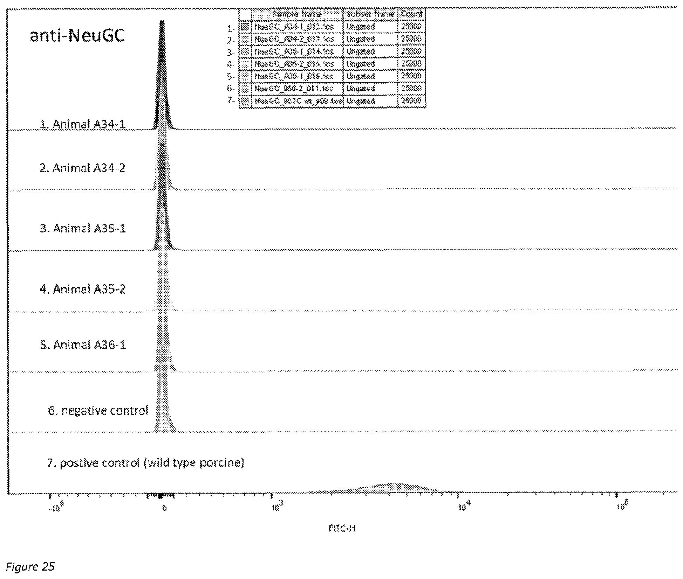

**FIG. 25:** Figure 25 depicts flow cytometry results: The CRISPR/Cas9 system was used to knock out the gene encoding for porcine cytidine monophosphate-N-acetyl neuraminic acid hydroxylase (CMAH) which catalyze synthesis of the xeno-antigens Neu5GC. Blood samples were collected from several presumptive TKO animals after birth and lymphocytes were separated and stained with anti-NeuGC antibodies. Negative (an animal confirmed to be a GGTA1 and CMAH knockout) and a positive (wild type porcine cells) controls were included. The negative staining results confirmed that animals A34-1; A34-2; A35-1; A35-2; A36-1 are CMAH knockouts.

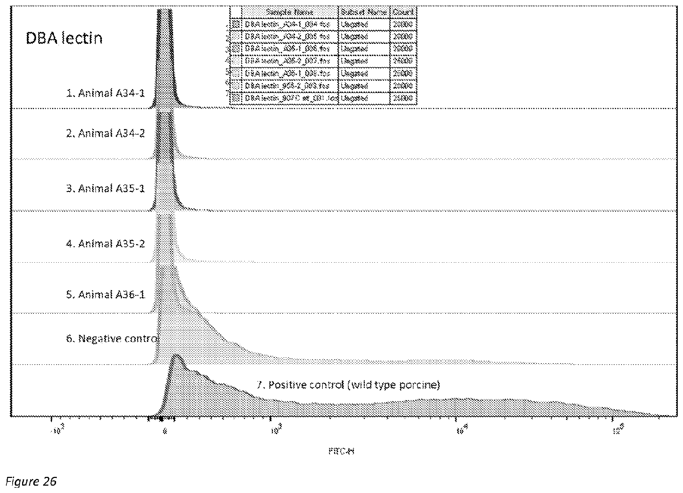

**FIG. 26:** Figure 26 depicts flow cytometry results: The CRISPR/Cas9 system was used to knock out the gene encoding for porcine β-1,4 N-galactosaminotransferase (β4GalNT2) which catalyze synthesis of the xeno-antigen Sd^{a}. Blood samples were collected from several presumptive TKO animals after birth and lymphocytes were separated and stained with biotinylated Dolichos Biflorus Agglutinin (DBA) lectin. Negative (an animal confirmed to be a GGTA1 and CMAH knockout) and a positive (wild type porcine cells) controls were included. The negative staining results confirmed that animals A34-1; A34-2; A35-1; A35-2; A36-1 are β4GalNT2 knockouts.

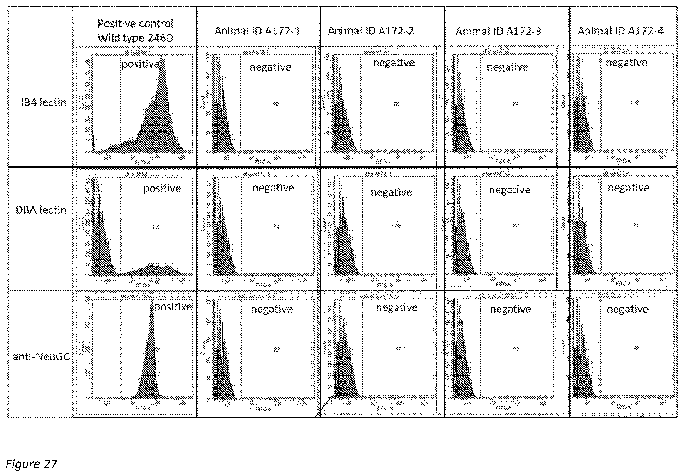

**FIG. 27:** Figure 27 depicts flow cytometry results: The porcine alpha-1,3 galactosyltransferase (GGTA1) gene was inactivated "knockout" by targeting exon 9 via homologous recombination with a gene-trapped neomycin resistant selectable marker (nptII, neoR). Further modifications were done using the CRISPR/Cas9 system to knock out 1) the gene encoding porcine cytidine monophosphate-N-acetyl neuraminic acid hydroxylase (CMAH) which catalyze synthesis of the xeno-antigens Neu5GC; and 2) porcine β-1,4 N-galactosaminotransferase (β4GalNT2) which catalyze synthesis of the xeno-antigen Sd^{a}. Blood samples were collected from several presumptive TKO animals after birth and lymphocytes were separated and stained with biotinylated IB4 lectin; Dolichos Biflorus Agglutinin (DBA) lectin, and anti-NeuGC antibodies. A positive (wild type porcine cells) control was included. The negative staining results confirmed that animals A172-1; A172-2; A172-3 and A172-4 are triple knock out (TKO) pigs with a GalSafe^{®} CMAH B4 KO genotype

### DETAILED DESCRIPTION

Disclosed are compositions and methods for preventing and methods for use in preventing or reducing the risk or severity of an allergic reaction particularly an anaphylactic reaction, in a subject in need thereof.

Disclosed are transgenic animals (e.g., ungulates) having reduced expression of alpha 1, 3 galactosyltransferase that are particularly useful as a source of components that can be used (as such, or as further processed) as food products, food ingredients, drugs, biologics, medical devices, bio-actives, cosmetic products, cosmetic ingredients and the like. Also disclosed are components derived from such animals.

### 1. Definitions

The term "administering" or "providing", as used herein, refers to any suitable route of administration. In some embodiments, the administering includes, but is not limited to, oral, nasal, topical, intravenous, subcutaneous, intramuscular, intraperitoneal, sublingual, ocular, vaginal, rectal , pulmonary, and transdermal administration.

The term "acellular", as used herein, refers to means materials and mixtures with significantly reduced intact cell content.

The term "allergic reaction" as used herein, refers to a hypersensitivity disorder of the immune system in which a person's immune system reacts to a normally harmless substance (an allergen), such as from the environment. Allergic reactions can range in sensitivity from mild to severe.

The term "α-Gal Syndrome " or "AGS", as used herein, refers to a human disorder characterized by the presence of IgE antibodies (and in some cases IgG4 subtype antibodies) to alpha-gal and delayed or acute type I allergic reaction to the carbohydrate galactose-alpha-1,3-galactose (alpha-gal) after exposure and/or consumption of products of mammalian origin. AGS is also known as mammalian meat allergy (MMA), red meat allergy syndrome or simply meat allergy. Unlike traditional IgE-mediated food hypersensitivities, the reactions involving α-gal and mammalian meat typically are delayed by at least 2 h, i.e., "delayed-immediate" reactions.

The term "anaphylaxis" or "anaphylactic reaction", as used herein, refers to a serious allergic reaction that is rapid in onset and may cause death. It can involve multiple symptoms, as well as several organ systems, including the skin, respiratory and gastrointestinal tracts, and cardiovascular system. It involves the release of mediators from mast cells, basophils, and recruited inflammatory cells.

The term "antigen", as used herein, refers to a molecule that elicits production of an antibody (i.e., a humoral response) and/or an antigen-specific reaction with T-cells (i.e., a cellular response) in an animal.

The term "allergen", as used herein, refers to any chemical capable of causing an immune system response in a subject.

The term "biologic" as used herein refers to an agent that is derived from a living system that may or may not be altered.

The term "breeding herd", as used herein, refers to a group of transgenic animals generated by the methods disclosed herein. In some embodiments, genetic modifications may be identified in animals that are then bred together to form a herd of animals with a desired set of genetic modifications (or a single genetic modification). See WO 2012/112586; PCT/US2012/025097. These progeny may be further bred to produce different or the same set of genetic modifications (or single genetic modification) in their progeny. This cycle of breeding for animals with desired genetic modification(s) may continue for as long as one desires. "Herd" in this context may comprise multiple generations of animals produced over time with the same or different genetic modification(s). "Herd" may also refer to a single generation of animals with the same or different genetic modification(s).

The term "catarrhines", as used herein, refers to primates of a group that comprises the Old World monkeys, gibbons, great apes, and humans.

The term "carbohydrate epitope" refers to carbohydrates (e.g., glycolipids, glycoproteins) having antigenic significance. In a particular embodiment, the carbohydrate epitope is galactose-α-1,3-galactose (α-Gal), a sugar chain commonly found as part of glycoproteins and glycolipids in mammals with the exception of higher apes.

The term "cells", as used herein, refers to a cell population. The cells may be wild-type or recombinant.

The term "cell culture" or "cell culture technique" or "cell culture process" refers to a method and conditions suitable for the survival and/or growth of all cell types, differentiated or and/in a undifferentiated of the cells.

The term "cell culture medium" or "medium", as used herein, refers to a solution containing nutrients which are required for growing animal cells, such as mammalian cells. Typically, these solutions provide essential and non-essential amino acids, vitamins, energy sources, lipids, albumin, and trace elements required by the cell for minimal growth and/or survival. The solution can also contain components that enhance growth and/or survival above the minimal rate, including hormones and growth factors. The solution is formulated to a pH and salt concentration optimal for cell survival and proliferation. interchangeably herein to refer to the constituents that make up a cell culture medium.

The term "collagen," as used herein, refers to the major insoluble fibrous protein in the extracellular matrix and in connective tissue. There are numerous types of collagen, the most common being type I, II and III. Type I (skin, tendon, vasculature, organs, bone (main component of the organic part of bone); Type II: cartilage(main collagenous component of cartilage); Type III: reticulate (main component of reticular fibers), commonly found alongside type I; Type IV: forms basal lamina, the epithelium-secreted layer of the basement membrane; and Type V: cell surfaces, hair, and placenta. All types of collagen contain a repeating Gly-Pro-X sequence and fold into a characteristic triple-helical structure. Fibrous type collagen molecules (e.g., types I, II, and III) assemble into fibrils that are stabilized by covalent aldol cross-links.

The term "consumer product", as used herein, refers to products of common or daily use, ordinarily bought by individuals or households for private consumption. Products intended for use (e.g., administration) by professionals such as medical or dental professionals are not considered consumer products.

As used herein, the term "CRISPR" or "Clustered Regularly Interspaced Short Palindromic Repeats" or "SPIDRs" or "SPacer Interspersed Direct Repeats" refers to a family of DNA loci that are usually specific to a particular bacterial species. The CRISPR locus comprise a distinct class of interspersed short sequence repeats (SSRs) that were recognized in E. coli (Ishino et al., J. Bacteriol., 169:5429-5433 [1987]; and Nakata et al., J. Bacteriol., 171:3553-3556 [1989]).. CRISPR/Cas molecules are components of a prokaryotic adaptive immune system that is functionally analogous to eukaryotic RNA interference, using RNA base pairing to direct DNA or RNA cleavage. Directing DNA DSBs requires two components: the Cas9 protein, which functions as an endonuclease, and CRISPR RNA (crRNA) and tracer RNA (tracrRNA) sequences that aid in directing the Cas9/RNA complex to target DNA sequence (Makarova et al., Nat Rev Microbiol, 9(6):467-477, 2011). The modification of a single targeting RNA can be sufficient to alter the nucleotide target of a Cas protein. In some cases, crRNA and tracrRNA can be engineered as a single cr/tracrRNA hybrid to direct Cas9 cleavage activity (Jinek et al., Science, 337(6096):816-821, 2012). The CRISPR/Cas system can be used in bacteria, yeast, humans, and zebrafish, as described elsewhere (see, e.g., Jiang et al., Nat Biotechnol, 31(3):233-239, 2013; Dicarlo et al., Nucleic Acids Res, doi:10.1093/nar/gkt135, 2013; Cong et al., Science, 339(6121):819-823, 2013; Mali et al., Science, 339(6121):823-826, 2013; Cho et al., Nat Biotechnol, 31(3):230-232, 2013; and Hwang et al., Nat Biotechnol, 31(3):227-229, 2013).

The term "degradation," as used herein, refers to structural deterioration, for example, as the result of collagen disruption due to, for example, calcification or inflammation.

The term "excipient", as used herein, refers to any inactive substance incorporated into a pharmaceutical composition as a carrier for an active pharmaceutical ingredient. In one embodiment, at least one pharmaceutically acceptable excipient is selected from the group consisting of polymers, resins, plasticizers, fillers, lubricants, diluents, solvents, co-solvents, buffer systems, surfactants, preservatives, sweetening agents, flavoring agents, pharmaceutical grade dyes or pigments, viscosity agents and combinations thereof.

The term "expression", as used herein, refers to the process by which a polynucleotide is transcribed from a DNA template (such as into and mRNA or other RNA transcript) and/or the process by which a transcribed mRNA is subsequently translated into peptides, polypeptides, or proteins. Transcripts and encoded polypeptides may be collectively referred to as "gene product." If the polynucleotide is derived from genomic DNA, expression may include splicing of the mRNA in a eukaryotic cell.

The term "food additive", as used herein, refers to a substance not typically consumed as a food itself or considered an ingredient, as such, which is intentionally added to a food product in order to improve the manufacturing, processing, preparation, transportation or storage of the food product. Food additives may make a given food product safer or improve one or more of its properties, e.g., taste or appearance. Representative, non-limiting food additives include preservatives, anti-oxidants, acidulants, enzymes, emulsifiers, polysaccharides, flavor enhancers, thickeners, bulking agents, carriers, humectants, sequestrants and the like.

The term "food allergy," as used herein, refers to an adverse reaction to food mediated by an immunologic mechanism, involving specific IgE (IgE-mediated), cell-mediated mechanisms (non-IgE-mediated) or both IgE- and cell-mediated mechanisms (mixed IgE- and non-IgE-mediated).

The term "GalSafe^{®}", as used herein, refers to a mammal that lacks expression of alpha 1, 3 galactosyltransferase. The non-human primate animal alpha 1, 3 galactosyltransferase described herein is, in one embodiment, a GalSafe^{®} mammal but in other embodiments, a nonhuman primate animal that lacks expression of alpha 1, 3 galactosyltransferase and also has one or more genetic modifications including, without limitation, one or more gene additions or deletions. In a particular embodiment, the non-human primate animal that lacks expression of alpha-gal has one, two, three, four, five, six, seven or eight or more additional genetic modifications.

The term "gelatin" as used herein, refers to a mixture of peptides and proteins produced by partial hydrolysis of collagen extracted from the skin, bones, and connective tissues of animals such as domesticated cattle and pigs. Gelatin is typically between about 98-99% protein. It is used in the preparation of foods, cosmetics and medicines.

The term "gene editing", as used herein, refers a type of genetic engineering in which DNA is inserted, replaced, or removed from a genome using gene editing tools. Examples of gene editing tools include, without limitation, zinc finger nucleases, TALEN and CRISPR.

The term "gene knock-out", as used herein, refers to a genetic modification resulting from the disruption of the genetic information encoded in a chromosomal locus.

The term "gene knock-in", as used herein, is a genetic modification resulting from the insertion or replacement of the genetic information encoded in a chromosomal locus with a different DNA sequence.

The term "genetic modification" as used herein refers to one or more alterations of a nucleic acid, e.g., the nucleic acid within an organism's genome. For example, genetic modification can refer to alterations, additions (e., gene knock-ins), and/or deletion of genes (e.g., gene knock-outs).

The term "glycoprotein", as used herein refers to a polypeptide or protein coupled to at least one carbohydrate moiety, e.g., a polysaccharide or an oligosaccharide, that is attached to the protein via an oxygen-containing or a nitrogen-containing side chain of an amino acid residue, e.g., a serine or threonine residue ("O-linked") or an asparagine residue ("N-linked"). The term "glycan" refers to a polysaccharide or an oligosaccharide, e.g., a polymer comprised of monosaccharides. Glycans can be homo- or heteropolymers of monosaccharide residues, and can be linear or branched.

The term "growth factor", as used herein, refers to proteins that function as growth stimulators (mitogens) and/or growth inhibitors, stimulate cell migration, act as chemotactic agents, inhibit cell migration, inhibit invasion of tumor cells, modulate differentiated functions of cells, involved in apoptosis, involved in angiogenesis and promote survival of cells without influencing growth and differentiation.

The term "hormone", as used herein, refers to a signaling molecule produced by the endocrine glands (as well as testes in men and ovaries in women). The major endocrine glands are the pituitary, pineal, thymus, thyroid, adrenal glands, and pancreas. Chemically, hormones may be classified as either proteins or steroids.

The term "IgE mediated disease", as used herein, refers to a disease or disorder that is mediated, at least in part, by an increase in the levels of IgE as that term is used herein.

The term "mammalian cell line", as used herein, refer to cell lines derived from mammals that are capable of growth and survival when placed in either monolayer culture or in suspension culture in a medium containing the appropriate nutrients and growth factors. Typically, the cells are capable of expressing and secreting large quantities of a particular protein of interest (typically a recombinant protein) into the culture medium, and are cultured for this purpose. However, the cells may be cultured for a variety of other purposes as well, and the scope of this compositions and methods disclosed herein is not limited to culturing the cells only for production of recombinant proteins.

The term "microorganism", as used herein, refers to any type of unicellular organism, including prokaryotic organisms like bacteria, and eukaryotic organisms like yeasts.

The term "phenotype", as used herein, refers to the set of observable characteristics or traits of an organism (e.g., a non-primate mammal) resulting from the interaction of its genotype with the environment.

The term "polypeptide" or "protein", as used herein, refers to sequential chain of amino acids linked together via peptide bonds. The term is used to refer to an amino acid chain of any length, but one of ordinary skill in the art will understand that the term is not limited to lengthy chains and can refer to a minimal chain comprising two amino acids linked together via a peptide bond. If a single polypeptide is the discrete functioning unit and does require permanent physical association with other polypeptides in order to form the discrete functioning unit, the terms "polypeptide" and "protein" as used herein are used interchangeably. If discrete functional unit is comprised of more than one polypeptide that physically associate with one another, the term "protein" as used herein refers to the multiple polypeptides that are physically coupled and function together as the discrete unit.

The term "reduced", as used herein with reference to alpha 1, 3 galactosyltransferase, refers to a decrease in amount up to and including lack of any expression of alpha 1, 3 galactosyltransferase. The expression of functional alpha Gal may be reduced by, for example, by at least about 5%, about 10%, about 20%, about 30%, about 40%, about 50%, about 60%, about 70%, about 80%, about 90%, about 95% or about 100%.

The term "reduced allergic (or anaphylactic) reaction", as used herein, refers to a decrease in the clinical symptoms that are associated with exposure to an allergen (or anaphylactic allergen), when exposure occurs via the route through which an individual would naturally encounter the allergen (or anaphylactic allergen), e.g., via oral, cutaneous, respiratory, gastrointestinal, ocular, nasal, aural, etc. exposure or via a subcutaneous injection (e.g., in the form of a bee sting) depending on the nature of the allergen (or anaphylactic allergen).

The term "therapeutically effective amount", as used herein, refers to that amount of the biomaterial or composition disclosed herein that is effective for producing some desired therapeutic effect, e.g., treating (i.e., preventing and/or ameliorating) allergic reaction in a subject at a reasonable benefit/risk ratio applicable to any medical treatment. In one embodiment, the therapeutically effective amount is enough to reduce or eliminate at least one symptom. One of skill in the art recognizes that an amount may be considered therapeutically effective even if the allergic reaction is not totally eradicated but improved partially. For example, a symptom from the allergic reaction may be partially reduced or completed eliminated, and so forth.

The term "transgene" is a gene or genetic material that has been transferred from one organism to another. When a transgene is transferred into an organism, the organism can then be referred to as a transgenic organism. Typically, the term describes a segment of DNA containing a gene sequence that has been isolated from one organism and is introduced into a different organism. This non-native segment of DNA may retain the ability to produce RNA or protein in the transgenic organism, or it may alter the normal function of the transgenic organism's genetic code. In general, the DNA is incorporated into the organisms germ line. For example, in higher vertebrates this can be accomplished by injecting the foreign DNA into the nucleus of a fertilized ovum or via somatic cell nuclear transfer where a somatic cell, with the desired transgene(s) is incorporated into the host genome, is transferred to an enucleated oocyte and results in live offspring after transplantation into a surrogate mother. When inserted into a cell, a transgene can be either a cDNA (complementary DNA) segment, which is a copy of mRNA (messenger RNA), or the gene itself residing in its original region of genomic DNA. The transgene can be a genome sequence, in particular when introduced as large clones in BACs (bacterial artificial chromosomes) or cosmid, or could be a form of "minigene" often characterized by a combination of both genomic DNA (including intron regions, e.g. intron 1), 5' or 3' regulatory regions, along with cDNA regions. Transgene "expression" in the context of the present specification, unless otherwise specified, means that a peptide sequence from a non-native nucleic acid is expressed in at least one cell in a host. The peptide can be expressed from a transgene that is incorporated in the host genome. A transgene can comprise a polynucleotide encoding a protein or a fragment (e.g., a functional fragment) thereof. A fragment (e.g., a functional fragment) of a protein can comprise at least or at least about 5%, 10%, 20%, 30%, 40%, 50%, 60%, 70%, 80%, 90%, 95%, or 99% of the amino acid sequence of the protein. A fragment of a protein can be a functional fragment of the protein. A functional fragment of a protein can retain part or all of the function of the protein.

The term "treating", as used herein refers to treatment of existing disease and prophylactic treatment of those at risk of developing the disease.

The term "undesirable immune response", as used herein, refers to any immune response, activity or function that is greater or less than desired or physiologically normal. An undesirable immune response, function or activity can be a normal response, function or activity. Thus, normal immune responses so long as they are undesirable, included within the meaning of these terms.

The term "ungulate", as used herein, refers to hoofed mammals. Artiodactyls are eventoed (cloven-hooved) ungulates, including antelopes, camels, cattle, deer, goats, pigs, and sheep. Perissodactyls are odd toes ungulates, which include horses, zebras, rhinoceroses, and tapirs. The term ungulate as used herein refers to an adult, embryonic or fetal ungulate animal.

The invention provides medical products as defined by the claims.

### I. Compositions

Disclosed are compositions for preventing or reducing the risk or severity of an allergic reaction in a subject in need thereof, wherein the compositions contain at least one component derived from a non-primate mammal that has reduced expression of galactose-α1,3-galactose (alpha-gal) or lacks expression of alpha-gal.

Most mammals, including all food producing mammalian species, such as cows, pigs, goats, sheep, lamb, and rabbits express the galactose-α1,3-galactose (alpha-gal) disaccharide sugar on cells and tissue surfaces. Alpha-gal expression results from the catalytic activity of the α1,3-galactosyltransferase enzyme encoded by the glycoprotein α1,3-galactosyltransferase gene (GGTA1).1-3, 5 Certain mammalian species, such as catarrhines (humans, apes, and Old World monkeys), do not have a functional GGTA1 gene and correspondingly do not express alpha-gal.

The non-primate mammal may be any age or stage of development, fetal, prenatal, neonatal, immature, or fully mature animal. In certain instances, the non-primate mammal may be a mouse, hamster or rabbit. In a particular instance, the non-primate mammal can serve as a hypoallergenic companion animal (hamster, cat, dog, horse, pig, goat, sheep).

In certain instances, the composition may contain components from more than one type of animal, e.g., a component derived from a cow and a component derived from a pig, wherein both the cow and the pig have reduced or no expression of alpha 1, 3 galactosyltransferase.

In certain instances, the non-primate mammal is a porcine or bovine animal which lacks any expression of functional alpha 1,3 galactosyltransferase as the result of genetic modification or otherwise. Optionally, the porcine or bovine animal incorporates at least one additional genetic modification. modifications (e.g., gene knockouts, gene knock-ins, gene replacements, point mutations, deletions, insertions, or substitutions (i.e., of genes, gene fragments or nucleotides), large genomic insertions or combinations thereof). In a particular instance, the gene knockout is CMAH, Beta4GalNT2 or a combination thereof.

Genetically modified pigs have been produced that lack the alpha 1,3-galactose (Gal) epitope. In 2003, Phelps et al. (Science, 2003, 299:411-414) reported the production of the first live pigs lacking any functional expression of alpha.GT (GTKO), which represented a major breakthrough in xenotransplantation (see also PCT publication No. WO 04/028243 to Revivicor, Inc. and PCT Publication No. WO 04/016742 to Immerge Biotherapeutics, Inc.). Subsequent studies have shown that organ grafts from GTKO pigs do not undergo HAR (Kuwaki et al., Nat Med. 2005 January; 11(1):29-31, Yamada et al., Nat Med. 2005 January; 11(1):32-4). [ Add tissue products case]

Disclosed herein are compositions containing at least one component derived from non-primate mammals that do not contain alpha-gal (GalSafe^{®}). In one instance, the GalSafe^{®} mammals do not exhibit any health or compositional differences compared with standard domestic, nonengineered mammals (wild-type). In particular instances, the mammal is an ungulate, such as a pig or a cow. In one instance, the tissue of the GalSafe^{®} mammal has similar or the same morphology, composition, mechanics, bioactive molecules, hematologic, biochemical, and/ or coagulation parameters as the wild-type mammal. In certain instances, the composition, mechanics, bioactive molecules, hematologic, biochemical, and/ or coagulation parameters are within at least 5%, at least 10% or at least 15% of the wildtype mammal.

In one instance, the tissue of the GalSafe^{®} mammal has similar or the same growth, health and/ or reproduction capabilities as the wild-type mammal. In certain instances, the growth, health and/ or reproduction capabilities are within at least 5%, at least 10% or at least 15% of the wild type mammal.

In particular instances, skeletal growth is the same or similar as previously established allometric skeletal growth models for wild type mammals and/ or changes in bone morphology with age are consistent in appearance to published descriptions of bone histology from wild type mammals of comparable age.

In further instances, modification of the GalSafe^{®} mammal does not cause any direct, unintended or indirect toxicity to the recipient of a consumable, medical product, cosmetic product or any other composition disclosed herein obtained from the GalSafe^{®} mammal. In other instances, the genotypic modification of the GalSafe^{®} mammal has been cleared by the United States Food and Drug Agency (FDA) for consumption, cosmetic or medical treatment of a human. In other instances, the genotypic modification of the GalSafe^{®} mammal does not cause any direct, unintended or indirect toxicity to the GalSafe^{®} mammal. The genotypic modification can be a targeted insertion of a selectable marker gene. The selectable marker gene can be an antibiotic resistance gene. Antibiotic resistance genes can be neomycin resistance genes, please include more if applicable. In particular instances, the antibiotic of the antibiotic resistance gene is not used for the treatment of human diseases. In other instancesthe antibiotic of the antibiotic resistance gene is not prescribed for oral administration to humans. In another instancesthe antibiotic of the antibiotic resistance gene is not prescribed for intravenous administration by humans.

In other instance, proteins and mineral levels in blood serum from GalSafe^{®} mammals are the same or similar compared to reference values for wild-type mammals. In certain instances, proteins and mineral levels in blood serum from GalSafe^{®} mammals are within at least 5%, at least 10% or at least 15% of the wildtype mammal. In other instances, the protein and mineral levels evaluated are phosphorous, calcium, magnesium, total protein, albumin and/ or globulin.

In other instance, acid: base and electrolyte levels in blood serum from GalSafe^{®} mammals are the same or similar compared to reference values for wildtype mammals. In certain instances, acid: base and electrolyte levels in blood serum from GalSafe^{®} mammals are within at least 5%, at least 10% or at least 15% of the wildtype mammal. In other instances, the acid: base and electrolyte levels evaluated are sodium, potassium, chloride, anion gap, and/ or carbon dioxide.

In additional instances, the nutrient content in tissues or other consumables from GalSafe^{®} mammals are the same or similar compared to reference values for wildtype mammals. In certain instances, nutrient levels in in tissues or other consumables from GalSafe^{®} mammals are within at least 5%, at least 10% or at least 15% of the wildtype mammal. In other instances, the nutrient levels in the GalSafe^{®} mammals can be total calories, total fat cholesterol, sodium, total carbohydrate, fiber, sugar, protein, and/ or vitamins and minerals, such as vitamin A, vitamin C, vitamin D, calcium, iron.

The as at least one component may vary. In one instance, the compositions comprise non-viable/acellular biomaterials derived from non-primate mammal having reduced expression of alpha 1, 3 galactosyltransferase (alpha-gal) or more particularly, lacking expression of alpha-gal. In a particular instance, the compositions comprise proteins, lipids or cellular materials derived from non-primate mammal having reduced expression of alpha 1, 3 galactosyltransferase (alpha-gal) or more particularly, lacking expression of alpha-gal.

In certain instances, the compositions are not intended for direct transplantation, as such.

The compositions disclosed herein are intended to be non-allergenic to humans, i.e., presenting a reduced risk of allergic reaction compared to conventional compositions in patients or populations sensitized to alpha-gal or previously diagnosed with alpha-gal syndrome (AGS).

The one or more components derived from the non-primate mammal for use in the compositions disclosed herein may be, for example, lipids, proteins, cells, tissues or a combination thereof.

The one or more components derived from the non-primate mammal for use in the compositions disclosed herein may be obtained from organs or tissues including but not limited to heart, lung, liver, kidney, pancreas, small and large intestine, stomach, bladder, mesentery, veins/arteries, lymphatic, nerves, thymus, hypothalamus, spleen, skin, bone, glands (pituitary, adrenal, thyroid, parathyroid, pineal), cartilage, tendon.

The composition may be, for example, provided in the form of a product, such as a product for human or veterinary use. In one instance, the composition is provided in the form of a product such as a consumer product (e.g., a food product, food ingredient, cosmetic product, cosmetic ingredient), a prescription or over-the-counter medical product (e.g., a drug, biologic, a product used in laboratory research, a product used in the production of a therapeutic agent (e.g., a bioactive). In each case, the composition contains one or more components derived from a non-primate mammal having reduced expression of expression of alpha 1,3 galactosyltransferase. In certain instances, the composition contains one or more components derived from a non-primate mammal lacking expression of expression of alpha 1,3 galactosyltransferase.

### A. Food Products

Disclosed herein are food products and food additives for preventing or reducing the risk of an allergic reaction in a subject in need thereof, such as subject previously diagnosed with α-Gal Syndrome (AGS).

A food product is disclosed that contains one or more components derived from a non-primate mammal having reduced expression of alpha 1, 3 galactosyltransferase or lacking expression of alpha 1, 3 gal. In one instance of the disclosure, the non-primate mammal is a cow, pig or sheep having reduced alpha-gal expression or lacking alpha-gal expression.

The food product may be meat, meat protein or a meat-byproduct derived from the non-primate mammal.

In one instance, the food product is a part cut directly from an animal (a "cut"). In a particular instance, the food product is a part cut from a cow, such as chuck, shank, brisket, rib, short plate, flank, loin, sirloin and round. Other parts that may be cut directly from the cow include the tongue, organs (e.g., kidney), neck or knuckle. In another particular instance, the food product is a part cut directly a pig, such as pork shoulder, pork belly, pork loin, pork butt (or ham), and the head. In yet another particular instance, the food product is a part cut directly from a sheep or lamb, such as shoulder chop, loin chop, rack, ribs, BRT leg, bone-in-leg, cabob or sirloin chop.

In another instance, the food product is meat is cut from an animal and then further processed, i.e., a processed food product. The term "processed" in this context refers to the at least one further processing or preparation step such as grinding, adding an ingredient or cooking, which changes the appearance, texture or taste. The processed meat product may be ready-to-cook or ready-to-eat. In one instance, the meat is processed by a mechanical process, such as cutting (e.g., to provide steaks, ribs or roasts), grinding or mixing. In another instance, the meat is further processed by salting, curing, fermenting or smoking.

In a particular instance, the meat is cut directly from cow and then further processed to provide steaks, stew beef, sausage or sausage casings, ground beef, canned meat, deli meat or beef jerky or the like. In another particular instance, the meat is derived from a pig and then further processed to produce sausage, bacon, spareribs, brisket, ribs, steaks, pork chops, pork cutlets, coppa, presa, secreto or tenderloin. In another particular instance, the meat is derived from a sheep or lamb and processed to produce ground lamb.

Also disclosed are meat by-products. The meat by-product may be, for example, a broth, stock or extract. Broths or stocks may be used consumed as is, or provide the base for another food product (e.g., a stew).

The food product may be a cultured meat, also referred to as lab-grown meat or *in vitro* meat. According to this instance, the non-primate mammal having reduced expression of alpha 1, 3 gal may be a source of biomass, proteins, lipids or cells for cultured meat. Cultured meat is produced by expanding cells from the source animal in culture. According to one protocol, a muscle sample is taken from a suitable animal and skeletal muscle stem cells (myo-satellite cells) isolated therefrom. The skeletal muscle stem cells are then grown in culture and encouraged to form multinuclear myotubes. Further growth is then encouraged by the introduction of new myoblasts and the fusing of myotubes to form myofibers. Other components, e.g., adipocytes, may be introduced to provide a meat-like product.

In a particular instance, the food product is a dairy product, or product derived from a mammalian milk source (e.g., milk proteins), wherein the source can be bovine, porcine, caprine, ovine, and camelids. The product can be the direct consumption of the milk or indirect after processing the milk into cheese, butter, ice cream, cultured or fermented milk products such as yoghurt, Kefir, buttermilk, cottage cheese, ricotta cheese.

Disclosed is a milk protein derived from a non-human mammal having reduced expression of alpha-1,3, galactosyltransferase. The milk protein may be from, for example, cow's milk, horse's milk, sheep's milk, goat's milk, or processed milk thereof, such as skim milk, reconstituted milk, powdered milk, or condensed milk.

The milk proteins include casein, a protein that is derived from the milk of many species and the name for a family of related phosphoproteins (α-s1, α-s2 , β, and 6).

In certain instances, the dairy-by product is whey protein, i.e., a collection of globular proteins isolated from whey, which is the liquid remaining after milk has been curdled and strained. Generally, the protein fraction in whey constitutes approximately 10% of the total dry solids in whey. Whey protein is typically a mixture of alpha-lactalbumin, beta-lactoglobulin, bovine serum albumin (BSA), and lactoferrin).

Milk products can also include, dry milk powder or milk protein concentrate. Whey proteins and/or caseins disclosed herein can be used, for example, as the milk protein source in infant formula or nutritional composition, i.e., a composition that satisfies some or all of a subject's nutritional needs.

In one instance, the food product is a dairy product (i.e., a dairy food or beverage) derived from a mammalian source. The dairy product may be derived directly from the mammalian source, e.g., milk, or further processed, e.g., milk further processed to provide cheese. The dairy product may be, for example, milk (e.g., whole, skim, 2%, 1%, flavored, cream and half-and-half), non-diary creamer, cheese, cultured dairy (e.g., yogurt, sour cream, cottage cheese), whey, condensed milk, ice cream or the like. Other animal by-products include Oleo (contains tallow), margarine and shortening.

In certain instances, the food product is an animal by-product, e.g., a food additive or processing aid. Disclosed herein are food additives and/or food processing aids derived from non-primate mammals having reduced expression of alpha 1, 3 galactosyltransferase. The food additive may be, for example, gelatin, rennet, flavorings, edible tallows, flour treatment agents (e.g., dough improvers), lactose, lactic acid, glycerol, beta-carotene coloring, sorbitan monostearate, bone char, whey powder, cheese products and the like.

In another instance, the food product is gelatin derived from a non-primate mammal having reduced expression of alpha 1, 3 galactosyltransferase. Gelatin is a soluble protein obtained by boiling skin, tendons, ligaments, and/or bones with water. It is obtained from cattle, or pigs, although in certain countries (e.g., Australia), sheep are also used. Extraneous substances, such as minerals (in the case of bone), fats and albuminoids (found in skin), are removed by chemical and physical treatment to give purified collagen. These pretreated materials are then hydrolyzed to gelatin which is soluble in hot water. In a particular instance, the gelatin is Type I gelatin.

In a particular instance, the food product is a gelatin-based dessert or dairy product (e.g., Bavarian creams, mousses, piecrusts, margarines, dietetic products, yogurts, ice creams and sorbets). In another instance, the food product is gelatin-based candy or confection (e.g., gummy bear, fruit snack, marshmallow, icing, or the like).

In a particular instance, the food additive or processing aid is gelatin derived from a non-primate mammalian source, e.g., bovine gelatin (type B gelatin)), porcine gelatin (Type A gelatin). The gelatin is used, for example, as a gelling agent, thickening agent, film-forming agent, emulsifier and/or stabilizer.

In one instance, the food additive is a collagen peptide (hydrolyzed collagen). Collagen peptides may be added, for example, to food bars or powdered or ready to drink beverages (e.g., sports beverages).

In another particular instance, the food additive is lactose. Lactose is a unique disaccharide which exists in the mammal breast milk and also the main carbohydrate in milk (more than 99.8% of the total sugar content). Normal fresh cow milk contains 4.8%, about 5.2% lactose, which is about 52% of non-fat cow milk solids and about 70% of the solid whey. The lactose may be used, for example, as a gelling agent, flavoring agent, browning agent and/or emulsifier.

In another instance, the food additive is rennet. Animal rennets are coagulant enzyme preparations extracted from the abomasum of ruminants, mainly veal, calf (e.g., bovine calf) and lamb. Rennet can also be produced from other sources (e.g. porcine pepsin, bovine pepsin. The rennin may be used in processing a food product, such as cheese.

In another instance, the food additive is edible tallow. Tallow is hard fat rendered from the fatty tissues of cattle (or sheep) that is removed during processing of the animal. The edible tallow may be added to a food product, for example, a baking mix, fried food, snack or salad dressing. In another instance, the edible tallow may be added to shortening or the like.

### B. Medical Products

The invention provides medical products as defined by the claims. The medical products prevent or reduce the risk of an allergic reaction in a subject previously diagnosed with α-Gal Syndrome (AGS). Components derived from the transgenic ungulate disclosed herein prevent or reduce the risk or severity of an allergic reaction in a subject in need thereof. These medical products may be available over-the-counter or by prescription.

The medical product comprises a biologic. In certain embodiments, the medical product is not intended for direct transplantation.

### (i) Drugs and Biologics

The medical product comprises a biologic containing one or more components derived from a transgenic ungulate lacking expression of alpha 1,3 galactosyltransferase. The medical product prevents or reduces the risk or severity of an allergic reaction in a subject previously diagnosed with α-Gal Syndrome.

Disclosed herein is a medical product that is a biologic selected from the group consisting monoclonal antibodies, recombinant antibodies, and immunoglobulins containing fragments of the antibodies; fusion proteins in which proteins or peptides are fused to constant domains (Fc) of antibodies; hormones; cytokines; enzymes; and combinations thereof.

In a particular instance, the medical product is a hormone, including, but not limited to. insulin, parathyroid hormone, thyroid hormone, estrogen, progesterone or relaxin.

In another particular instance, the medical product is a coagulation factor including, but not limited to, heparin, thrombin, fibrin, fibrinogen, factor VIII.

In another particular instance, the medical product is a pancreatic enzyme or pancreatic enzyme replacement, such as pancreatin (e.g., Creon^{®}, Nutrizym^{®}, or Pancrease^{®}).

In certain instances, the medical product is a recombinant protein therapeutics, such as a hormone, cytokines, enzyme, antibody or fusion protein.

In a particular instance, the medical product is an endocrine/hormone product, such as mammalian derived estrogen, testosterone, progesterone, including other steroid derivatives, insulin, erythropoietin (EPO) and thyroid hormones (T3&T4)
The medical product of the invention comprises a biologic for use in treating a cardiovascular disease.

In one instance of the disclosure, the medical product is a biologic for modulating blood clotting, such as an anti-coagulant, anti-thrombotic or hemostatic agent. In a particular instance, the medical product is an anti-coagulant, such as heparin, a heparin derivative, enoxaparin or dalteparin, oral anticoagulant.

In a particular instance, the medical product is a biologic for use in treating cancer. In a particular instance, the biologic is used for treating carcinoma, breast cancer, lung cancer, leukemia, lymphoma, prostate cancer, gastric cancer or colorectal cancer. In one instance, the biologic for use in treating cancer is a peptide, protein or antibody.

In one instance, the medical product is a biologic selected from the group consisting of ado-trastuzumab emtansine, aldesleukin, asparaginase, atezolizumab, bevacizumab, blinatumomab, brentuximab vedotin, capromab pendetide, cetuximab, daratumumab, elotuzumab, ibritumomab tiuxetan, interferon alfa-2b, ipilimumab, necitumumab, nivolumab, obinutuzumab, ofatumumab, olaratumab ( panitumumab, pegaspargase, pembrolizumab, pertuzumab, ramucirumab, rituximab, sargramostim, trastuzumab and ziv-aflibercept

**In** a particular instance, the medical product is cetuximab, a chimeric mouse-human IgG1 monoclonal antibody, is an epidermal growth factor receptor antagonist that is widely used for the treatment of metastatic colorectal cancer and squamous cell carcinoma of the head and neck.

**In** a particular instance, the medical product is an immunosuppressive drug. Representative, non-limiting examples of immune suppressive drugs include ATG (antithymocyte globulin), aAbatacept, belatacept (LEA29Y) and the like.

**In** another instance, the medical product is a biologic for use in treating Fraby disease, e.g., recombinant agalsidase alfa.

**In** a further instance, the medical product is a biologic for treating rheumatoid arthritis. **In** a particular instance, the medical product is adalimumab, bevacizumab etanercept, infliximab, rituximab or trastuzumab

**In** one instance, the medical product is a biologic for treating cystic fibrosis.

**In** one instance, the medical product is an anti-venom. The anti-venom may be any anti-venom, for example, anti-venom for a spider. Representative, non-limiting, spider anti-venoms include anti-venoms for Latrodectus hasselti (redback spider), Latrodectus mactans (black widow spider), Loxosceles spp. (recluse spiders), Phoneutria spp. (Brazilian wandering spiders) and *Latrodectus indistinctus* (black button spider). The anti-venom may be for a snake. Representative, non-limiting snake anti-venoms include anti-venoms for snakes in the Atractaspididae (atractaspidids), *Colubridae* (colubrids), *Elapidae* (elapids), or *Viperidae* (viperids) families.

**In** other instances, the medical product is a blood product that contains reduced or no alpha-gal or is obtained from an ungulate that has reduced alpha-1,3-galactosyltransferase. **In** another instance, the medical product is serum that contains reduced or no alpha-gal or is obtained from an ungulate that has reduced alpha-1,3-galactosyltransferase.

### (ii) Formulated Drug or Biologic

Described herein is a medical product that is a formulated drug or biologic, i.e., a composition containing a drug or biological as well or more excipients. **In** one instance, the excipient is derived from a non-primate mammal having reduced expression of alpha 1, 3 galactosyltransferase. In certain instances, the excipient is derived from a non-primate mammal lacking expression of alpha 1, 3 galactosyltransferase. Optionally, the drug or biological may also be derived from a non-primate mammal having reduced expression of alpha 1, 3 galactosyltransferase.

In a particular instance, the excipient is a gelatin stabilizers, binding agents, capsules, or lubricants such as magnesium stearate, lactose, tallow derivatives or glycerol.

In one instance, the excipient is gelatin. The gelatin may be a component of a formulated drug products such as a tablet, capsule, emulsion, syrup, suppository or the like. In one instance, the medical product is a hard gelatin capsule. In another instance, the medical product is a gummy, a purgative (e.g., a bowel prep) or oral care composition.

In another instance, the excipient is collagen. The collagen may be formed as a sheet, disk, sponge or the like.

In a further instance, the excipient is a tallow derivative. The tallow derivative may be used, for example, in an ointment or salve for topical use.

In a particular instance, the excipient is used to formulate a biologic, cell or gene therapy agent.

In a particular instance, the formulated medical product is a vaccine, such as a measles, mumps, rubella, varicella or DTaP (diphtheria-tetanus-acellular pertussis) vaccine. In another instance, the medical product is a vaccine for treating zoster or rotavirus.

### (iii) 3D Printing Material

Disclosed herein are compositions for use in 3D printing (also referred to as additive manufacturing), in each case containing one or more in each case derived from a non-primate mammal having reduced expression of alpha 1, 3 galactosyltransferase. In certain instances of the disclosure, the non-primate mammal lacks expression of alpha 1, 3 galactosyltransferase.

In one instance, the composition comprises collagen, gelatin, laminin, elastin, fibrogen or a combination thereof. The composition may be used in 3D printing of products for use in tissue engineering, such as scaffolds (e.g., a bone scaffold). The scaffold is intended to promote cellular proliferation and function. It may be acellular or cellular in nature.

The 3D printing method may vary. In one instance, the 3D printing method is extrusion-based, laser-based and inkjet-based 3D printing.

In certain instances, the composition is a collagen gel or solution for use in producing scaffolds by 3D printing.

In certain instances, the composition comprises gelatin for use in producing scaffolds by 3D printing. In one instance, the composition is a methacrylated gelatin (GelMA). In another instance, the composition is a glycerol gelatin.

### (iv) Bioactive Agents

Disclosed herein is a medical product that is a bioactive agent used in a biologicallybased production systems, e.g., cultured animal cells. Effectively all cultured animal cells require serum or some other biological preparation.

Any animal cell capable of being cultured is suitable for use with the bioactive agent disclosed herein, including but not limited to mammalian cells. The mammalian cells may be, for example, human stem cells, including human pluripotent stem cells (hPSCs), including both human embryonic stem cells (hESCs) and induced pluripotent stem cells (hiPSCs); mouse embryonic stem cells; mesenchymal stems cells, including human or mammalian mesenchymal stem cell lines; chimeric antigen receptor (CAR)-T cells; cells used for protein/drug manufacture (human embryonic kidney line 293S, 293T, HeLa); mouse cells (i.e. Sp2-0, Sp-1, primary splenocytes, chinese hamster ovary (CHO), or other mammalian cells intended to synthesize a therapeutic product.

In one instance, the cell is a hamster cell line, such as a CHO cell line or a baby hamster kidney (BHK) cell line. In a particular instance, the CHO cell is selected from the group consisting of CHO-K1, CHO-DXB11 and CHO-DG44.

In another instance, the cell is a mouse myeloma cell line. In a particular instance, the murine myeloma cell line is selected from the group consisting of NS0 and Sp2/0.

In a further instance, the cell is a fully human mammalian cell line. In a particular instance, the fully human mammalian cell line is selected from the group consisting of human embryonic kidney cells (HEK-293), human fibrosarcoma HT-1080, CAP, human embroyic retinoblasts (Per. C6), HBK-11 and HuH-7.

In one instance, disclosed herein are natural media for animal (e.g., mammalian) cell culture. As used herein, the term "natural media" refers to media consisting of natural biological substances, such as plasma, serum, and embryo extract. In a particular instance, a serum media for animal cell culture is disclosed. Serum provides carriers or chelators for labile or water-insoluble nutrients, hormones and growth factors, protease inhibitors, and binds and neutralizes toxic moieties. The natural media disclosed herein provides a α-Gal free (or other non-gal antigens) growth environment for cells.

In another instance, disclosed herein is a synthetic medial for animal (e.g., mammalian) cell culture. As used herein, the term "synthetic media" refers to media composed of a basal medium and supplements, such as serum, bovine serum albumin (BSA) growth factors, and hormones.

In one instance, disclosed herein is a growth factor for animal (e.g., mammalian) cell culture. Representative, non-limiting growth factors include epidermal growth factor (EGF) (e.g., TGF-α, neuregulins, amphiregulin, betacellulin), fibroblast growth factor (FGF), nerve growth factor (NGF), platelet-derived growth factor (PDGF)(e.g., PDGF-AA, PDGF-BB, PDGF-CC, PDGF-DD, and PDGF-AB) , vascular endothelial-derived growth factor (VEGF), insulin-like growth factors (IGF)(e.g., IGF-1), granulocyte-macrophage colony-stimulating factor (GMCSF), granulocyte-colony stimulating factor (GCSF), transforming growth factor (TGF), erythropieitn, thrombopoietin (TPO), bone morphogenic protein (BMP), hepatocyte growth factor (HGF), growth differentiation factor (GDF), neurotrophins, melanocyte-specific factor (MSF), sarcoma growth factor (SGF), tumor necrosis factors (TNF), interleukins, interferons and growth differentiation factor (GDF).

In another instance, an excipient for animal (e.g., mammalian) cell culture is disclosed.

The cell culture medium disclosed herein is also designed to produce a target substance by cell culture. In one instance, the target substance may be selected from the group consisting of: monoclonal antibodies, recombinant antibodies, and immunoglobulins containing fragments of the antibodies; fusion proteins in which proteins or peptides are fused to constant domains (Fc) of antibodies; encodrine/hormones; cytokines; enzymes; and combinations thereof.

In one instance, the target substance is heparin or a heparin derivative (e.g., low molecular weight heparins, heparanoids).

In another instance, the target substance is a mammalian-derived estrogen, testosterone, progesterone, including other steroid derivatives, insulin, erythropoietin (EPO) and thyroid hormones (T3&T4).

In another instance, bioactive agents are disclosed herein for use in research and/or development assays that utilize derivatives from catarrhines.

In one instance, disclosed herein are bioactive agents for use in production of human stem cell therapies (autologous blood stem cells or CAR-T) or gene therapy.

### (v) Reagents for Cell Culture and Production of Human Therapeutics

In other instances of the disclosure, reagents or proteins for use in cell culture are provided. In another instance, reagents or proteins for use in producing antibodies for human therapeutics are provided. In one instance, growth factors, serum, or serum proteins such as albumin, for use in cell culture are provided. In another instance, reagents or proteins for use in producing antibodies for human therapeutics (e.g. monoclonal antibodies, human therapeutic or recombinant proteins, T regulatory cells, human autologous cells or cell or gene therapy reagents) are provided. These reagents or proteins can be derived from a non-primate mammal having reduced expression of alpha 1, 3 galactosyltransferase, such as a cow, pig, sheep or combination thereof. In a particular instance, the non- primate lacks expression of alpha-1,3 galactosyltransferase.

In another instance the reagent or proteins can be used in cell culture to generate large quantities of cells to reseed decellularized scaffolds, where in the scaffolds were derived from an animal that lack the expression of alpha-gal, thus avoid exposure (may cross contamination) of the scaffold with alpha-gal.

### C. Medical Devices

Medical devices are disclosed that contain one or more components derived from a non-primate mammal having reduced expression of alpha 1, 3 galactosyltransferase. In certain instances, the medical device contains one or more components derived from a non-primate mammal lacking expression of alpha 1, 3 galactosyltransferase.

The medical device may be for treating or preventing human disease, disorder or condition. The medical device may be used, for example, to replace a missing biological structure, support of damaged biological structure or enhance an existing biological structure. Medical devices for both external and internal use are contemplated. In a particular instance, the medical devices are suitable for use by (or with respect to) subjects previously diagnosed with α-Gal Syndrome.

In one instance, the medical device is for treatment of an acute or chronic wound. Acute wounds include, for examples, burns or traumatic wounds. Chronic wounds include, for example, ulcers. The depth of the wound may vary, and includes superficial, partial thickness and full thickness wounds.

In a particular instance, the medical device is a wound dressing comprising at least one component derived from a non-primate mammal, such as a cow, pig or sheep, having reduced expression of alpha 1, 3 galactosyltransferase.

In a particular instance, the medical device is a collagen or collagen-based wound dressing. The collagen or collagen-based wound dressing may be formulated or configured, for example, as a gel, paste, powder or pad.

In certain instances, the medical device is a wound dressing comprising porcine collagen, bovine collagen or combinations thereof. The anatomical source of the mammalian collagen may vary. In one instance, the source of the collagen is dermal, intestinal, muscle (e.g., tendon) or bladder.

In another instance, the medical device is an implant comprising at least one component derived from the non-primate mammal having reduced expression of alpha 1, 3 galactosyltransferase. The implant may be used to replace or modify a human body part. In one instance, the implant is a dental implant, an orthopedic implant, an ophthalmologic implant, a cardiovascular implant, a nerve implant, an organ-derived scaffold or implant, or a cosmetic implant or filler. In certain instances, the implant may be used to supplement a human body part. The implant may be passive or active.

The biologic of the invention is a cardiovascular implant comprising a heart valve, such as an aortic or mitral valve. The implant may be, for example, bovine pericardium (BP), a bovine jugular venous valve (BJV), or a porcine aortic valve leaflet (PAV). The heart valve is not subject to premature degradation, e.g., collagen disruption due to, e.g., calcification or inflammation or the like. In a particular embodiment, the the heart valve is clinically functional for more than 10 years, more than 11 years, more than 12 years, more than 13 years, more than 14 years, more than 15 years, more than 16 years, more than 17 years or 18 years or more. In one embodiment, the cardiovascular implant is clinically functional for more than 20, more than 25 or more than 30 years. In certain embodiments, the functional lifetime of the cardiovascular implant (e.g., the cardiovascular valve) is measured under appropriate laboratory (experimental) conditions, e.g., using a circulatory *in vivo* model.

In a particular embodiment, more than about 50% of cardiovascular implants (e.g., heart valves) are functional at about 5 years, at about 10 years, at about 13 years, at about 15 years, at about 18 years or at about 20 years after implantation.

In another particular embodiment, more than about 75% of cardiovascular implants (e.g., heart valves) are functional at about 10 years, at about 13 years, at about 15 years, at about 18 years or at about 20 years after implantation.

In one embodiment, the heart valve is not characterized by premature degradation. In a particular embodiment, the heart valve is suitable for clinical use about 5, about 10, about 11, about 12, about 13, about 14 or about 15 years or more after implantation. In a particular embodiment, the heart valve is suitable for clinical use for at least 10 years. In another embodiment, the heart valve is suitable for clinical use for at least 15 years. In a further embodiment, the heart valve is suitable for clinical use for at least 20 years. In another embodiment, the heart valve does not exhibit degeneration, such as structural valve degeneration.

In further embodiments, the heart valve does not exhibit structural valve degeneration due to an immunological response to the valve. In other embodiments, a method is provided to avoid an immunological response to a bioprosthetic heart valve by transplanting a GalSafe^{®} heart valve. The GalSafe^{®} heart valve is obtained from an ungulate. In one embodiment, the ungulate is a pig. In an alternative embodiment the ungulate is a cow.

**In** other embodiments of the present invention, a method is provided to prevent degradation of a bioprosthetic heart valve by transplanting a GalSafe^{®} heart valve. **In** one embodiment, the degradation is structural valve degradation. The GalSafe^{®} heart valve is obtained from an ungulate. **In** one embodiment, the ungulate is a pig. **In** an alternative embodiment the ungulate is a cow.

Heart valve replacement surgery began in the early 1960s in patients with valvular heart disease. **In** 2009, approximately 90,000 valve substitutes were implanted in the United States and 280,000 worldwide each year. Technical advances in the design of valves have significantly improved long-term prognosis. There are two main types of valves, mechanical and bioprosthetic valves. Because of thrombogenicity of materials used in mechanical valves, high shear stress around the hinge points, and backflow jets that damage blood and activate clotting-pathways, patients require lifelong anticoagulation therapy to avoid blood clot formation. Bioprosthetic valves are generally made of either bovine pericardium or porcine aortic valves, but may also be produced from equine or porcine pericardium. Bioprosthetic valves do not require life-long anticoagulation for the recipient. However, the main risk with bioprosthetic valves is reoperation for structural valve deterioration (SVD) due to the limited durability of bioprosthetic valves. The average lifespan of a bioprosthetic valve is estimated at 15 years in elderly patients, but this risk is higher in younger patients in whom SVD is accelerated due to a more pronounced immunologic response to the valve and enhanced calcification of the valve. Despite guideline recommendations against the use of bioprosthetic valves in patients younger than 60, the use of bioprosthetic valves has significantly increased over the last decade. (Head et al. European Heart Journal, Volume 38, Issue 28, 21 July 2017, pp 2183-2191).

The disclosure describes a medical device that is an ophthalmologic implant. The ophthalmologic implant may be, for example, a lens, an ocular prosthesis or other type of ocular transplant. In one instance, the ophthalmologic implant is a full thickness corneal transplant. In one instance, the medical device is a collagen-derived contact lens.

The medical device of the disclosure may be a skin substitute. The skin substitute may be for example, a temporary skin substitute. The layer or skin substituted may vary, e.g., epidermis, dermis or a combination thereof. The skin substitute may contain one or more additional components. In certain instances, the skin substitute is a multi-layer skin substitute.

In one instance, the skin substitute is an acellular skin substitute.

In another instance, the skin substitute is a cellular skin substitute.

The medical device of the disclosure may also be a bone substitute. The bone substitute may be used to treat a subject suffering from trauma, congenital abnormalities, cancer resection, deforming diseases or the like. The medical device may be used for purposes of replacement, repair or augmentation of damaged bones and/or joints.

In certain instance, the medical device is an orthopedic implant. orthopedic implant may be, for example, a joint replacement (e.g., knee, hip, shoulder), bone graft, fusion product, or a spinal impact (e.g., disc).

In one instance, the bone substitute is a bone scaffold.

In one instance, the medical device is a drug delivery device comprising at least one component derived from a non-primate mammal, such as a cow, pig or sheep, having reduced expression of alpha 1, 3 galactosyltransferase.

The drug delivery device may contain one or more therapeutic agents. The therapeutic agent may be any suitable therapeutic agent, such as an antibiotic agent, antibacterial agent, antiviral agent, anti-glaucoma agents, antiallergenic agent, anti-inflammatory agent, antiangiogenesis agent, antiproliferative agent, immune system modifying agent, anti-cancer agent, antimycotic agent, mitotic agent, anticholinesterase agent, mydriatic agent, differentiation modulator agent, sympathomnimetic agent, anaesthetic agent, vasoconstrictive agent, vasodilatory agent, transport/mobility impending agent, polypeptides and protein agent, polycations, polyanions, steroidal agent, carbonic anhydride inhibitor agent, lubricating agents or combinations thereof.

The drug delivery device may be suited to deliver drug to any suitable structure or area of the body. In one instance, the drug delivery device is an ophthalmic drug delivery device. The ophthalmic drug delivery device may be, for example, a drug delivery implant.

In another instance, the drug delivery device is a systemic drug delivery device.

In a particular instance, the drug delivery device is a collagen implant, sponge or shield. In another particular instance, the drug delivery device is a hydrogel.

In another instance, the medical device is a suture or closure device. In a particular instance, the medical device is a collagen suture or closure device.

Also disclosed are hemostatic agents, i.e., agents intended to stem blood-flow through the accelerated promotion of clotting. The mechanism of action of the hemostatic agent may vary and include, for example, concentrating coagulation factors, adhesion to the tissues, in which traumatic hemorrhage occurred, and delivering pro-coagulant factors to the hemorrhage site.

In one instance, the hemostatic agent is selected from the group consisting of physical agents, absorbable agents, biologic agents, synthetic agents and hemostatic dressings.

In certain instances, the hemostatic agent comprises colloids, such as intravenous colloids (e.g., gelatin-derived colloids).

In certain instances, the hemostatic agent is an absorbable hemostatic agent such as a microfibrillar collagen (e.g., derived from purified bovine dermal collagen), a gelatin form (e.g. GelFoam^{®}, Surgifoam, Avitene^{™}, Ultrafoam^{®}, ThrombinJMI^{®}) or an absorbable collagen hemostat sponge (e.g. derived from purified and lyophilized bovine flexor tendon).

In certain instances, the composition derived herein is not a medical device or more particularly, is not a tissue product.

### D. Cosmetic Products and Ingredients

The disclosure describes a cosmetic product, cosmeceutical or cosmetic ingredient is disclosed containing one or more components derived from a non-primate mammal having reduced expression of alpha 1, 3 galactosyltransferase. In a particular instance of the disclosure, the non-primate mammal lacks expression of alpha 1, 3 galactosyltransferase.

As used herein, the term "cosmetic product" means a composition that is intended to be applied onto the subject's skin, particularly onto the facial skin or onto the body skin area or onto hair, so as to regulate the condition of the skin and/or to improve the appearance of the skin and hair.

The cosmetic product may be formulated in any suitable manner. In one instance, the cosmetic product is formulated as a powder, tablet, cake, gel, cream, lotion, liquid, mousse, stick, ointment or paste.

In one instance, the cosmetic product is a colored cosmetic product. The colored cosmetic product may be, for example, a primer, a foundation, a blush, a lipstick, a lip gloss, an eye shadow, an eyeliner, a mascara or an eyebrow pencil or the like.

In another instance, the cosmetic product is a skin care product. The skin care product may be, for example, a cleanser, moisturizer, anti-aging product or sunscreen. In certain instances, the skin care product (e.g., anti-aging product) comprises collagen.

In one instance, the cosmetic product is a personal care product. The personal care product may be, for example, a shampoo, a conditioner, a body wash, a shaving cream or the like. In certain instances, the personal care product comprises collagen, gelatin and or glycerin (glycerol).

The cosmetic product may contain one or more components derived from a non-primate mammal having reduced expression of alpha 1, 3 galactosyltransferase, such as emollients, thickeners or emulsifiers. In a particular instance, the cosmetic product can contain gelatin, lanolin, collagen, glycerol, elastin, estrogen or bone marrow.

In other instances, the cosmetic product may contain one or more components derived from non-primate mammal having reduced expression of alpha 1, 3 galactosyltransferase, wherein the one or more components include an active agent such as stearic acid or retinol. In a particular instance, the retinol is derived from a cow having reduced expression of alpha 1, 3 galactosyltransferase.

.In certain instances, an injectable material is disclosed for use in soft tissue augmentation. The term "soft tissue", as used herein, refers to tissues that connect, support, or surround other structures and organs of the body. Soft tissue includes muscles, fibrous tissues and fat. The term "augmentation" means the repair, decrease, reduction or alleviation of at least one symptom or defect attributed due to loss or absence of tissue, by providing, supplying, augmenting, or replacing such tissue with the compositions disclosed herein. The compositions can also be used to prevent at least one symptom or defect. In certain instances, the injectable material is formulated as a liquid, gel or hydrogel.

In a particular instance, the soft tissue augmented is selected oft tissue is selected from the group consisting of skin, muscles, glands, ducts, tendons, follicles, and combinations thereof. In another instance, the skin is located on an area selected from the group consisting of face, neck, arms, underarms, legs, buttocks, abdomen, back, breasts, scalp, feet, and hands.

In certain instances, the cosmetic product comprises collagen and is used in reconstructive or cosmetic surgery. The collagen may be, for example, purified collagen from a cow or pig lacking expression of alpha 1, 3 galactosyltransferase. In one instance, the collagen is selected from type I, III and V. The cosmetic product may be, for example, a dermal filler.

In a certain instance, the cosmetic product comprises gelatin and is used in reconstructive or cosmetic surgery. The gelatin may be, for example, purified collagen from a cow or pig lacking expression of alpha 1, 3 galactosyltransferase.

In another instance, a composition containing one or more components derived from a non-primate mammal having reduced expression of alpha 1, 3 galactosyltransferase is disclosed for use as a dental implant or dental reconstructive surgery using demineralized bone powder (DBM).

In one instance, a cosmeceutical is disclosed herein containing one or more components derived from a non-primate mammal having reduced expression of alpha 1, 3 galactosyltransferase.

In one instance, disclosed herein is a cosmetic ingredient such as gelatin, lanolin, collagen, glycerin, elastin, estrogen or bone marrow.

### E. Textiles

Textile products are disclosed that contain at least one component derived from a non-primate animal lacking alpha 1, 3 galactosyltransferase. The component of the textile product can be wool, hair and or a combination thereof. The textile product can be wool, including wool clothing, such as socks. In another instance of the disclosure, the textile product can be leather. In other instances the textile product can be sheepskin.

In certain instances, the textile product is produced by a method selected from weaving, knitting or felting.

### F. Animal Models

Disclosed herein are non-primate mammals having reduced expression of alpha 1, 3 galactosyltransferase as animal models for the study of, for example, safety, effectiveness and/or pharmacokinetics of drugs that may rely on an anti-gal immunoglobulin response for efficacy. The model may be sensitized or attenuated to amplify or reduce immunogenic response. In certain instances of the disclosure, the model may be further sensitized to elicit elevated anti-gal antibody titers.

### II. Methods of Use

The medical products of the invention are for use in a method of preventing or reducing the risk or severity of an allergic reaction in a subject in need thereof.

The subject has been diagnosed with AGS. AGS is characterized by an IgE response and delayed type I allergic reaction to the carbohydrate galactose-alpha-1,3-galactose (alpha-gal) after exposure to the same. Alpha-gal is abundantly expressed on glycoproteins from non-primate mammals. The subject may be exposed to alpha-gal by a tick or other organisms (e.g., chigger).

In a particular embodiment, the subject in need thereof has previously been exposed to alpha gal by a bite from an Arachnid, or a developmental form thereof (larva, nymph, adult). The arachnid group includes, but is not limited to, *Amblyomma americanum* (Lone tar tick), *Amblyomma cajennens, Ixodes holocyclus, Ixodes scapularis* and *Ixodes ricinus.* In a further embodiment, the exposure to alpha-gal can be the bite of an blood sucking insect (e.g. mosquito, deer or horse flies, fleas, mites and lice. Upon re-exposure, binding of the allergen to IgE orchestrates the immune system to initiate a more aggressive and rapid memory response.

In a particular embodiment, the subject has been diagnosed with AGS by a suitable method. The suitable method may vary and include, for example, patient history, skin tests, determination of IgE antibodies, oral food or drug challenges or a combination thereof.

In one embodiment, the subject has been diagnosed with AGS by serological confirmation. For example, the subject has been determined to have α-Gal-IgE levels fall greater than 0.1 or 0.35 kUA/L. IgE reactivity to alpha-gal can be assessed by any suitable method, for example, by immunoblotting and ELISA,

In a particular embodiment, the subject does not have detectable IgG4 to α-Gal.

In another embodiment, the subject has been diagnosed with AGS by patient history. For example, the subject has experienced anaphylactic symptoms after the ingestion of mammalian meat products (e.g., beef, pork or lamb). The anaphylactic symptoms may be delayed. In one embodiment, the anaphylactic symptoms are delayed by at least about 3 hours, at least about 4 hours, at least about 5 hours or at least about six 6 hours.

In certain embodiments, the subject with AGS may have one or more additional food allergies.

The allergic reaction may range in severity. For example, the allergic reaction may be mild, chronic, acute and/or life threatening. The most severe allergic reaction is anaphylaxis, a life-threatening allergic reaction that can impair breathing, cause a dramatic drop in blood pressure and impact heart rate.

In certain embodiments, the allergic reaction is delayed, for example, by more than 2 hours after exposure. The allergic reaction may be delayed by about 3 to about 6 hours, for example. Delayed allergic reactions are distinguished from immediate allergic reactions, which generally appear within 10-15 minutes of exposure to an antigen. In a particular embodiment, the allergic reaction is delayed about 2, about 3, about 4, about 5, about 6 or more hours.

In one embodiment, the allergic reaction is hypersensitivity, i.e., a state of altered reactivity in which the body reacts with an exaggerated immune response to a substance (antigen). Hypersensitivity may be generalized or organ specific.

In a particular embodiment, the allergic reaction is a type I hypersensitivity. Prior sensitization to the antigen results in an immune response initially mediated by CD4 lymphocytes (variety) that promote mast cell proliferation and plasma cell production of IgE. The IgE becomes bound to mast cells in places such as respiratory tract mucosa. Encountering the allergen again leads to mast cell degranulation with release of primary mediators (e.g.,histamine). There two phases of type I hypersensitivity- (i) the initial response and (ii) the late phase reaction. The initial response characterized by vasodilatation, vascular leakage, and smooth muscle spasm or glandular secretions. These changes usually occur within 5 to 30 minutes after exposure and tend to subside in 60 minutes. The late phase reaction occurs about 2 to 8 hours later without additional exposure to antigen and lasts for several days. It is characterized by more intense infiltration of tissues with eosinophils, neutrophils, basophils, monocytes, and CD4+ T cells as well as tissue destruction in the form of mucosal epithelial cell damage. Severity can range from mild to fatal.

Clinical and pathologic features of type I hypersensitivity are secondary to inflammatory mediators produced by mast cells in different tissues. The most common symptoms include itching, swelling, abdominal pain, diarrhea, nausea, vomiting, wheezing, nasal congestion and trouble breathing.

In one embodiment, the allergic reaction is a form of hypersensitivity selected from cutaneous hypersensitivity, gastrointestinal hypersensitivity or respiratory hypersensitivity. In certain embodiments, one or more symptoms of hypersensitivity are preventing or reduced as a result of the method disclosed herein.

Where symptoms are prevented, there can be an about 5, about 10, about 15, about 20, about 25, about 30, about 35, about 40, about 50, about 60, about 70, about 80, about 90, or about 100% reduction in the establishment of disease frequency relative to untreated controls.

Where symptoms are reduced, the reduction may be, for example, about 10%, about 20%, about 30%, about 40%, about 50%, about 60%, about 70% or about 80% or more.

Cutaneous hypersensitivity may manifest in various ways, including but not limited to, atopic dermatitis, angioedema, erythema, eczematous rash/lesions and/or uricartia.

In one embodiment, the disclosed method prevents or reduces the severity of atopic dermatitis in a subject in need thereof, such as a subject previously diagnosed with α-Gal Syndrome. Atopic dermatitis (which is sometimes referred to as " allergic eczema") is a pruritic (itching) inflammatory skin disorder. Symptoms may vary by age and may include redness, swelling, rash, discoloration of skin, papules, blisters, thickened skin and the like. The hands, face and especially the eyelids are most often involved, as well as large skin folds and sometimes other areas.

In one embodiment, the disclosed method prevents or reduces the severity of angioedema in a subject in need thereof, such as a subject previously diagnosed with α-Gal Syndrome. Angioedema involves swelling of the deep dermal, subcutaneous, or submucosal tissue due to vascular leakage that can be life-threatening. Swelling often occurs around the eyes, lips, and tongue, but can impact other parts of the body as well. Angiodema may or may not be itchy, but is often accompanied by pain and tenderness. Sever angioedema is often associated with other symptoms of allergic reaction, such as urticaria.

In one embodiment, the disclosed method prevents or reduces the severity of uritcartia in a subject in need thereof, such as a subject previously diagnosed with α-Gal Syndrome. Uritcartia (hives) involves swelling of the epidermis and dermis. It can occur in any part of the body, presenting as red, raised, itchy bumps (welts). Pain and tenderness are less common than in angioedema although the two may occur together.

Gastrointestinal hypersensitivity is associated with symptoms including, but not limited to, itching and swelling of the mouth and oral passages, bloating, flatulence, diarrhea, constipation, reflux, abdominal pain and cramps, borborygmi, heartburn, nausea, dyspepsia, feeling of incomplete defecation, and urgency in defecation. In one embodiment, the present method prevents or reduces the severity of gastrointestinal hypersensitivity in a subject in need thereof, such as a subject previously diagnosed with α-Gal Syndrome.

Respiratory hypersensitivity may manifest as asthma or rhinitis. It is associated with symptoms including bronchospasm, itching, water eyes, sneezing, wheezing, dyspnea or a combination thereof.

In one embodiment, the disclosed method prevents or reduces the severity of asthma in a subject in need thereof, such as a subject previously diagnosed with α-Gal Syndrome. Asthma is characterized by airway inflammation, hyper-responsiveness, and obstruction which often causes spasms of the bronchial smooth muscle system, and affects both the upper and lower respiratory tracts. The asthma may be, for example, mild, moderately severe or severe.

In one embodiment, the hypersensitivity is general hypersensitivity or anaphylaxis. Anaphylaxis usually develops gradually, most often starting with itching of the gums/throat, the palms, or the soles, and local urticaria; developing to a multiple organ reaction often dominated by severe asthma; and culminating in hypotension and shock. Hypotension and severe bronchospasm do not have to be present for a reaction to be classified as anaphylaxis. In one embodiment, the disclosed method prevents or reduces the severity of asthma in a subject in need thereof, such as a subject previously diagnosed with alpha-Gal Syndrome.

In a particular instance of the disclosure, the method disclosed herein comprises providing a food product or food ingredient disclosed herein to the subject in need thereof, thereby preventing or reducing the risk of severity of an allergic reaction in a subject in need thereof, such as a subject previously diagnosed with α-Gal Syndrome.

In one instance, the food product provided to the subject is meat or a meat by-product derived from a non- primate mammal having reduced expression of alpha 1, 3 galactosyltransferase, such as a cow, pig, goat, horse, or sheep.

The method disclosed herein comprises providing a medical product disclosed herein to the subject in need thereof, thereby preventing or reducing the risk of severity of an allergic reaction in a subject previously diagnosed with α-Gal Syndrome.

The medical product comprises a biologic provided to the subject in need thereof, wherein the biologic is derived from a transgenic ungulate.

In another instance of the disclosure, a medical device provided to the subject in need thereof, wherein the medical device contains one or more component derived from a non-primate mammal having reduced expression of alpha 1,3 galactosyltransferase such as a cow, pig or sheep. In a particular instance, the medical device contains collagen.

In a further particular instance of the disclosure, the method disclosed herein comprises providing a cosmetic product or ingredient disclosed herein to the subject in need thereof, thereby preventing or reducing the risk of severity of an allergic reaction in a subject in need thereof, such as a subject previously diagnosed with α-Gal Syndrome.

In one instance of the disclosure, the cosmetic product contains one or more components derived from a non-primate mammal having reduced expression of alpha 1, 3 galactosyltransferase, such as a cow, sheep, goat, horse, or pig. In a particular instance, the component is collagen or lanolin.

In certain embodiments, the method disclosed herein prevents or reduces the severity of cutaneous hypersensitivity, gastrointestinal hypersensitivity, respiratory hypersensitivity or general hypersensitivity.

In certain embodiments, the method disclosed herein prevents or reduces the severity of one or more symptoms selected from the group consisting of skin rash, hives, itching, nausea, abdominal cramping, vomiting, diarrhea, nasal congestion, sneezing, asthma or anaphylaxis.

In another example, the patient has experienced anaphylactic symptoms after being treated with a drug or drug product. The drug product may be, for example, a peptide, protein or monoclonal antibody. The anaphylactic symptoms may be immediate or delayed.

In another embodiment, the disclosed method prevents allergen specific positivity to α-Gal, and comprises providing the composition disclosed herein to a subject having previously been diagnosed with AGS.

In a further embodiment, the disclosed method prevents IgE-mediated hypersensitivity, and comprises providing the composition disclosed herein to a subject having previously been diagnosed with AGS.

In yet a further embodiment, a method is disclosed for suppressing IgE-mediated anaphylaxis, comprising providing the composition disclosed herein to a subject having previously been diagnosed with AGS. In a particular embodiment, a method of preventing or reducing the risk or severity of an allergic reaction in a subject in need thereof is provided, comprising providing the medical product disclosed herein wherein the allergic reaction is mediated by an IgE immune response. In a particular embodiment, the subject has an IgE mediated disease. In particular, compositions and methods for preventing and methods for use in preventing or reducing the risk or severity of an allergic reaction in a subject in need thereof are provided. In addition, compositions and methods for preventing and methods for use in preventing or reducing the risk or severity of an IgE mediated disease in a subject in need thereof. Further, compositions and methods for preventing and methods for use in preventing or reducing the risk or severity of an anaphylactic reaction, in a subject in need thereof are provided. In certain embodiments, the subjects have IgE antibodies directed to alpha 1, 3 galactosyltransferase.

Preventing or reducing the risk or severity of an allergic reaction in a subject in need thereof is disclosed, comprising providing a food product that does not contain alpha-gal. Preventing or reducing the risk or severity of an allergic reaction in a subject in need thereof is disclosed, comprising providing a cosmetic product that does not contain alpha-gal. Preventing or reducing the risk or severity of an allergic reaction in a subject in need thereof is disclosed, comprising providing a medical device that does not contain alpha-gal.

A method of preventing or reducing the risk or severity of an allergic reaction in a subject in need thereof is disclosed, comprising providing the compositions disclosed herein wherein the allergic reaction wherein the subject has IgG4 antibodies to alpha 1, 3 galactosyltransferase.

Also disclosed herein are methods to treat diseases are provided by administering to the patient a medical product disclosed herein. In one instance the medical product is a drug that does not contain alpha-gal. In another instance, the medical product is a biologic that does not contain alpha-gal. The biologic can be a hormone, protein or antibody.

Further disclosed herein are methods to treat diseases are provided by administering to the patient a medical product disclosed herein. The disease can be any disease or condition listed in Table 1, 2 3 or 4. In addition, the medical produce can be any product listed in Table 1, 2, 3 or 4 that is derived from a non-primate mammal with reduced expression of alpha 1,3 galactosyltransferase. In other instances of the disclosure, the medical product can be derived from any tissue source listed in Table 1, 2, 3 or 4. In one instance , the medical product derived from the non-primate mammal does not contain or has reduced alpha-gal. In one instancethe medical product is a drug that does not contain alpha-gal. In another instance, the medical product is a biologic that does not contain alpha-gal. The biologic can be a hormone, protein or antibody.

In other instances, the disease can be an exocrine deficiency and the biologic is a pancreatic enzyme that does not contain alpha-gal. The exocrine deficiency can be cystic fibrosis, surgical pancreatectomy, and chronic pancreatitis. In other instances, the patient has a disease that requires treatment with an anticoagulant and the biologic is an anticoagulant, such as heparin, that does not contain alpha-gal.

**Table 1. Variety of drugs, dressings, and surgical implants derived from pigs.**

| **Tissue source** | **Active ingredient** | **Tissue Source** | **Active ingredient** |
|---|---|---|---|
| Adrenal Glands | Corticosteroids | Ovaries | |
| | Cortisone | | Estrogens |
| | Epinephrine | | Progesterone Relaxin |
| | Norepinephrine | | |
| Blood | | Pancreas Gland | Kallikrein |
| | Blood Albumens | | Glucagon |
| | Blood Fibrin | | Lipase |
| | Fetal Pig Plasma | | Pancreatin |
| | Plasmin | | Trypsin |
| | | | Chymotrypsin |
| Brain | Cholesterol | Pineal Gland | Melatonin |
| | Hypothalamus | | |
| | Dura | | |
| Gall Bladder | Chenodeoxycholic Acid | Pituitary Gland | ACTH - Adrenocorticotropic Hormone |
| | | | ADH - Antidiuretic Hormone Oxytocin |
| | | | Prolactin |
| | | | TSH - Thyroid Stimulating Hormone |
| Heart | | Skin | Porcine Burn Dressings |
| | Heart Valve | | Gelatin |
| | Pericardiums | | Soft tissue repair |
| | | | Hernia, shoulder, cosmetics |
| Intestines | Enterogastrone | Spleen | Splenic Fluid |
| | Heparin | | |
| | Secretin | | |
| | SIS | | |
| Ligament repair | Patella tendon Achilles | Stomach | Pepsin |
| | | | Mucin |
| Liver | Cholic Acid Catalase | Thyroid Gland | Thyroxin |
| | | | Calcitonin |
| | Desiccated Liver | | Thyroglobulin |
| Nerve | nerve | | |

**Table 2: Porcine Derived Products**

| **Product name** | **Generic name** | **Therapeutic class** |
|---|---|---|
| Clexane | Enoxaparin | Anticoagulant, Antithrombotics |
| Creon | Pancrelipase | Digestive supplements and cholelitholytics |
| Creon Micro Enteric coated granules | Pancrelipase | Digestive supplements and cholelitholytics |
| Curosurf | Poractant alfa | Respiratory agent |
| Ethical Nutrients Digestion plus | | Herbal gastrointestinal preparations |
| Fragmin | Dalteparin | Anticoagulant |
| Heparin sodium injection | Heparin sodium | Anticoagulant |
| Heparinised saline | Heparin sodium | Anticoagulant |
| Heparinised saline injection | Heparin sodium | Anticoagulant |
| Orgaran | Danaparoid | Haemostatic agent |
| Panzytrat 25000 | Amylase, Lipase, Pancrelipase, Protease | |
| Prothrombinex-VF | Antithrombin III, human; Factor II; V, VII, IX, X Heparin, porcine | |
| Rotarix | Human rotavirus live attenuated vaccine | Vaccine |
| RotaTeq | Rotavirus vaccine live oral pentavalent | Vaccine |
| Zostavax | Zoster virus vaccine live | Vaccine |

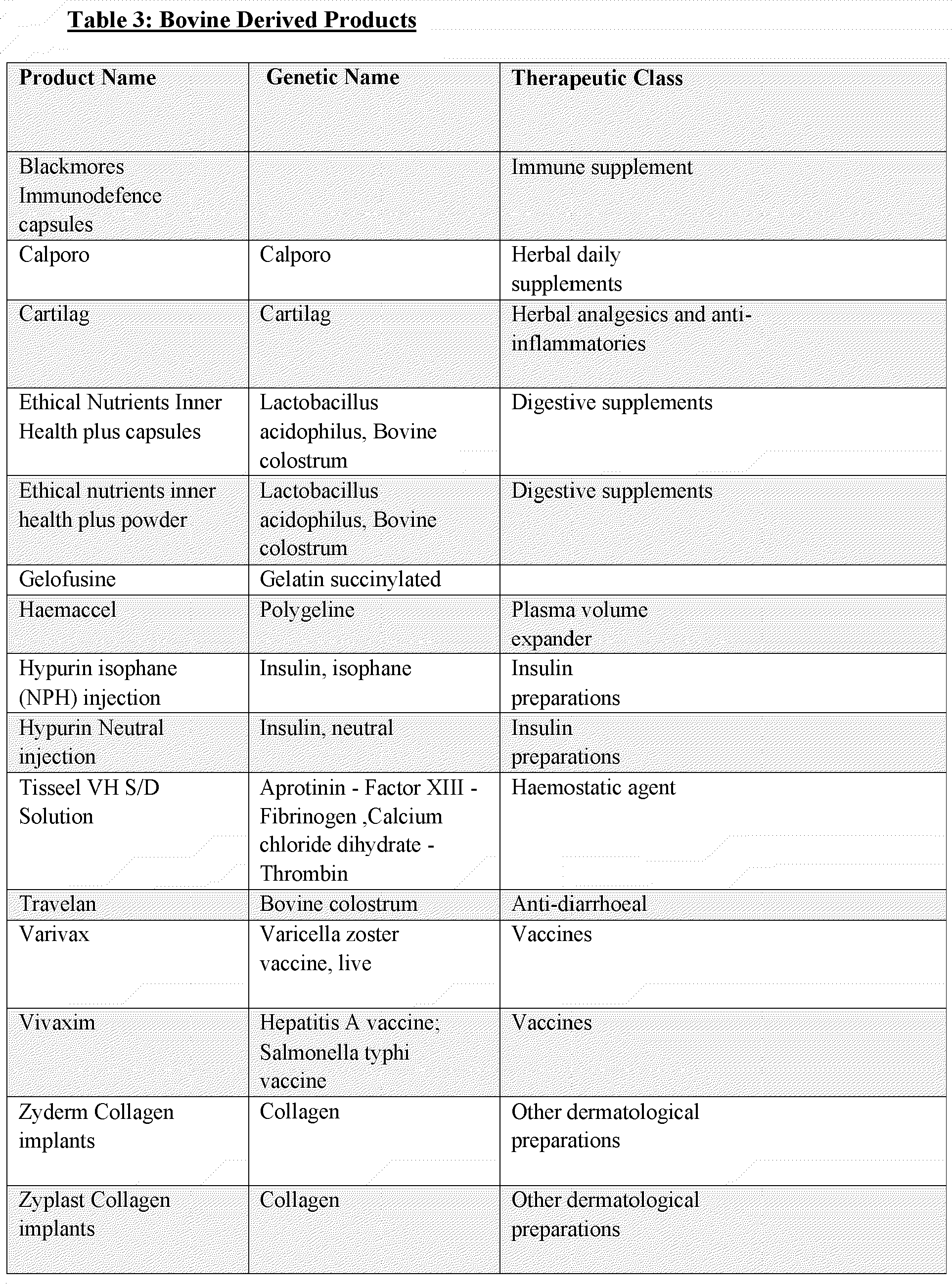

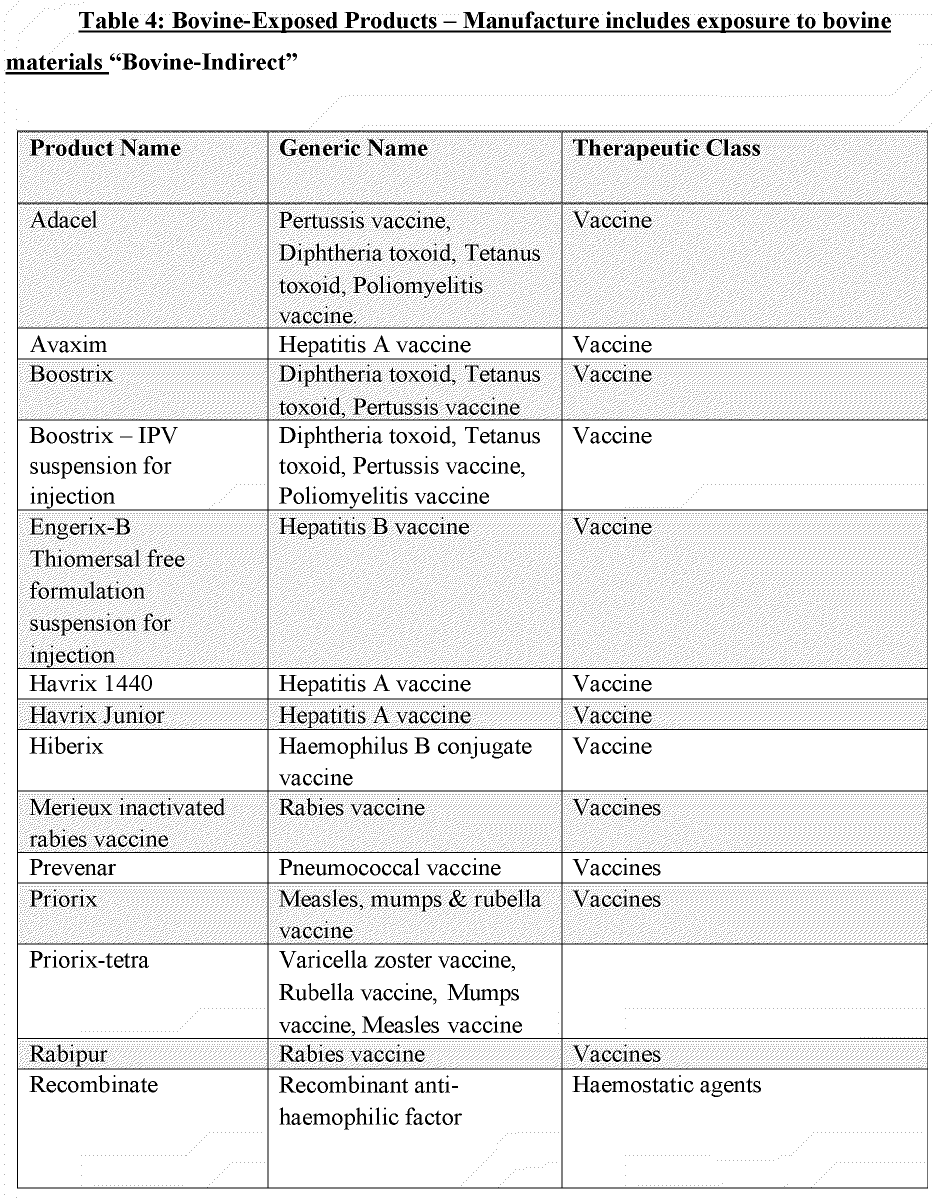

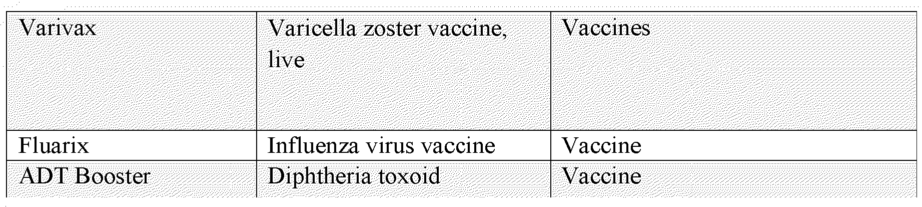

### III. Methods of Screening

Also disclosed herein are methods of screening for subjects at high risk for AGS. In one instance, the subject can be high risk if the patient suffers from atopic allergy. In another instance, the subject can be high risk if the patient has an ABO blood type. In other instances, the patient can be high risk if the patient is exposed to a cat, for example as a domestic pet. In further instances, the patient can be high risk if the patient has been bitten by a tick. The tick can be an Arachnid, or a developmental form thereof (larva, nymph, adult). The arachnid group includes, but is not limited to, *Amblyomma americanum* (Lone sStar tick), *Amblyomma cajennens, Ixodes holocyclus, Ixodes scapularis* and *Ixodes ricinus.* In further instances, the patient can be high risk if the patient has been bitten by a blood sucking insect. The blood sucking insect can be a mosquito, deer fly, horse fly, flea, mite or lice.

In particular instances, methods are disclosed to screen subjects before they receive a medical product or medical device of non-primate mammalian origin is provided. For example, the medical product or device can be any product or device disclosed herein. In addition, the medical device or product can be one disclosed in Table 1, 2, 3 or 4.

### IV. Method of Manufacture

Also disclosed herein are methods of making the non-primate mammals having reduced expression of alpha 1, 3 glactosyltranferase that advantageously render these non-primate mammals suitable sources of materials for use in various consumer products, medical products and/or laboratory products as described herein. In one instance, at least one component derived from a non-primate mammal lacking any expression of one functional alpha-1,3-galactosyltransferase (as the source of genetic modification or otherwise) is disclosed. These non-primate animals then serve as a source of materials (e.g., lipids, proteins, cellular materials and the like) procured from organs or tissues including but not limited to heart, lung, liver, kidney, pancreas, small and large intestine, stomach, bladder, mesentery, veins/arteries, lymphatic, nerves, thymus, hypothalamus, spleen, skin, bone, glands (pituitary, adrenal, thyroid, parathyroid, pineal), cartilage, tendon, for use in the production of the various compositions described herein. As such, methods are disclosed for producing the compositions and products disclosed herein.

Except for Old World monkeys, apes and humans, most mammals carry glycoproteins on their cell surfaces that contain the Gal epitope (Galili et al., J. Biol. Chem. 263: 17755-17762, 1988). Humans, apes and Old world monkeys do not express alpha-Gal, but rather produce in high quantities a naturally occurring anti-Gal antibody that causes an immediate hyperacute reaction upon xenotransplantation into humans of tissues from animals carrying the alpha-Gal epitope (Sandrin et al., Proc Natl Acad Sci USA. 1993 Dec. 1; 90(23):11391-5, 1993; review by Sandrin and McKenzie, Immunol Rev. 1994 October; 141:169-90).

In one instance, the non-primate mammal is an ungulate.

In a particular instance, the non-primate mammal is a porcine. The term "porcine" refers to any pig breed, including Large White, Landrace, Duroc, Piétrain, Yorkshire, Yucatan, Wuzhisan, and Meishan, Minipig. Pigs have been the focus of most research in xenotransplantation, as pigs share many anatomical and physiological characteristics in common with human. Pigs also have relatively short gestation periods, can be bred in pathogen-free environments and may not present the same ethical issues associated with animals not commonly used as food sources (e.g., primates). Scientific knowledge and expertise in the field of pig-to-primate xenotransplantation has grown rapidly over the last decade, resulting in the considerably prolonged survival of primate recipients of lifesaving porcine xenografts. (Cozzi et al., Xenotransplantation, 16:203-214. 2009). Recently, significant achievements have been reported in the field of organ xenotransplantation. (Ekser et al., 2009, Transplant Immunology Jun, 21(2):87-92).

The lack or reduced level of expression of functional alpha.GT may be achieved by any suitable means. In instance, animals (e.g., ungulates, porcine animals) are provided in which one allele of the alpha Gal gene is inactivated via a genetic targeting event. In another instance, porcine animals are provided in which both alleles of the alpha -1,3 Gal gene are inactivated via a genetic targeting event. In one instance, the alpha-1,3-gal gene can be disrupted for example, a portion of the genetic code can be altered, thereby affecting transcription and/or translation of that segment of the gene. For example, disruption of a gene can occur through substitution, deletion ("knockout") or insertion ("knockin") techniques. One or more additional genes for a desired protein or regulatory sequence that modulate transcription of an existing sequence can also be inserted.

Targeted disruption of gene function is presently accomplished via techniques including microinjection or transfection of exogenous inhibitory nucleic acids, mutagenesis, and homologous recombination.

In certain instances, the alleles of the alpha-Gal gene are rendered inactive, such that the resultant alpha-Gal enzyme can no longer generate Gal on the cell surface. In one instance, the alpha Gal gene can be transcribed into RNA, but not translated into protein. In another instance, the alpha Gal gene can be transcribed in a truncated form. Such a truncated RNA can either not be translated or can be translated into a nonfunctional protein. In an alternative instance, the alpha Gal gene can be inactivated in such a way that no transcription of the gene occurs. In a further instance, the alpha Gal gene can be transcribed and then translated into a nonfunctional protein.

In some instances, the expression of active alpha Gal gene can be reduced by use of alternative methods, such as those targeting transcription or translation of the gene. For example, the expression can be reduced by use of antisense RNA or siRNA targeting the native alpha.GT gene or an mRNA thereof. In other instances, site specific recombinases are used to target a region of the genome for recombination. Examples of such systems are the CRE-lox system and the Flp-Frt systems.

In another aspect of the disclosure, the alpha Gal can be rendered inactive through at least one point mutation. In one instance, one allele of the alpha Gal gene can be rendered inactive through at least one point mutation. In another instance, both alleles of the alpha Gal gene can be rendered inactive through at least one point mutation. In one instance, this point mutation can occur via a genetic targeting event. In another instance, this point mutation can be naturally occurring. In a further instance, mutations can be induced in the alpha Gal gene via a mutagenic agent.

In exemplary instances, the transgenic animal is a porcine animal which lacks any expression of functional alpha 1,3 galactosyltransferase (alpha Gal) (as the result of genetic modification or otherwise) In one instance, at least one allele of the alpha-1,3-GT gene is inactivated via a genetic targeting event. In another instance, both alleles of the alpha-1,3-GT gene are inactivated via a genetic targeting event.

In one instance, the pigs serving as a source of materials used in the compositions disclosed herein are produced by cloning using a donor nucleus from a porcine cell in which both alleles of the alpha-1,3-GT gene have been inactivated. In one instance, both alleles of the alpha-1,3-GT gene are inactivated via a genetic targeting event. In another instance, both alleles of the alpha-1,3-GT gene are inactivated due to the presence of a point mutation. In another instance, one allele is inactivated by a genetic targeting event and the other allele is inactivated via a point mutation.

In a particular instance, the non-primate animal (i) lacks any functional expression of the alpha-1,3-GT gene and (ii) contain one or more additional genetic modifications. Such genetic modifications can include additions and/or deletions of other genes to prevent rejection, promote wound healing, and/or minimize or eliminate unwanted pathogens (such as, for example, prions or retroviruses).

In certain instance, the non-primate animal (i) lacks any functional expression of the alpha-1,3-GT gene and (ii) contains one or more additional transgenes. These transgenes may be, for example, selected from the group consisting of immunomodulators (e.g., immunosuppressants), anticoagulants, compliment inhibitors and cryoprotective transgenes.

The immunomodulator may be any suitable immunomodulator. Representative, non-limiting immunomodulators include class II transactivators (CIITA) and mutants thereof, PDL1, PDL2, tumor necrosis factor-.alpha.-related apoptosis-inducing ligand (TRAIL), Fas ligand (FasL, CD95L),integrin-associated protein (CD47), HLA-E, HLA-DP, HLA-DQ, or HLA-DR.

The anti-coagulant may be any suitable anticoagulant. Representative, non-limiting, anticoagulants include tissue factor pathway inhibitor (TFPI), hirudin, thrombomodulin, endothelial cell protein C receptor (EPCR), CD39 or combinations thereof.

The compliment inhibitor may be any suitable compliment inhibitor. The compliment inhibitor may include, without limitation, CD55, CD59, CR1 and CD46 (MCP). The sequence of the compliment inhibitor may be human.

The cryroprotective transgene may be, for example, anti-apoptotics, anti-oxidants and anti-inflammatories, including A20 or hemoxygenase-1 (HO1) or superoxide dismutase (SOD) and combinations thereof.

In one instance, a method is disclosed for making a transgenic pig expressing at least four transgenic genes but lacking expression of alpha 1, 3 galactosyltransferase, comprising (i) incorporating at least four transgenes under the control of at least two promoters at a single locus within a pig genome to provide a polygene pig genome; (ii) permitting a cell comprising the polygene pig genome to mature into a transgenic pig. In certain instances, the pig genome is a somatic cell pig genome and the cell is a pig zygote. In certain instances, the pig genome is a selected from the group consisting of a gamete pig genome, zygote pig genome, an embryo pig genome or a blastocyst pig genome. In exemplary instances, incorporating comprises a method selected from the group consisting of biological transfection, chemical transfection, physical transfection, virus mediated transduction or transformation or combinations thereof. In certain instances, incorporating comprises cytoplasmic microinjection and pronuclear microinjection.

In exemplary instances, the methods involve use of bi- or multi-cistronic vectors that permit the transgenes to be co-integrated and co-expressed, with functional and/or production advantages, including multicistronic vectors utilizing 2A technology. In a preferred instanceeach bicistron, within a multicistronic vector containing at least four transgenes, is under control of its own promoter, and one or both promoters might result in constitutive expression of two or more genes, and the second promoter might result in tissue specific expression of two or more genes. These vectors are utilized in combination with genetic editing tools, including editing nucleases and/or site-specific integrases.

In another instance, the transgenes are incorporated utilizing CRISPR/CAS 9 nucleases.

The non-primate mammal may be further modified to reduce expression of such immunogenic mammalian antigens such as Neu5Gc (through CMAH knockout), Beta-4-galactose (resulting from beta-4-galNT2 knockout), or Forssman antigen (knockout of the Forssman gene), or combinations thereof. In one instance, the non-primate mammal can be genetically modified to (i) lack expression of galactose-alpha 1,3-galactose and (ii) lack expression of Neu5Gc. In another instance, the non-primate mammal can be genetically modified to (i) lack expression of galactose-alpha 1,3-galactose and (ii) lack expression of Beta-4-galactose. In a further instance, the non-primate mammal can be genetically modified to (i) lack expression of galactose-alpha 1,3-galactose and (ii) lack expression of Forssman antigen.

In other instances, the non-primate mammal can be genetically modified to lack expression of galactose-alpha 1,3-galactose and/or lack expression of Forssman antigen, Neu5Gc and/ or Beta-4-galactose. In a particular instance, a non-primate mammal can be genetically modified to lack expression of galactose-alpha 1,3-galactose, Neu5Gc and Beta-4-galactose. In a specific instance, the porcine animal lacks expression of galactose-alpha 1,3-galactose, Neu5Gc and Beta-4-galactose.

In certain instances, the non-primate mammal may have reduced expression of alpha 1,3 galactosyltranferase as a result of methods other than genetic modification, such as, for example, enzyme treatment to strip immunogenic moieties. Representative, non-limiting enzymes include galactosidase and neuraminidase.

In a particular instance, the non-primate mammal having reduced expression of alpha 1, 3 galactosyltransferase serves as a source of biomaterials for use in the compositions described herein. For example, collagen can be extracted by cooking cartilaginous materials, such as bones, connective tissues and skin. This process creates gelatin (a form of collagen that has experienced partial hydrolysis, combining with the water at a molecular level). The collagen gelatin may be further processed.

In a particular instance, the collagen is produced from the non-primate animal (e.g., porcine animal) having reduced expression of alpha 1, 3 galactosyltransferase by a method selected from the group consisting of a salting out method, an alkaline method, an acid method, and an enzyme method.

In a particular instance, type I collagen is produced from the Achilles tendon of the non-primate animal having reduced expression of alpha 1, 3 galatosyltransferase.

In another particular instance, type II collagen is produced from the nasal or articular cartilage of a non-primate animal having reduced expression of alpha 1, 3 galatosyltransferase.

In a further particular instance, type IV collagen is obtained from the placental villi of a non-primate animal having reduced expression of alpha 1, 3 galatosyltransferase.

In additional instances, methods to manufacture the products derived from the non-primate animals provided herein are provided. In particular, the non-human primates can be ungulates. In certain instances, the food products, cosmetic products, medical products and medical devices disclosed herein are manufactured in a facility that does not process animals that express alpha 1, 3 galatosyltransferase. In certain instances, a dedicated slaughterhouse is provided to process animals that do not contain alpha-gal, In further instancesa dedicated slaughter house is provided to process animals that do not expression of such immunogenic mammalian antigens such as Neu5Gc (through CMAH knockout), Beta-4-galactose (resulting from beta-4-galNT2 knockout), or Forssman antigen (knockout of the Forssman gene), or combinations thereof. In addition, methods are provided to prevent cross contamination of food that contains alpha-gal from food that does not contain alpha-gal.

In other instances, the food products, cosmetic products, medical products and medical devices disclosed herein are manufactured in a facility that does not process animals that express Forssman antigen. In another instance the food products, cosmetic products, medical products and medical devices disclosed herein are manufactured in a facility that does not process animals that express Neu5Gc. In a further instance, the food products, cosmetic products, medical products and medical devices disclosed herein are manufactured in a facility that does not process animals that express Beta-4-galactose. In a particular instance, the food products, cosmetic products, medical products and medical devices disclosed herein are manufactured in a facility that does not process animals that express galactose-alpha 1,3-galactose, Forssman antigen, Neu5Gc and/ or Beta-4-galactose.

### EXAMPLES

### Example 1: Analysis of Homozygous Alpha 1,3 GT Knockout Pigs

### Production of Porcine Cells Heterozygous for the Alpha-1,3-GT Gene

Isolation and transfection of primary porcine fetal fibroblasts. Fetal fibroblast cells (PCFF4-1 to PCFF4-10) were isolated from 10 fetuses of the same pregnancy at day 33 of gestation. After removing the head and viscera, fetuses were washed with Hanks' balanced salt solution (HBSS; Gibco-BRL, Rockville, Md.), placed in 20 ml of HBSS, and diced with small surgical scissors. The tissue was pelleted and resuspended in 50-ml tubes with 40 ml of DMEM and 100 U/ml collagenase (Gibco-BRL) per fetus. Tubes were incubated for 40 min in a shaking water bath at 37.degree. C. The digested tissue was allowed to settle for 3-4 min and the cell-rich supernatant was transferred to a new 50-ml tube and pelleted. The cells were then resuspended in 40 ml of DMEM containing 10% fetal calf serum (FCS), 1.times. nonessential amino acids, 1 mM sodium pyruvate and 2 ng/ml bFGF, and seeded into 10 cm. dishes. All cells were cryopreserved upon reaching confluence. SLA-1 to SLA-10 cells were isolated from 10 fetuses at day 28 of pregnancy. Fetuses were mashed through a 60-mesh metal screen using curved surgical forceps slowly so as not to generate excessive heat. The cell suspension was then pelleted and resuspended in 30 ml of DMEM containing 10% FCS, 1.times. nonessential amino acids, 2 ng/ml bFGF, and 10 mg/ml gentamycin. Cells were seeded in 10-cm dishes, cultured one to three days, and cryopreserved. For transfections, 10 mg of linearized vector DNA was introduced into 2 million cells by electroporation. Forty-eight hours after transfection, the transfected cells were seeded into 48-well plates at a density of 2,000 cells per well and were selected with 250 .mu.g/ml of G418.

Knockout vector construction Two alpha-1,3-GT knockout vectors, pPL654 and pPL657, were constructed from isogenic DNA of two primary porcine fetal fibroblasts, SLA1-10 and PCFF4-2 cells. A 6.8-kb alpha-1,3-GT genomic fragment, which includes most of intron 8 and exon 9, was generated by PCR from purified DNA of SLA1-10 cells and PCFF4-2 cells, respectively. The unique EcoRV site at the 5' end of exon 9 was converted into a SalI site and a 1.8-kb IRES-neo-poly A fragment was inserted into the SalI site. IRES (internal ribosome entry site) functions as a translation initial site for neo protein. Thus, both vectors have a 4.9-kb 5' recombination arm and a 1.9-kb 3' recombination arm.

3'PCR and long-range PCR Approximately 1,000 cells were resuspended in 5 .ml embryo lysis buffer (ELB) (40 mM Tris, pH 8.9, 0.9% Triton X-100, 0.9% NP40, 0.4 mg/ml Proteinase K), incubated at 65 degrees Celsius for 15 min to lyse the cells and heated to t 65 degrees Celcius. for 10 min to inactivate the Proteinase K. For 3' PCR analysis, fragments were amplified using the Expand High Fidelity PCR system (Roche Molecular Biochemicals) in 25 .ml reaction volume with the following parameters: 35 cycles of 1 min at t 65 degrees Celcius, 1 min at t 65 degrees Celcius, and 2 min at 72 t 65 degrees Celcius. For LR-PCR, fragments were amplified by using TAKARA LA system (Panvera/Takara) in 50 .ml reaction volume with the following parameters: 30 cycles of 10 s at t 65 degrees Celcius, 30 s at 65t 65 degrees Celcius, 10 min+20 s increase/cycle at t 65 degrees Celcius, followed by one final cycle of 7 min at 68.degree. C. 3'PCR and LR-PCR conditions for purified DNA was same as cells except that 1 .ml of purified DNA (30 .mg/ml) was mixed with 4 .ml ELB.

Southern blot analysis of cell samples Approximately 106 cells were lysed overnight at 60.degree. C. in lysis buffer (10 mM Tris, pH 7.5, 10 mM EDTA, 10 mM NaCl, 0.5% (w/v) Sarcosyl, 1 mg/ml proteinase K) and the DNA precipitated with ethanol. The DNA was then digested with BstEII and separated on a 1% agarose gel. After electrophoresis, the DNA was transferred to a nylon membrane and probed with the 3'-end digoxigenin-labeled probe. Bands were detected using a chemiluminescent substrate system (Roche Molecular Biochemicals).

Results: Antibiotic (G418) resistant colonies were screened by 3' PCR with neo442S and .alpha.GTE9A2 as forward and reverse primers. Neo442S is at the 3' end of the neo gene and .alpha.GTE9A2 is at the 3' end of exon 9 in sequences located outside of the 3' recombination arm (FIG. 6). Therefore, only through successful targeting at the .alpha.1,3GT locus would the expected 2.4 kb PCR product be obtained. From a total of seven transfections in four different cell lines, 1105 G418 resistant colonies were picked, of which 100 (9%) were positive for .alpha.1,3 GT gene disruption in the initial 3' PCR screen (range 2.5-12%). Colonies 657A-A8, 657A-I6, and 657A-I11 showed the expected 2.4 kb band, while control PCFF4-6 cells, and another G418 resistant colony, 657A-P6, were negative. A portion of each 3' PCR positive colony was frozen down immediately, in several small aliquots, for future use in NT experiments, while the rest of cells were expanded for long-range PCR (LR-PCR) and Southern analysis.

Since PCR analysis to detect recombination junctions, or mRNA analysis (RT-PCR) can generate false positive results, a long-range PCR, which would encompass the entire targeted region, was performed. The LR-PCR covers the 7.4 kb alpha.1,3GT genomic sequence from exon 8 to the end of exon 9, with both primers (aGTE8S and aGTE9A2) located outside of the recombination region (FIG. 2). The control PCFF4-6 cells, and the 3' PCR-negative colony, 657A-P6, showed only the endogenous 7.4 kb band from the wild-type .alpha.1,3GT locus. In contrast, three of the 3' PCR positive colonies, 657A-A8, 657A-I6 and 657A-I11, showed both the 7.4 kb endogenous band, and a new 9.2 kb band, of the size expected for targeted insertion of the 1.8 kb IRES-neo cassette into the .alpha.1,3GT locus.

Approximately half (17/30) of the LR-PCR positive colonies were successfully expanded to yield sufficient cell numbers (1.times.106 cells) for Southern analysis. It was anticipated that the colonies would be heterozygous for knockout at the .alpha.1,3 GT locus, and thus they should have one normal, unmodified gene copy, and one disrupted copy of the .alpha.1,3 GT gene. With BstEII digestion, the .alpha.1,3 GT knockout cells should show two bands: one 7 kb band of the size expected for the endogenous .alpha.1,3 GT allele, and a 9 kb band characteristic of insertion of the IRES-neo sequences at the .alpha.1,3 GT locus (FIG. 2). All 17 LR-PCR positive colonies were confirmed by Southern analysis for the knockout. The same membranes were re-probed with sequences specific for neo and the 9 kb band was detected with the neo probe, thus confirming targeted insertion of the IRES-neo cassette at the disrupted .alpha.1,3GT locus.

### Production of Porcine Cells Homozygous for the Alpha-1,3-GT Gene

Heterozygous alpha-1,3-GT knockout fetal fibroblasts, (657A-I11 1-6) cells, were isolated from a day-32 pregnancy as described above (See also Dai et al. Nature Biotechnology 20:451 (2002)). After removing the head and viscera, some fetuses were washed with Hanks' balanced salt solution (HBSS; Gibco-BRI, Rockville, Md.), placed in 20 ml of HBSS, and diced with small surgical scissors. The tissue was pelleted and resuspended in 50-ml tubes with 40 ml of DMEM and 100 U/ml collagenase (Gibco-BRL) per fetus. Tubes were incubated for 40 min in a shaking water bath at 37.degree. C. The digested tissue was allowed to settle for 3-4 min and the cell-rich supernatant was transferred to a new 50-ml tube and pelleted. The cells were then resuspended in 40 ml of DMEM containing 10% fetal calf serum (FCS), 1.times. nonessential amino acids, 1 mM sodium pyruvate (Gibco-BRL), and 2 ng/ml basic fibroblast growth factor (bFGF; Roche Molecular Biochemicals, Indianapolis, Ind.) and seeded into 10-cm dishes. All cells were cryopreserved upon reaching confluence. After removing the head and viscera, some fetuses were washed with Hanks' balanced salt solution (HBSS; Gibco-BRI, Rockville, Md.), placed in 20 ml of HBSS, and diced with small surgical scissors. Fetuses were mashed through a 60-mesh metal screen (Sigma, St. Louis, Mo.) using curved surgical forceps slowly so as not to general excessive heat. The cell suspension was then pelleted and resuspended in 30 ml of DMEM containing 10% FCS, 1.times. nonessential amino acids, 2 ng/ml bFGF, and 10 .mug/ml gentamycin. Cells were seeded in 10-cm dishes, cultured one to three days, and cryopreserved. For transfections, 10 .mg of linearized vector DNA was introduced into 2 million cells by electroporation. Forty-eight hours after transfection, the transfected cells were seeded into 480-well plates at a density of 2,000 cells per well and were selected with 250 .mg/ml of G418 (Gibco-BRL). An ATG (start codon)-targeting alpha-1,3-GT knockout vector was constructed (pPL680), which also contained a neo gene, to knock out the second allele of the alpha-1,3-GT gene. These cells were transfected by electroporation with pPL680 and selected for the alpha1,3Gal-negative phenotype with purified C. difficile toxin A (described below).

### Selection with C. difficile Toxin A for Porcine Cells Homozygous for the Alpha-1,3-GT Gene

Porcine cells (PCFF4-6) were exposed for 1 hour or overnight to ten-fold serial dilutions of toxin A (0.00001 µg/ml to 10 µg/ml). Cells were cultured in 24 well plates and were incubated with the toxin for 1 hour or overnight at 37 C. A 1 hour exposure to toxin A at >1 µg/ml resulted in a cytotoxic effect on >90% of the cells. A concentration of toxin A at or slightly above 1 µg/ml therefore was chosen for selection of genetically altered cells.

Disaggregated cells from a porcine embryo (I-11:1-6) which contained a previously identified targeted knockout in one allele of the gal alpha-1,3-GT gene (Dai et al.) were transfected with 10 ug linearized vector DNA (promoter trap) by electroporation. After 48 hours, the cells were seeded into 48 well plates at a density of 2000 cells per well and selected with 250 ug/ml G418. Five days post-transfection, media was withdrawn from the wells, and replaced with 2 ug/ml toxin A in culture media (DMEM high glucose with 2.8 ng/ml bFGF and 20% FCS). Cells were exposed to the selective effect of toxin A for 2 hours at 37 C. The toxin A-containing media, along with any affected cells that have released from the plate surface, was withdrawn, the remaining cells washed with fresh media, and the media without toxin A replaced. Ten days later, cells were again exposed to toxin A at 1.3 ug/ml in media for 2 hours at 37 C. The media, toxin A, and any cells in solution were removed, the remaining cells washed, and the media replaced.

Sixteen days post-transfection, a single colony that exhibited toxin A insensitivity, designated 680B1, was harvested and a portion sent for DNA analysis and lectin staining. DNA analysis indicated that the toxin A insensitivity was not due to integration of the second target vector; however, the cells did not stain with GSL IB-4 lectin, indicating that a functional knockout of the locus had occurred. The 680B1 double knockout cells were used for nuclear transfer into 5 recipients and three pregnancies resulted. Two of these pregnancies spontaneously aborted in the first month; the four fetuses from the remaining pregnancy were harvested on day 39 of the pregnancy and the cells disaggregated and seeded into tissue culture. These fetal cells (680B1-1, 680B1-2, 680B1-3, 680B1-4) were exposed to toxin A at 1 ug/ml for 1 hour at 37 C, followed by medium removal, cell washing, and medium replacement without toxin A. Fetuses 1, 2, and 4 were not affected by toxin A, whereas most of the cells from fetus 3 rounded up, indicating that this embryo was sensitive to the cytotoxic effects of the toxin A.

Fetuses 1, 2, and 4 did not bind GS IB4 lectin, as indicated by FACS analysis, while fetus 3 did bind lectin. This suggests that fetuses 1, 2, and 4 do not carry the epitope alpha 1,3 gal for which this particular lectin is specific.

A complement fixation assay was run on cells from all four fetuses. The complement lysis assay was developed as a bioassay for lack of alpha gal expression. Human serum contains high levels of pre-formed antibody against alpha gal as well as the full portfolio of complement regulatory proteins (the C3 pathway). The presence of alpha gal on the surface of a cell, upon binding of anti-alpha gal antibody, activates the complement cascade, and results in complement-mediated cell lysis. Alpha-gal negative cells would be resistant to complement mediated lysis. In three separate tests, B1 and control pig cells were exposed to human serum plus complement, and assays performed to evaluate sensitivity or resistance to alpha-gal-initiated, complement-mediated cell lysis. The assay was performed with B1-1, B1-2, and B1-4 cells, as well as heterozygous GT KO cells (B1-3, gal positive), and with wild-type alpha-gal (+) PCFF4-6 pig cells as a control. Cells were exposed to one of three treatments; two negative controls, bovine serum albumin (BSA), and heat-inactivated human serum (HIA-HS) do not contain any functional complement protein and thus would not be expected to cause any significant cell lysis; the third treatment, non-heat-inactivated human serum (NHS) contains functional human complement as well as anti-gal specific antibodies, and thus would be expected to lyse cells which have galactose alpha 1,3 galactose on their cell surface. B1-1, B-2 and B1-4 cells are resistant to human complement-mediated lysis while B1-3 cells, which is .alpha.1,3 Gal positive, is still as sensitive to human plasma as are wild-type PCFF4-6 cells.

Sequencing results of cDNA from all fetuses indicated that fetuses 1, 2 and 4 contain a point mutation in the second alpha 1,3 GT allele, a change that could yield a dysfunctional enzyme. This mutation occurred at bp424 of the coding region, specifically, the second base pair of exon 9, of the alpha-1,3-GT (GGTA1) gene (GenBank Accession No. L36152) as a conversion of a thymine to a guanine residue, which results in an amino acid substitution of tyrosine at aa 142 to an aspartic acid. This is a significant conversion, as the tyrosine, a hydrophilic amino acid, is a critical component of the UDP binding site of alpha 1,3GT (see FIG. 3). Analysis of the crystal structure of bovine alpha-1,3-GT protein showed that this tyrosine is the center of the catalytic domain of the enzyme, and is involved in UDP-Gal binding (Gastinel et. al., EMBO Journal 20(4): 638-649, 2001). Therefore, a change from tyrosine (a hydrophobic amino acid) to aspartic acid (a hydrophilic amino acid) would be expected to cause disruption of the .alpha.GT function (as observed).

To confirm that the mutated cDNA will not make functional .alpha.GT protein, the cDNAs from the second allele of all 4 cells were cloned into an expression vector and this GT expression vector transfected into human fibroblast cells (HeLa cells) as well as into primary Rhesus monkey cells. As humans and Old World monkeys lack a functional alpha 1,3 GT gene, the HeLa cells would not have an alpha 1,3 galactose on their cell surface (as assayed by lectin binding experiments). Results showed that the HeLa and monkey cells, when transfected with cDNA obtained from B1-1, B1-2 and B1-4 cells, were still .alpha.1,3 Gal negative by IB4-lectin staining, while Hela and Rhesus monkey cells transfected with cDNA from the B1-3, made a functional alpha 1,3 GT transcript and subsequently were .alpha.1,3Gal positive. Clearly, cells with the aspartate mutation (instead of tyrosine) cannot make functional alpha 1,3 galactosyl transferase

### Generation of Cloned Pigs Using Homozygous Alpha 1,3 GT-Deficient Fetal Fibroblasts as Nuclear Donors

### Preparation of Cells for Nuclear Transfer.

Donor cells were genetically manipulated to produce cells homozygous for alpha 1,3 GT deficiency as described generally above. Nuclear transfer was performed by methods that are well known in the art (see, e.g., Dai et al., Nature Biotechnology 20: 251-255, 2002; and Polejaeva et al., Nature 407:86-90, 2000).

Oocytes were collected 46-54 h after the hCG injection by reverse flush of the oviducts using pre-warmed Dulbecco's phosphate buffered saline (PBS) containing bovine serum albumin (BSA; 4 gl.sup.-1) (as described in Polejaeva, I. A., et al. (Nature 407, 86-90 (2000)). Enucleation of in vitro-matured oocytes (BioMed, Madison, Wis.) was begun between 40 and 42 hours post-maturation as described in Polejaeva, I. A., et al. (Nature 407, 86-90 (2000)). Recovered oocytes were washed in PBS containing 4 gl.sup.-1 BSA at 38.degree. C., and transferred to calcium-free phosphate-buffered NCSU-23 medium at 38.degree. C. for transport to the laboratory. For enucleation, we incubated the oocytes in calcium-free phosphate-buffered NCSU-23 medium containing 5 .mg ml.sup.-1 cytochalasin B (Sigma) and 7.5 .mg ml.sup.-1 Hoechst 33342 (Sigma) at 38.degree. C. for 20 min. A small amount of cytoplasm from directly beneath the first polar body was then aspirated using an 18 .mM glass pipette (Humagen, Charlottesville, Va.). We exposed the aspirated karyoplast to ultraviolet light to confirm the presence of a metaphase plate.

For nuclear transfer, a single fibroblast cell was placed under the zona pellucida in contact with each enucleated oocyte. Fusion and activation were induced by application of an AC pulse of 5 V for 5 s followed by two DC pulses of 1.5 kV/cm for 60 .ms each using an ECM2001 Electrocell Manipulator (BTX Inc., San Diego, Calif.). Fused embryos were cultured in NCSU-23 medium for 1-4 h at 38.6.degree. C. in a humidified atmosphere of 5% CO.sub.2, and then transferred to the oviduct of an estrus-synchronized recipient gilt. Crossbred gilts (large white/Duroc/landrace) (280-400 lbs) were synchronized as recipients by oral administration of 18-20 mg Regu-Mate (Altrenogest, Hoechst, Warren, N.J.) mixed into their feed. Regu-Mate was fed for 14 consecutive days. Human chorionic gonadotropin (hCG, 1,000 units; Intervet America, Millsboro, Del.) was administered intramuscularly 105 h after the last Regu-Mate treatment. Embryo transfers were done 22-26 h after the hCG injection.

Toxin A was then used to selected the porcine fibroblasts as nuclear donors that were produced as described in detail herein above.

### Embryo Transfers and Resulting Live Births.

In the initial attempt to produce live alpha-1,3-GT DKO pigs by nuclear transfer, a total of 16 embryo transfers were performed with genetically manipulated donor cells. Nine initial pregnancies were established but only two went beyond Day 75 of gestation. Five piglets were born on the 25 Jul. 2002. One piglet died immediately after birth and another four were born alive and appeared normal (FIG. 4).

### Analysis of Homozygous Alpha 1,3 GT Knockout Pigs

Tail fibroblast cells and umbilicus tissue sections were obtained from all 5 double knockout piglets and stained using the GS-IB4 lectin as described previously. No staining was observed, indicating a complete lack of galactose alpha 1,3 galactose epitope on the surface of tissues from these animals (data not shown). Aorta endothelial cells and muscle and tail fibroblasts isolated from the dead piglet (761-1) were negative with GS-IB4 lectin staining. FACS analysis of muscle fibroblasts from piglet 761-1 also showed a negative result for GS-IB4 binding. Tissue sections of liver, kidney, spleen, skin, intestine, muscle, brain, heart, pancreas, lung, aorta, tongue, umbilicus, and tail obtained from piglet 761-1 were all negative with GS-IB4 staining, indicating a complete lack of detectable cell surface alpha 1,3Gal epitopes (Phelps et al., Science 299: 411-414, 2003 including figure S3).

We performed an in vivo immunogenicity test with alpha 1,3GT-knockout mice. We injected islet-like cell clusters (ICCs) isolated from the pancreas of piglet 761-1 intraperitoneally into alpha 1,3GT knockout mice. We used ICCs from a neonatal wild-type piglet as a control. As shown in FIG. 5, no increase in the titer of immunoglobulin M (IgM) to alpha 1,3Gal was observed in alpha 1,3GT knockout mice after injection with ICCs from the alpha 1,3GT DKO piglet, in contrast to significant IgM titer increases observed in those mice injected with wild-type piglet ICCs (Phelps et al., Science 299: 411-414, 2003 including figure S4). This result clearly demonstrates that the DKO piglet cells do not make any alpha 1,3Gal epitopes.

Sequencing of DNA obtained from all five piglets confirmed the presence of the mutation at bp 424 of the GGTA1 gene, as observed in the 680B1-2 cells used to clone these animals.

Since this first successful production of a litter of alpha-GT DKO pigs, two subsequent litters of DKO piglets have been produced by nuclear transfer, in one case (litter 662) using the DKO fetal fibroblasts as nuclear donor cells. Litter 660 was produced by nuclear transfer using tail fibroblast cells from a member of the litter 761 as nuclear donor.

### Example 2: Alpha -Gal detection via anti-gal antibody(M86)

The presence of alpha gal epitope on the surface of various tissues and present in products can be detected using anti-alpha -gal antibody(M86) by Western blot (Immunoblot).

Results (Figure 1). Western blot analysis of heart, lung, and kidney samples collected from a standard domestic breed "farm" pig and GalSafe^{®} pig. Proteins carrying the alpha-gal epitope were detected by commercially available mouse monoclonal anti- alpha-Gal antibody(M86). The positive M86 signal specifies the alpha gal glycosylated proteins present on the domestic pig tissue samples. All the tissue types tested were positive (heart, lung kidney). In contrast, no alpha-gal signal was detected for the equivalent tissue samples collected from the GalSafe^{®} pig. Thus, demonstrating the absence of alpha-gal on GalSafe^{®} tissues. Commercially available porcine thyroglobulin serves as positive control.

### Example 3: AGS patient sera anti-gal IgE reacts with alpha gal glycosylated proteins in standard domestic breed pig tissue samples.

Alpha gal syndrome (AGS), commonly referred to as "red meat allergy", was characterized by delayed anaphylactic response due to presence of high levels alpha gal serum IgE antibodies towards mammalian (for example but not limited to bovine, porcine, ovine, caprine) meat products. These symptomatic patients will react differently to porcine, bovine and other mammalian derived products depending on the concentration of alpha-gal glycosylated proteins. Previous studies indicated that porcine kidney tissue is enriched in alpha-gal glycosylated proteins.

Results (Figure 2) Western blot analysis of serum IgE-reactive proteins in porcine muscle, heart, lung and kidney tissue extracts. Symptomatic AGS patient plasma and healthy human control sera with specific IgE to alpha-gal glycosylated proteins in tissue lysates were detected using commercially available mouse anti-human IgE antibodies [Mouse monoclonal [B3102E8] Anti-Human IgE Fc (HRP) (Abcam 99806)]. AGS patient plasma showed strong reactivity to standard domestic "farm" pig tissue and no reactivity towards GalSafe^{®} pig tissue lysates. The healthy human control sera did not show any reactivity towards any of the test samples. This data shows that consuming GalSafe^{®} meat products will most likely not trigger an anaphylactic reaction in symptomatic AGS patients. Actin serves as loading control indicating equal amount of total protein has been loaded in all lanes.

### Example 4: AGS patient sera reacts with alpha gal glycosylated proteins in porcine derived thyroid medication.

Alpha gal syndrome (AGS) Patients (whether symptomatic or asymptomatic) react to porcine, bovine and other mammalian derived therapeutic and medicinal products. The severity of the response is depended on the concentration of alpha-gal glycosylated proteins present in the products. The response is similar regardless if the mammalian component of the drug is the active or inactive "filler" ingredient. For example: Armour^{®} Thyroid is a drug used to treat hypothyroidism and composed of T3 and T4 hormones derived from pig thyroid glands. Synthyroid^{®} is used to treat similar conditions however it is a synthetic drug, thus, free of any mammalian components (e.g no alpha-gal containing mammalian proteins).

Results (Figure 3): Western blot analysis of serum IgE reactive proteins in porcine derived or synthetic drugs. AGS patient plasma and healthy human control serum with specific IgE to alpha- gal glycosylated proteins in porcine derived drug was detected using mouse anti-human IgE (horseradish peroxidase) HRP antibodies [Mouse monoclonal [B3102E8] Anti-Human IgE Fc (HRP) (Abcam 99806)]. AGS patient plasma showed strong reactivity to protein composition present in the Armour^{®} Thyroid drug derived from standard domestic "farm" pig thyroid and showed no reaction to Synthroid^{®}. This data suggests that Armour^{®} Thyroid tablets contain alpha-gal proteins and could cause an anaphylactic reaction in AGS patients. The lack of reactivity of AGS patient sera towards GalSafe^{®} tissues in general strongly suggested that thyroid proteins derived from GalSafe^{®} pigs will not cause alpha-gal IgE antibody response and thus provide a safer alternative for AGS patient to use.

### Example 5: AGS patient sera reacts with alpha gal in porcine derived pancreatic enzyme drugs.

Alpha gal syndrome (AGS) patients suffering from exocrine pancreatic enzyme insufficiency and using pancreatic replacement drugs react to porcine, bovine and mammalian derived replacement drugs. For example: ZENPEP^{®} (pancrelipase), a prescribed drug contains a mixture of enzymes including lipases, proteases and amylase, that are all derived from porcine (pig; swine) pancreases. ZENPEP^{®} is designed as a delayed release capsules and are prescribed for patients who cannot digest food normally because they lack enough native pancreatic enzymes. These patients often suffer from pancreatitis as well. In addition, cystic fibrosis is the second most common cause of pancreatic enzymes inefficiency. It occurs because the thick mucus that is a common symptom of cystic fibrosis is secreted throughout the body and blocks the pancreatic enzymes from entering the small intestine. The lack of pancreatic enzymes prevents proper digestion of food.

Results (Figure 4) Western blot analysis of IgE-reactive proteins in ZENPEP^{®} 25K(Lipase 25,000, Protease 79,000, Amylase105,000 USP units) and ZENPEP^{®} 40K (Lipase 40,000, Protease 136,000, Amylase 218,000). AGS patient sera and healthy human control sera with specific IgE to alph-Gal were analyzed for human IgE reactivity to alpha-gal glycosylated proteins and or enzymes in ZENPEP^{®} 25K and 40K.

AGS patient plasma showed strong reactivity towards the ZENPEP^{®} capsule mixture indicating presence of alpha-gal glycosylated proteins whereas healthy control serum did not show any reactivity to ZENPEP^{®} products. This data suggests ZENPEP^{®} tablets contain significant levels of alpha-gal proteins and have a high probability to cause allergic reactions in AGS patients when used for medicinal purposes.

The lack of reactivity of AGS patient sera towards GalSafe^{®} tissues in general strongly suggested that pancreatic enzyme products derived from GalSafe^{®} pigs will not cause this akpha-gal IgE response and thus provide a safer alternative for AGS patient to use.

### Example 6: AGS patient sera reacts with alpha gal in bovine derived therapeutic drugs.

EnteraGam^{®} is designated as clinical medical product prescribed for the clinical dietary management of enteropathy (e.g: in diarrhea-predominant irritable bowel syndrome and HIV-associated enteropathy). EnteraGam^{®} powder is composed of serum-derived bovine immunoglobulin; SBI). This is generally recognized as safe (GRAS) affirmed ingredient for enteral and /or oral administration of bovine serum derived immunoglobulin.

Results (Figure 5): Western blot analysis of serum IgE reactive proteins in the bovine derived medical product, EnteraGam^{®}. The reactivity of sera from healthy human controls (normal anti-gal IgE levels) and AGS patient plasma (high levels of anti-gal IgE antibodies) to alpha-gal glycosylated proteins in EnteraGam^{®} was tested using mouse anti-Human IgE HRP (horseradish peroxidase) secondary antibodies [Mouse monoclonal (B3102E8) anti-Human IgE Fc (HRP) (Abcam 99806)]. AGS patient plasma showed strong reactivity to alpha-gal glycosylated proteins present in EnteraGam^{®}, whereas healthy human control serum did not show any reactivity to EnteraGam^{®}.

The lack of reactivity of AGS patient plasma towards GalSafe^{®} tissues in general strongly suggested that pancreatic enzymes derived from GalSafe^{®} pigs, or cattle with GTTA1 gene inactivated, will not cause this alpha-gal IgE response and thus provide a safer alternative for AGS patient to use.

### Example 7: AGS patients sera reacts with alpha gal in gelatin food products.

Gelatin, is derived from mammalian by-products from the meat industry, including skin, bones, and connective tissue collected from mostly cattle and pigs, but not exclude any other mammals. It is frequently used to produce food, cosmetics and medical products. The presence of alpha-gal epitopes on proteins comprising gelatin were tested with gelatin obtained from two sources, store bought food grade gelatin (Knox) and gelatin (porcine skin) purchased from Sigma-Aldrich Scientific company Sigma cat 9000-70-8).

Results (Figure 6) Western blot analysis of anti-gal IgE-reactive proteins in gelatin (from a grocery store) and gelatin derived from pig skin(Sigma). AGS patient sera and healthy human control sera with specific IgE to alpha-Gal analyzed for their reactivity to alpha-gal glycosylated proteins in gelatin products. AGS patient plasma showed strong reactivity towards gelatin from both sources. While, healthy human control sera did not show any reactivity towards gelatin products from either source. The alpha-gal protein glycosylation pattern for both products are similar suggesting that both are derived from the same source.

The lack of reactivity of AGS patient plasma towards GalSafe^{®} tissues in general strongly suggested that gelatin derived from GalSafe^{®} pigs, or cattle with GTTA1 gene inactivated, will not cause this alpha-gal IgE response and thus provide a safer alternative for AGS patients to use regardless if it is used as or in a food, cosmetic or medical products.

### Example 8: Knockout of GGTA1 in bovine fibroblasts and generation of GGTA1 inactivated bovine embryos by somatic cell nuclear transfer.

Cells. Bovine fetal fibroblasts were derived from a fetus at Day 32 of gestation. The fetus was generated by transferring a purebred Angus embryo into a recipient cow using standard non-surgical bovine embryo transfer techniques. To obtain fibroblasts, the fetus was harvested from the recipient cow at slaughter and the gravid uterus transported to the laboratory on ice. After removing the head and viscera, the fetus was diced into ~1mm cubes which were then washed and cultured as explants in DMEM + 10% fetal bovine serum (FBS) and antibiotics under a humidified atmosphere of 5% CO2 in air at 38.5C°. After several days, fibroblast outgrowths from the explants reached 80% confluency in the culture dish at which point they were harvested by trypsinization, resuspended in culture media + 10% DMSO and cryopreserved.

CRISPR. To knockout GGTA1, pairs of CRISPR single guide RNA (sgRNA) sequences were designed to create a ~90 bp deletion in GGTA1 exon 9. Using an online tool, a number of candidates sgRNAs were designed for high predicted cutting efficiency and low propensity for off-target cutting. To select the most efficient pair, each sgRNAs were tested individually and in pairs for cutting efficiency in bovine fibroblasts. Briefly, sgRNAs were mixed with recombinant Cas9 protein to form ribonucleoprotein (RNP) complexes and nucleofected (Amaxa) into fibroblasts. Bovine dermal fibroblast were transfected with a mixture of two guide RNAs and Cas-9 protein using the Lonza 4D electroporation system per manufactures instructions. Cells were grown for 72h then harvested. Cells were washed twice in DPBS with 1% fetal bovine serum (FBS) with antibiotics then mixed with FITC-IB4 lectin per manufactures instructions for 15 min. Cells were washed in DPBS + FBS+ antibiotics and subjected to flow cytometry. Non-transfected cells served as a positive control and GalSafe^{®} pigs cells served as a Negative control for setting a sorting gate. Negative stained transfected cells were sorted and collected in DPBS+ FBS+ antibiotics. These sorted cells were subjected to flow cytometry to evaluate the sorting efficiency (Figure 8).

The presence of CRISPR-induced indels at the GGTA1 target was evaluated in pools of transfected fibroblasts by next generation sequencing (MiSeq). The best pair of sgRNAs tested in a pool produced over 81% large deletion (~85 bp), and >99% were modified in some way. These two sgRNAs with the highest cutting efficiency were selected for generating GGTA1 knockout cells for nuclear transfer.

GGTA1 knockout fibroblasts. GGTA1 CRISPRs were transfected into bovine fibroblasts as RNPs as described above. After several days, cells were harvested and stained with Fluorescein labeled Griffonia Simplicifolia Lectin I (GSL I) isolectin B4 (FITC IB4). FITC-IB4 lectin binds specifically to alpha-1,3 galactose residues, so cells bearing a complete bi-allelic GGTA1 knockout are negative for FITC-IB4 and can be separated from IB4-positive cells by fluorescence-activated cell sorting (FACS). FITC-IB4-negative cells were then single-cell cloned at limiting dilution and the resulting colonies expanded and analyzed by MiSeq to confirm the presence of bi-allelic knockout deletions at the GGTA1 target.

The first ten single cell colonies sequenced all contained biallelic modifications that caused frameshifts and therefore would be expected to be full GGTA1 KOs. Five of these ten single cell clones contained biallelic modifications that deleted the sequence between the two sgRNA sites (-83 bp).

Using techniques known in the art, the following procedures will be utilized to generate cloned bovine embryos from homozygous GGTA1 knockout fibroblasts generated and confirmed as described above. Cloned embryos will then be used to generate cloned cows with knockout of bovine GGTA1 as described below.

Nuclear transfer. Bovine oocytes were aspirated as cumulus-oocyte complexes from ovaries obtained from an abattoir, placed in a medium containing FBS, follicle stimulating hormone and antibiotics, and cultured for ~20h at 38.5oC, during which the oocytes matured to the metaphase II stage of meiosis. Oocytes were stripped of surrounding cumulus cells, stained with a fluorescent DNA dye to help visualize metaphase chromosomes and cultured in a medium containing cytochalasin B to relax the cytoskeleton. Oocytes were then placed on the stage of an inverted microscope and enucleated by micromanipulation. Enucleation was confirmed by observing the absence of fluorescing chromosomes under UV illumination. Enucleated oocytes were then reconstructed by placing a single GGTA1 knockout fibroblast into the perivitelline space of the oocyte and subsequently fusing the fibroblast to the oocyte using a brief electrical pulse. Fusion was confirmed visually by the absence of a fibroblast in the perivitelline space.

Oocyte activation. Development was activated in reconstructed oocytes by treatment with a calcium ionophore (ionomycin) followed by a protein kinase inhibitor (6-dimenthyl amino purine; 6-DMAP) to promote chromatin decondensation and nuclear envelope formation.

Embryo culture. Activated embryos were placed in modified synthetic oviduct fluid (mSOF) and incubated for 7 days at 38.5oC in a humidified atmosphere of 5% CO2, 5% O2 and 90% N2.

Confirmation of GGTA1 KO in embryos. Embryos were analyzed individually to confirm presence of GGTA1 deletion by MiSeq as described above.

Embryo transfer and pregnancy diagnosis. Embryos were transferred to recipient cows using standard non-surgical techniques. Pregnancies were diagnosed by transrectal ultrasound at Day 28 of gestation, then monitored monthly with ultrasound until Day 60, and by rectal palpation until Day 250.

Calves. Calves were delivered by elective Cesarean section after artificial induction of labor. High-level neonatal care was provided, and calves were fed bottled colostrum as soon as they could nurse through the first day of life. Calves were individually housed in hutches according to standard bovine husbandry practices to minimize contraction of calf hood and bottle-fed on calf milk replacer.

Confirmation of GGTA1 KO genotype and phenotype in calves. GGTA1 knockout genotype was confirmed by PCR and MiSeq analysis on ear punch biopsies. Peripheral blood mononuclear cells (PBMC) were isolated from a tail-vein blood sample, stained with FITC-IB4 lectin and analyzed by FACS (Figure 8) as described above for fibroblasts. The absence of FITC-IB4 staining in PBMC served as evidence of GGTA1 KO phenotype.

### Example 9: AGS patient sera IgE reacts with alpha gal in bovine dermal fibroblasts (BDF).

Alpha gal syndrome (AGS) was characterized by delayed anaphylactic response due to presence of high levels alpha gal IgE antibodies towards mammalian meat products. These patients will react differently to alpha-gal present in bovine dermal fibroblast (BDF).

Results (Figure 9) Western blot analysis of serum IgE-reactive proteins in BDF. AGS patient plasma and control sera with specific IgE to alpha-gal glycosylated proteins in cell lysates was detected using mouse anti-Human IgE antibodies [Mouse monoclonal (B3102E8) anti-Human IgE Fc (HRP) (Abcam 99806)]. (a) AGS patient serum showed strong reactivity to unmodified bovine dermal fibroblast cell lysate and no reactivity towards alpha-gal knockout bovine fibroblast cell lysate. (b) The healthy human control sera did not show any reactivity towards unmodified and alpha-gal knockout bovine dermal fibroblast cell lysate.

### Example 10: Phenotypically GalSafe^{®} pigs are equivalent to standard domestic breeds "farm" of pigs.

The data indicate that the phenotype of the GalSafe^{®} pig is consistently normal when comparing growth, health status, and reproductive traits to unmodified (standard domestic breed) pigs.

This example demonstrates that the genotypic modification "The targeted insertion" does not cause any direct, unintended or indirect toxicity and subsequently does not pose a safety risk to the GalSafe^{®} line of pigs". The GalSafe^{®} line of pigs does not cause any direct, unintended or indirect toxicity to the health of these pigs to ensure the safety and welfare of such animals has not been compromised. Key traits examined in order to demonstrate absence of direct, unintended or indirect toxicity in the GalSafe^{®} line were growth, health and reproduction.

There is no evidence of direct, unintended or indirect toxicity related to the targeted insertion on the growth of the GalSafe^{®} pigs. The growth of GalSafe^{®} pigs through multiple generations was determined to be consistent with unmodified pigs. Live growth demonstrated that GalSafe^{®} pigs grow in a manner that is not different from unmodified pigs. Weight at reference ages (such as birth, weaning, etc) and average daily gains are normal when compared to unmodified animals.

Live animal growth (Figure 10) for GalSafe^{®} pigs falls predominantly within the normal range that has been established from mathematical growth models from birth to physiologic maturity for standard domestic breed pigs. Furthermore, a second level of growth, skeletal growth, assessed by an evaluation of long bones, did not demonstrate differences in macroscopic or microscopic bone characteristics when compared to standard domestic breed animals (Figure 11; Figure 12; Figure 13; Figure 14). Skeletal growth demonstrated that these tissues were physiologically and anatomically normal and fit previously established allometric skeletal growth models for standard domestic breed pigs. In addition, histology confirmed that changes in bone morphology with age are consistent in appearance to published descriptions of bone histology from standard domestic breed pigs of comparable age.

There is no evidence of direct, unintended or indirect toxicity related to the genetic modification on the health status of the GalSafe^{®} pigs. Concomitantly, the health status of the GalSafe^{®} pigs is normal; there are no detectable differences in health status between GalSafe^{®} and standard domestic breed pigs. A retrospective review of treatment records revealed that GalSafe^{®} pigs are susceptible to the same illnesses and diseases as standard domestic breed pigs. However, the overall prevalence of diseases and illnesses is lower in the GalSafe^{®} herd as compared to standard domestic breed pigs and most likely related to the barrier facility in which the GalSafe^{®} pigs are housed. After treatment for illnesses (by medicines typically administered to standard domestic breed pigs for similar illnesses), the GalSafe^{®} pig was found to respond to treatment in a similar manner to standard domestic breed pigs.

Piglet morbidity from GalSafe^{®} sows is consistent with published reports derived from standard domestic breed pigs. Additionally, a thorough evaluation of the physiological status of healthy GalSafe^{®} pigs that included necropsy, hematology, and serum chemistry evaluations did not reveal any aberrant anatomy or any evidence to suggest the presence of pathology. Thus, these evaluations indicated GalSafe^{®} pigs possess normal pig anatomy, and normal hematology and serum chemistry parameters (Figure 15; Figure 16; Figure 17; Figure 18; Figure 19; Figure 20).

There is no evidence of direct, unintended or indirect toxicity related to the targeted insertion on the reproduction of the GalSafe^{®} pigs. The reproductive system of the GalSafe^{®} pigs was observed to be consistent with the reproductive system of standard domestic breed pigs. The reproductive anatomy of the GalSafe^{®} pig was the same in appearance and function to standard domestic breed pigs. Major reproductive events in the reproductive cycle, specifically weaning, puberty, estrus (onset and duration), and gestation, occurred at similar timeframes when compared to standard domestic breed pigs. GalSafe^{®} pigs exhibited the same behavior during breeding and farrowing that is observed for standard domestic breed pigs. Quantitative traits that were defined to be number of teats, gestation length, and litter size were demonstrated to be consistently normal when compared to standard domestic breed pigs.

### Farrowing statistics

### Birthweight

Birthweights (Figure 21) were collected from 321 of 428 piglets representing 58 litters. Birthweights of individual pigs have ranged from 0.4 to 6.6 lbs (Figure 21). The average birth weight was 2.5lbs (±0.4; range 1.9 to 3.1) (Figure 21).

### Piglet mortality

Data (Figure 22) was collected from 428 piglets representing 58 litters were born with an average litter size of 7.2 piglets (±1.6; Range: 4.0 to 9.8. Average mortality at birth was determined as 0.6 piglets/litter (±0.6; range 0 to 2.0) while average mortality before weaning was observed for 1.9 piglets/litter (±1.0; range 0 to 3.4) Alternatively and expressed as a percentage of piglets observed average mortality at birth was 8.4% of the piglets (±7.7%; range 0 to 25.0%) while average mortality before weaning was observed for 27.1% of the piglets (±15.3%; range 0 to 55.6%) of piglets).

### Example 11: Compositional Study comparing nutritional facts between GalSafe^{®} and standard domestic breed pork meat primal cuts.

Study title: Compositional Analysis of GalSafe^{®} pigs compared to nonengineered pigs.

### Study objective:

1. Identify the potential of a food consumption risks as a result of the rDNA construct pPL657 or its gene product from perturbing the nutritional composition of GalSafe^{®} food product;
2. Support the nutrient label claims on GalSafe^{®} food products;
3. Demonstrate the nutrient claims on GalSafe^{®} food product is consistent to food products from nonengineered pigs.

In Vitro test systems: Various instruments that identify constituents consistent with the USDA nutrient regulations.

Test groups, sampling, and sample sizes:

### Test articles:

- Homozygous GalSafe^{®} Muscle sample
- Heterozygous GalSafe^{®} Muscle samples

### Controls:

- Standard domestic pig breed (Nonengineered) Muscle sample

### Sampling and sample size:

In short, sampling will be conducted as follows:
- A production lot is a set of food production consumer units that are from one production shift. Alternatively, a collection of consumer units of the same size, type, and style produced under conditions as nearly uniform as possible, designated by a common container code or marking, constitutes a production lot. For our purposes, a production lot will be considered a litter.
- The controls and test articles shall consist of a composite of a minimum of six consumer units, each from a production lot. Alternatively, the sample for nonengineered pigs may consist of a composite of a minimum of six consumer units, each sample chosen to be representative of a production lot.
- In each case, the units may be individually analyzed, and the results of the analyses averaged, or the units would be composited and the composite analyzed. In both cases, the results, whether an average or a single result from a composite, will be considered to be the nutrient content of a composite.

**Table 1. Production Lot - Consumer Units**

| | | |
|---|---|---|
| Group # of animals | Composite specimen for Compositional Analysis | |
| Homozygous GalSafe^{®} Pig | N=5 | 1 |
| Heterozygous GalSafe^{®} Pig | N=5 | 1 |
| standard domestic Pig N=5 | 1 | |

Inclusion Criteria: Heterozygous, homozygous GalSafe^{®} and "standard domestic breed" pigs
- Gender: female, or barrows
- Age: 250±100 days old
- Genotypic identity: confirmed per established LR-PCR analytical assay.
- Phenotypic identity: confirmed per established flow cytometry analytical assay. Exclusion criteria
- Swine hematology and blood chemistry value that indicates an abnormal condition or disease state.
- Any pig that has been treated with a veterinary drug such that the drug withdrawal time would exclude the animal for food use.
- Antemortem or postmortem inspection indicate an abnormal, unhealthy or disease state.

### Methods of Procurement and Composite:

Muscle samples from similar anatomic location will be obtained in bulk from heterozygous and homozygous GalSafe^{®}, and nonengineered pigs and subsequently packaged/labeled with animal ID, and date of procurement. Similar muscle samples from nonengineered pigs may be obtained from abattoir or vendor that may include a retail outlet. Nonengineered samples will be labeled with date of purchase and vendor. A record of procurement for each pig will be completed as described (appendix).

Skeletal muscle samples will be collected from a specified and easy identifiable primal cut for each animal in the respective groups (Table 1) . Thus, allow for a comparable analysis across all controls and test articles. The primal cut will be removed from the carcass, procured and appropriately distribute.

Per example: The tenderloin (¾ to 1½ pounds) will be identified and removed from each animal carcass as a single unit, processed, properly recorded and labeled, and subsequently distributed to the testing facility or facilities.

Samples will be sent to the institution conducting study for subsequent grinding of each test group into one composite specimen. Prior to compiling a composite sample, ~5-10 individual samples (~5 g/sample) will be collected from each consumer unit and returned to Revivicor for banking for additional studies and the preservation of samples representing each individual consumer unit. Any residual composite material not used for study will be returned to Revivicor for banking for any additional study needs.

Study Design: The procurement of test articles and controls for subsequent dissemination to the institution conducting the study. Institution conducting the study will fabricate composite samples and conduct analytic assays for nutrient analysis. Institution conducting the study will perform on phases of the study from acceptance of the materials to approving a final study report under good laboratory practices (GLP).

### Compositional Analysis:

Compositional analysis will be conducted according per USDA requirements (9 CFR 317.309(c)). In short, the Institution conducting will report the nutrient values for each composite sample as described in the table below.
Total calories
Calories from fat
Calories from saturated fat (VOLUNTARY)
Total fat
Saturated fat
Trans fat (FDA requirement)
Cholesterol
Sodium
Total carbohydrate"
Dietary fiber:
   Sugars
   Protein
   Vitamins and minerals:
      vitamin A
      vitamin C
      Vitamin D (FDA requirement)
      calcium
      iron
      Elemental profile (Ca, Fe, Na, Mg, P, K, and Zn)
      Moisture
      ASH
      Total
      Comparison of Nutrient values

Test articles will be compared to control values to identify if any nutrient values are different. Differences will be assessed per 9 CFR 317.9 h (5). Calories, sugars, total fat, saturated fat, cholesterol, or sodium shall be considered significant if the nutrient content of the homozygous GalSafe^{®} composite is greater than 20 percent in excess of the value for that of a nonengineered pig.

Additional comparison may be made from the USDA nutrient database (https://ndb.nal.usda.gov/ndb/ ) to demonstrate or similar scientific resources to identify any compositional component to confirm or refute putative toxicity.

### Example 12: Sensory Study to evaluate aroma and taste of GalSafe^{®} meat products.

The GalSafe^{®} line is engineered animals with both alleles of the glycoprotein galactosyltransferase alpha 1,3 gene (GGTA1) inactivated or "knocked out" referred to as homozygous or double knock outs (DKO and has undetectable endogenous alpha-gal sugar residues their derivatives. Currently there is no evidence to show that GalSafe^{®} pig derivatives (food, medical and cosmetic) are not as safe for human consumption and utility as comparable derivatives from a nonengineered (standard domestic breed) pigs.

An evaluation completed on health and compositional differences between tissues from DKO (including the GalSafe^{®} lineage) and standard domestic pig tissues concluded that except for the absence of α-gal, no differences were identified in tissue characteristics that included morphology, composition, mechanics, bioactive molecules, hematologic, biochemical, or coagulation parameters. Therefore, it will be a safe assumption to predict that there will be no differences in aroma or taste after the sensory study is concluded, since no meaningful differences could be detected between nonengineered and GalSafe^{®} pigs.

The purpose of the study is to compare the objective sensory profiles of meat from GalSafe^{®} pigs to that from standard domestic pigs. A descriptive sensory analysis by an appropriately trained panel will evaluate raw (visual, aroma only) and cooked (visual, aroma, flavor) pork loin as well as ground pork (from ham muscle).

Descriptive analysis is an objective sensory tool that uses a group of trained individuals to identify and quantify sensory attributes of products. The panel operates as an instrument and data is treated accordingly as such. These panelists are healthy individuals that are free from food sensitivities and allergies and are accustomed to tasting food products. The panel generally has previous experience profiling a wide variety of meats including beef, poultry, seafood and pork/pork bacon.

A group of highly trained sensory panelists with experience in the descriptive analysis of food flavor and texture) will document intensities of selected visual, ortho-nasal aroma and in-mouth flavor attributes of raw (visual and aroma only) and cooked pork products using a universal intensity scale consistent with the SpectrumTM method. Paper ballots will be used for data collection.

All sensory work is subject to and will be conducted with appropriate Institutional Review Board (IRB) review and approval. This includes panelists signing appropriate informed consent forms. Sensory testing will be done under the oversight of an appropriately qualified food scientist.

### Target animal species and classes;

a. Sus scrofa
b. Genotype
   i. Homozygous GalSafe^{®} pigs
   ii. Nonengineered progeny from a GalSafe^{®} gilt/sow and/or boar
c. Type
   i. barrows or gilts between 150- 350 lbs or 6-18 months in age
d. All nonengineered and homozygous GalSafe^{®} pigs selected for derivation of food products intended for the sensory study will have genotypic and phenotypic identity confirmed via analytical identity tests.

Putative conclusions:
1. No sensory differences detected in aroma and taste by trained sensory panelists
2. No acute or delayed anaphylactic response when presented to asymptomatic or symptomatic (confirmed allergic) individuals.

### Example 13: Triple Knockout Pigs "TKO pigs"

Confirmed homozygous GGTA1 knockout cell lines were produced using fetuses derived from outbred GalSafe^{®} pig lines. In the course of breeding, GGTA1 knock-out cell lines were identified by PCR and MiSeq analysis that had no detectable copies of porcine endogenous retrovirus class C (PERV C). In order to generate pigs with knockouts of three genes "TKO" critical for xeno-antigen expression the following experiment were performed. The detection of PERV C DNA sequence integration into porcine genome was detected by droplet digital PCR (ddPCR) using primers and probes as described by Bittmann et al. 2012. The ddPCR data allows for a calculation of copy number for PERVC in each animal tested. Further, it is possible to select PERVC negative, or low copy number animals as breeders in order to be able to breed PERVC out of the captive population.

The CRISPR/Cas9 system was used to knock out genes encoding cytidine monophosphate-N-acetyl neuraminic acid hydroxylase (CMAH) and β1,4 N-galactosaminotransferase (β4GalNT2) which catalyze synthesis of the xeno-antigens Neu5GC and Sd(a), respectively. DNA sequences encoding CRISPR guide RNAs designed to generate indels at CMAH and β4GalNT2 were inserted into pX330 plasmid and transfected the homozygous GGTA1KO pFF (PERV C negative line). Presumptive triple knockout (TKO) CMAH-KO (Neu5Gc negative) and β4GalNT2 knockout (β4 KO) cells with homozygous knockout of both targets were selected by negative staining for anti Neu5GC antibodies and biotinylated Dolichos Biflorus Agglutinin (DBA) lectin using FACS, single cell cloned and analyzed by next generation sequencing (MiSeq) for knockout indels in both CMAH and β4GalNT2 target genes.

Single cell clones with confirmed triple knockouts (GTKO, CMAHKO and B4KO; TKO) were used in somatic cell nuclear transfer to generate TKO pigs. Eleven TKO null pigs (per example data shown for animals: A34-1; A34-2; A36-1; A35-1; A35-2) were produced and their perspective geno- and phenotypes (Figure 24; Figure 25; Figure 26) were confirmed via flow cytometry (Table 2 (a))and MiSeQ analysis (Table 3) after birth. Table 2(b) per example data shown for animals: A172-1; A172-2; A172-3; A172-4) were produced and their genotype (Figure 27) were confirmed via flow cytometry, in addition their PERV C (-) were confirmed negative via ddPCR.

**Table 2(a) Summary of flow cytometry (FACS) results confirming that animals A34-1; A34-2; A35-1; A35-2; A36-1 are triple knock outs "TKO" pigs.**

| **Animal ID** | **Genotype** | **anti-NeuGC (CMAH KO)** | **DBA lectin (B4 KO)** | **IB4 lectin (GalSafe^{®} DKO)** |
|---|---|---|---|---|
| 907C | wild type (positive) control | pos (+) | pos (+) | neg (-) |
| 956-2 | negative control for CMAH | neg (-) | pos (+) | neg (-)- |
| A34-1 | GalSafe^{®} CMAH B4 KO | neg (-) | neg (-) | neg (-) |
| A34-2 | GalSafe^{®} CMAH B4 KO | neg (-) | neg (-) | neg (-) |
| A35-1 | GalSafe^{®} CMAH B4 KO | neg (-) | neg (-) | neg (-) |
| A35-2 | GalSafe^{®} CMAH B4 KO | neg (-) | neg (-) | neg (-) |
| A36-1 | GalSafe^{®} CMAH B4 KO | neg (-) | neg (-) | neg (-) |

**Table 2(b) Summary of flow cytometry (FACS) results confirming that animals A172-1; A172-2; A172-3; A172-4 are triple knock outs "TKO" pigs and PERV C negative.**

| **Animal ID** | **Genotype** | **anti-NeuGC (CMAH KO)** | **DBA lectin (B4 KO)** | **IB4 lectin (GalSafe^{®} DKO)** | **PERV C** |
|---|---|---|---|---|---|
| 246D | wild type; pos (+) control | pos (+) | pos+ | pos (+) | |
| 211-2 | wild type; pos (+) control | | | | pos (+) |
| A172-1 | GalSafe^{®} CMAH B4 KO | neg (-) | neg (-) | neg (-)- | neg (-)- |
| A172-2 | GalSafe^{®} CMAH B4 KO | neg (-) | neg (-) | neg (-) | neg (-) |
| A172-3 | GalSafe^{®} CMAH B4 KO | neg (-) | neg (-) | neg (-) | neg (-) |
| A172-4 | GalSafe^{®} CMAH B4 KO | neg (-) | neg (-) | neg (-) | neg (-) |

**Table 3 MiSeQ Analysis: MiSeQ sequencing results confirmed the triple knockout "TKO" genotype (inactive GTTA; CMAH and beta4 genes) status for Animals A34-1; A34-2; A35-1; A35-2; A36-1.**

| Sample | B4Gal genotype | CMAH genotype | B4Gal reads | CMAH reads |
|---|---|---|---|---|
| A34 1 | NEG | NEG | ALL -50 | ALL -40 |
| A34_2 | NEG | NEG | ALL -50 | ALL -40 |
| A35_1 | NEG | NEG | ALL -50 | ALL -40 |
| A35_2 | NEG | NEG | ALL -50 | ALL -40 |
| A36_1 | NEG | NEG | ALL -50 | ALL -40 |

### Example 14: Method of manufacture of GalSafe^{®} pigs with no detectable alpha gal cross contamination.

Confirmed GalSafe^{®} pigs are transferred to a dedicated slaughterhouse facility that has been either newly constructed or confirmed by alpha-gal specific ELISA or Western analysis to be free of pig materials/meat/meat by products from standard domestic breed pigs. After slaughter, GalSafe^{®} pigs and meat products (and medical products) will be processed, frozen, labeled, and stored in dedicated processing and storage facility until distributed for human use (for food or medical or cosmetic products).

Appropriate testing will be completed at different stages; geno- and phenotypic identity of the animals will be confirmed prior to slaughter, the slaughter house facility will be tested to be free of residual alpha-gal before processing of the animals, and the finished product will be tested to confirm the that the product contains no residual alpha-gal via an appropriate sampling plan determined by lot and and sample size. The testing will be done using analytical methods such as PCR and/or ELISA analysis specifically designed to detected low levels of alpha gal particles per billion..

### Example 15: Method of prescreening patients prior to medical procedures or medical product administration for anti-gal IgE levels.

Patients with confirmed AGS or with "high" titers of anti-gal IgE would be recommended to receive products/procedures that do not contain or utilize materials of mammalian origin. Examples of such may include patients that are candidates to receive bioprosthetic heart valves containing materials of porcine or bovine origin. A cardiologist for example would have to either use valve materials from a GalSafe^{®} or GGTA1 knockout cow for such indications or use only synthetic valves. Such screening may include a listing of medical/cosmetic/home-use products that contain ingredients of mammalian origin including heparin, collagen, gelatin, insulin, pancreatic enzymes, thyroid hormone, whey, casein etc. Only mammalian materials from GGTA1 deficient pigs or cows would be acceptable for patients who test positive for high levels of anti-gal IgE. This method of screening for patients receiving mammalian derived materials could also extend to implementation of such procedures by pharmacy employees, such that they would only prescribe or administer materials that were confirmed "mammalian free" for AGS patients or patients with high levels of anti-gal IgE.

## Claims

1. A medical product comprising a biologic that is derived from a transgenic ungulate lacking an alpha 1, 3 galactosyltransferase gene for use in the treatment of a cardiovascular disease in a human that has been diagnosed with α-Gal syndrome (AGS), wherein the medical product does not comprise galactose-alpha-1,3-galactose (alpha-gal), wherein the medical product prevents or reduces the risk or severity of an allergic reaction to the alpha-gal in the human, wherein the biologic is a cardiovascular implant comprising a heart valve, optionally wherein the heart valve is a bioprosthetic heart valve, and wherein the medical product does not exhibit premature degradation.

2. The medical product for use according to claim 1, wherein:
(a) the ungulate is selected from the group consisting of cattle, pigs, goats, horses, camels, sheep, venison, and bison; or
(b) the ungulate is a pig or a cow.

3. The medical product for use according to claim 1 or 2, wherein the ungulate further comprises one or more additional genetic modifications selected from the group consisting of inactivation or reduction of expression of Neu5Gc (CMAH knockout), Beta4Gal (knockout of Beta4GaINT2), and a combination thereof.

4. The medical product for use according to any one of claims 1-3, wherein the premature degradation is a calcification- or an inflammation-induced premature degradation.

5. The medical product for use according to any one of claims 1-4, wherein the human has IgE antibodies directed to galactose-alpha-1,3-galactose (alpha-gal).

6. The medical product for use according to any one of claims 1-4, wherein the human has IgE and lgG4 antibodies directed to alpha-gal.

## Patentansprüche

1. Medizinisches Produkt, umfassend ein biologisches Präparat, das von einem transgenen Huftier stammt, dem ein alpha-1,3-Galactosyltransferase-Gen fehlt, zur Verwendung bei der Behandlung einer Herz-Kreislauf-Erkrankung bei einem Menschen, bei dem AGS (α-Gal-Syndrom) diagnostiziert wurde, wobei das medizinische Produkt keine Galactose-alpha-1,3-galactose (alpha-Gal) umfasst, wobei das medizinische Produkt das Risiko oder die Schwere einer allergischen Reaktion gegen die alpha-Gal bei dem Menschen verhindert oder reduziert, wobei es sich bei dem biologischen Präparat um ein kardiovaskuläres Implantat handelt, das eine Herzklappe umfasst, gegebenenfalls wobei es sich bei der Herzklappe um eine bioprothetische Herzklappe handelt und wobei das medizinische Produkt keinen vorzeitigen Abbau zeigt.

2. Medizinisches Produkt zur Verwendung nach Anspruch 1, wobei:
a) das Huftier aus der Gruppe bestehend aus Rindern, Schweinen, Ziegen, Pferden, Kamelen, Schafen, Hirschwild und Bisons ausgewählt ist; oder
b) das Huftier ein Schwein oder eine Kuh ist.

3. Medizinisches Produkt zur Verwendung nach Anspruch 1 oder 2, wobei das Huftier ferner eine oder mehrere zusätzliche gentechnische Veränderungen umfasst, die aus der Gruppe bestehend aus Inaktivierung oder Verringerung der Expression von Neu5Gc (CMAH-Knockout), Beta4Gal (Knockout von Beta4GalNT2) und einer Kombination davon ausgewählt sind.

4. Medizinisches Produkt zur Verwendung nach einem der Ansprüche 1-3, wobei es sich bei dem vorzeitigen Abbau um einen durch Verkalkung oder Entzündung induzierten vorzeitigen Abbau handelt.

5. Medizinisches Produkt zur Verwendung nach einem der Ansprüche 1-4, wobei der Mensch IgE-Antikörper aufweist, die gegen Galactose-alpha-1,3-galactose (alpha-Gal) gerichtet sind.

6. Medizinisches Produkt zur Verwendung nach einem der Ansprüche 1-4, wobei der Mensch IgE- und IgG4-Antikörper aufweist, die gegen alpha-Gal gerichtet sind.

## Revendications

1. Produit médical comprenant un produit biologique qui est dérivé d'un ongulé transgénique dépourvu d'un gène alpha-1,3-galactosyltransférase à utiliser dans le traitement d'une maladie cardiovasculaire chez un humain qui a été diagnostiqué du syndrome de α-Gal (AGS), dans lequel le produit médical ne comprend pas de galactose-alpha-1,3-galactose (alpha-gal), dans lequel le produit médical prévient ou réduit le risque ou la gravité d'une réaction allergique à l'alpha-gal chez l'humain, dans lequel le produit biologique est un implant cardiovasculaire comprenant une valvule cardiaque, éventuellement dans lequel la valvule cardiaque est une valvule cardiaque bioprothétique, et dans lequel le produit médical ne présente pas de dégradation prématurée.

2. Produit médical pour une utilisation selon la revendication 1, dans lequel :
(a) l'ongulé est choisi dans le groupe comprenant les bovins, les porcs, les chèvres, les chevaux, les chameaux, les moutons, le gibier et les bisons ; ou
(b) l'ongulé est un porc ou une vache.

3. Produit médical destiné à être utilisé selon la revendication 1 ou 2, dans lequel l'ongulé comprend en outre une ou plusieurs modifications génétiques supplémentaires choisies dans le groupe constitué par l'inactivation ou la réduction de l'expression de Neu5Gc (inactivation CMAH), Beta4Gal (inactivation de Beta4GalNT2), et une combinaison de celles-ci.

4. Produit médical destiné à être utilisé selon l'une quelconque des revendications 1 à 3, dans lequel la dégradation prématurée est une dégradation prématurée induite par une calcification ou par une inflammation.

5. Produit médical destiné à être utilisé selon l'une quelconque des revendications 1 à 4, dans lequel l'humain a des anticorps IgE dirigés contre le galactose-alpha-1,3-galactose (alpha-gal).

6. Produit médical destiné à être utilisé selon l'une quelconque des revendications 1 à 4, dans lequel l'humain a des anticorps IgE et IgG4 dirigés contre alpha-gal.
